# EUROPEAN PATENT APPLICATION

(11) **EP 2 410 061 A1**
(43) Date of publication of application: **25.01.2012**
(21) Application number: 10007525.8
(22) Date of filing: 20.07.2010
(51) Int. Cl.: C12P 7/10, C12N 9/42

(54) **Artificial cellulosome and the use of the same for enzymatic breakdown of resilient substrates**

(71) Applicant: Schwarz, Wolfgang, 80637 München (DE)
(72) Inventor: Schwarz, Wolfgang, 80637 München (DE); Krauss, Jan, 85354 Freising (DE); Zverlov, Vladimir, 80797 München (DE); Hornburg, Daniel, 85356 Freising (DE); Köck, Daniela, 85354 Freising (DE); Schulte, Louis-Philipp, 80634 München (DE)
(74) Representative: Grünecker, Kinkeldey, Stockmair & Schwanhäusser

(57) **Abstract**

The present invention relates to an artificial cellulosome for enzymatic breakdown of resilient substrates. In particular, the present invention provides a complex having an increased activity on resilient substrates, such as crystalline cellulose. The complex comprises a backbone scaffold having at least four binding sites capable of binding the enzyme components, whereby at least two of the binding sites have essentially the same binding specificity; and at least three different enzyme components being randomly bound to the at least four binding sites. Method for preparing the complex and uses of the same for enzymatic breakdown of resilient substrates are also provided.

SEQUENCE LISTING

<110>  Schwarz, Wolfgang H.

<120>  Artificial cellulosome and the use of the same for enzymatic
       breakdown of resilient substrates

<130>  EP70777FZapu

<140>  not yet assigned
<141>  herewith

<160>  26

<170>  PatentIn version 3.3

<210>  1
<211>  1488
<212>  DNA
<213>  Clostridium thermocellum

<220>
<223> CelA-d1

<400>  1
atgagaggat cgcatcacca tcaccatcac ggatccgcat gcgagctcaa aaaagtgaag      60

aacgtaaaaa aaagagtagg tgtggttttg ctgattcttg cagtgttggg ggtttatatg     120

ttggcaatgc cggcaaacac tgtgtcagcg gcaggtgtgc cttttaacac aaaatacccc     180

tatggtccta cttctattgc cgataatcag tcggaagtaa ctgcaatgct caaagcagaa     240

tgggaagact ggaagagcaa gagaattacc tcgaacggtg caggaggata caagagagta     300

cagcgtgatg cttccaccaa ttatgatacg gtatccgaag gtatgggata cggacttctt     360

ttggcggttt gctttaacga acaggctttg tttgacgatt tataccgtta cgtaaaatct     420

catttcaatg gaaacggact tatgcactgg cacattgatg ccaacaacaa tgttacaagt     480

catgacggcg gcgacggtgc ggcaaccgat gctgatgagg atattgcact tgcgctcata     540

tttgcggaca agttatgggg ttcttccggt gcaataaact acgggcagga agcaaggaca     600

ttgataaaca atctttacaa ccattgtgta gagcatggat cctatgtatt aaagcccggt     660

gacagatggg gaggttcatc agtaacaaac ccgtcatatt ttgcgcctgc atggtacaaa     720

gtgtatgctc aatatacagg agacacaaga tggaatcaag tggcggacaa gtgttaccaa     780

attgttgaag aagttaagaa atacaacaac ggaaccggcc ttgttcctga ctggtgtact     840

gcaagcggaa ctccggcaag cggtcagagt tacgactaca aatatgatgc tacacgttac     900

ggctggagaa ctgccgtgga ctattcatgg tttggtgacc agagagcaaa ggcaaactgc     960

gatatgctga ccaaattctt tgccagagac ggggcaaaag gaatcgttga cggatacaca    1020

attcaaggtt caaaaattag caacaatcac aacgcatcat ttataggacc tgttgcggca    1080

gcaagtatga caggttacga tttgaacttt gcaaaggaac tttataggga gactgttgct    1140

gtaaaggaca gtgaatatta cggatattac ggaaacagct tgagactgct cactttgttg    1200

tacataacag gaaacttccc gaatcctttg agtgaccttt ccggccaacc gacaccaccg    1260

tcgaatccga caccttcatt gcctcctcag gttgtttacg gtgatgtaaa tggcgacggt 1320

aatgttaact ccactgattt gactatgtta aaaagatatc tgctgaagag tgttaccaat 1380

ataaacagag aggctgcaga cgttaatcgt gacggtgcga ttaactcctc tgacatgact 1440

atattaaaga gatatctgat aaagagcata ccccacctac cttattag 1488

<210> 2
<211> 495
<212> PRT
<213> Clostridium thermocellum

<220>
<223> CelA-d1

<400> 2

Met Arg Gly Ser His His His His His His Gly Ser Ala Cys Glu Leu
1               5                   10                  15

Lys Lys Val Lys Asn Val Lys Lys Arg Val Gly Val Val Leu Leu Ile
            20                  25                  30

Leu Ala Val Leu Gly Val Tyr Met Leu Ala Met Pro Ala Asn Thr Val
        35                  40                  45

Ser Ala Ala Gly Val Pro Phe Asn Thr Lys Tyr Pro Tyr Gly Pro Thr
    50                  55                  60

Ser Ile Ala Asp Asn Gln Ser Glu Val Thr Ala Met Leu Lys Ala Glu
65                  70                  75                  80

Trp Glu Asp Trp Lys Ser Lys Arg Ile Thr Ser Asn Gly Ala Gly Gly
                85                  90                  95

Tyr Lys Arg Val Gln Arg Asp Ala Ser Thr Asn Tyr Asp Thr Val Ser
            100                 105                 110

Glu Gly Met Gly Tyr Gly Leu Leu Leu Ala Val Cys Phe Asn Glu Gln
        115                 120                 125

Ala Leu Phe Asp Asp Leu Tyr Arg Tyr Val Lys Ser His Phe Asn Gly
    130                 135                 140

Asn Gly Leu Met His Trp His Ile Asp Ala Asn Asn Asn Val Thr Ser
145                 150                 155                 160

His Asp Gly Gly Asp Gly Ala Ala Thr Asp Ala Asp Glu Asp Ile Ala
                165                 170                 175

Leu Ala Leu Ile Phe Ala Asp Lys Leu Trp Gly Ser Ser Gly Ala Ile
            180                 185                 190

Asn Tyr Gly Gln Glu Ala Arg Thr Leu Ile Asn Asn Leu Tyr Asn His
        195                 200                 205

Cys Val Glu His Gly Ser Tyr Val Leu Lys Pro Gly Asp Arg Trp Gly
    210                 215                 220

Gly Ser Ser Val Thr Asn Pro Ser Tyr Phe Ala Pro Ala Trp Tyr Lys
225                 230                 235                 240

Val Tyr Ala Gln Tyr Thr Gly Asp Thr Arg Trp Asn Gln Val Ala Asp
                245                 250                 255

Lys Cys Tyr Gln Ile Val Glu Glu Val Lys Lys Tyr Asn Asn Gly Thr
            260                 265                 270

Gly Leu Val Pro Asp Trp Cys Thr Ala Ser Gly Thr Pro Ala Ser Gly
        275                 280                 285

Gln Ser Tyr Asp Tyr Lys Tyr Asp Ala Thr Arg Tyr Gly Trp Arg Thr
    290                 295                 300

Ala Val Asp Tyr Ser Trp Phe Gly Asp Gln Arg Ala Lys Ala Asn Cys
305                 310                 315                 320

Asp Met Leu Thr Lys Phe Phe Ala Arg Asp Gly Ala Lys Gly Ile Val
                325                 330                 335

Asp Gly Tyr Thr Ile Gln Gly Ser Lys Ile Ser Asn Asn His Asn Ala
            340                 345                 350

Ser Phe Ile Gly Pro Val Ala Ala Ala Ser Met Thr Gly Tyr Asp Leu
        355                 360                 365

Asn Phe Ala Lys Glu Leu Tyr Arg Glu Thr Val Ala Val Lys Asp Ser
    370                 375                 380

Glu Tyr Tyr Gly Tyr Tyr Gly Asn Ser Leu Arg Leu Leu Thr Leu Leu
385                 390                 395                 400

Tyr Ile Thr Gly Asn Phe Pro Asn Pro Leu Ser Asp Leu Ser Gly Gln
                405                 410                 415

Pro Thr Pro Pro Ser Asn Pro Thr Pro Ser Leu Pro Pro Gln Val Val
            420                 425                 430

Tyr Gly Asp Val Asn Gly Asp Gly Asn Val Asn Ser Thr Asp Leu Thr
        435                 440                 445

Met Leu Lys Arg Tyr Leu Leu Lys Ser Val Thr Asn Ile Asn Arg Glu
    450                 455                 460

Ala Ala Asp Val Asn Arg Asp Gly Ala Ile Asn Ser Ser Asp Met Thr
465                 470                 475                 480

Ile Leu Lys Arg Tyr Leu Ile Lys Ser Ile Pro His Leu Pro Tyr
                485                 490                 495

<210>  3
<211>  2502
<212>  DNA
<213>  Thermotoga maritima

<220>
<223>  BglB-d1

<400>  3
atgagaggat ctcaccatca ccatcaccat gggatccaaa tggcggtaga tatcaagaaa      60

ataataaagc agatgacttt ggaagaaaaa gcagggttgt gctcgggact ggatttttgg     120

cataccaagc ctgttgagag actgggcatt ccttcaataa tgatgactga cggacctcat     180

ggactgagaa agcagaggga agatgcagag attgcggaca tcaacaacag cgttccagca     240

acctgtttc cgtctgcagc aggtttggca tgttcctggg acagagaact ggttgagaga     300

gtaggtgcag cactaggaga agaatgtcag gcggaaaatg tctcaatact gcttggacca     360

ggtgcaaata taaagcgttc acctttgtgt ggaagaaatt ttgaatattt tcccgaagac     420

ccttatcttt cgtcagagct ggcggcaagc catataaaag gagttcaaag tcagggagtg     480

ggtgcatgtc ttaaacattt tgccgcaaac aaccaggaac accggagaat gaccgttgat     540

accattgtag atgaaagaac gttgagggaa atatattttg caagctttga gaatgctgta     600

aaaaaagcac ggccttgggt ggttatgtgt gcatataaca agctcaacgg tgaatattgt     660

tcggagaaca gatatctttt gacggaagtt ttaaagaatg aatggatgca tgacggcttt     720

gtggtatccg actggggtgc ggtaaatgac agggtcagcg gcctggatgc aggtcttgac     780

ctggaaatgc ccaccagtca tggtattacg gataaaaaga tagttgaagc cgtaaaaagc     840

ggaaagctgt ctgaaaatat tttaaacaga gctgtggaaa gaattttgaa agtaattatt     900

atggcactgg aaaacaaaaa agaaaacgcg cagtatgaac aagatgctca tcacagactg     960

gcaaggcagg ctgcggccga atcgatggtt cttcttaaaa acgaggacga tgtgcttcct    1020

ttaaaaaaga gcggaaccat agctttgata ggagcttttg tgaaaaaacc aagataccag    1080

ggttcgggca gttctcatat taccccgaca agacttgatg atatttatga agagataaaa    1140

aaggccggag ccgacaaagt aaaccttgta tattcggaag gatacaggct tgaaaatgac    1200

ggtattgatg aggaattgat aaacgaagct aaaaaggcgg catcaagctc ggatgttgcg    1260

gtagtatttg cagggcttcc ggatgaatat gaatctgaag gatttgacag aactcacatg    1320

agtattccgg aaaatcaaaa caggctgata gaagcggtgg ccgaagtcca gagtaatatt    1380

gttgtggtat tgcttaacgg ctcaccggtt gaaatgccgt ggattgacaa ggtaaaatcc    1440

gtgcttgaag cttatcttgg aggccaggcg ctgggaggcc gctggcggat gtgctattcg    1500

gtgaagtcaa tcgtcggaaa acttgcggag accttcccgg tgaaattaag ccataatccg 1560
tcctatttga attttcccgg agaggatgac cgagtggagt ataaagaagg gttgtttgtc 1620
ggatacagat attatgatac aaagggaatt gagccattgt tcccctttgg tcacggactt 1680
agctatacca aatttgaata cagtgatata tcagtcgata aaaaagatgt ttcggacaat 1740
agcatcataa atgtcagcgt taaagtcaaa aatgttggaa aaatggcagg aaaagaaatt 1800
gtgcagctgt atgtaaaaga tgtgaaaagc agcgtcagaa gacctgagaa agagcttaaa 1860
ggatttgaaa aggtcttcct taatccggga gaagaaaaga cggttacatt tactttggac 1920
aaaagggctt ttgcatatta caatactcag attaaggact ggcatgttga aagcggagag 1980
tttctgatat taataggaag gtcctccagg gacatagttt taaaagaatc agtgagagta 2040
aattcaacgg tgaagataag aaaaagattc acagtgaatt cagcggttga agatgtaatg 2100
tccgattctt cggctgcggc cgttttaggg cctgtactaa aagagataac cgatgcactg 2160
cagattgata tggacaatgc tcatgacatg atggcggcca atataaagaa tatgcctttg 2220
cgctcacttg tcggttactc tcagggaagg ttaagcgaag aaatgctgga ggaactggtt 2280
gacaaaataa acaacgtcga ctgcaatggc gacggaaaag ttaattcaac tgacgctgtg 2340
gcattgaaga gatatatctt gagatcaggt ataagcatca acactgataa tgctgatgta 2400
aatgctgatg gcagagttaa ctctacagac ttggcaatat tgaagagata tattcttaaa 2460
gagctcggta ccccgggtcg acctgcagcc aagcttaatt ag 2502

<210> 4
<211> 833
<212> PRT
<213> Thermotoga maritima

<220>
<223> BglB-d1

<400> 4

Met Arg Gly Ser His His His His His His Gly Ile Gln Met Ala Val
1               5                   10                  15

Asp Ile Lys Lys Ile Ile Lys Gln Met Thr Leu Glu Glu Lys Ala Gly
            20                  25                  30

Leu Cys Ser Gly Leu Asp Phe Trp His Thr Lys Pro Val Glu Arg Leu
        35                  40                  45

Gly Ile Pro Ser Ile Met Met Thr Asp Gly Pro His Gly Leu Arg Lys
    50                  55                  60

Gln Arg Glu Asp Ala Glu Ile Ala Asp Ile Asn Asn Ser Val Pro Ala
65                  70                  75                  80

Thr Cys Phe Pro Ser Ala Ala Gly Leu Ala Cys Ser Trp Asp Arg Glu

85                  90                  95

Leu Val Glu Arg Val Gly Ala Ala Leu Gly Glu Glu Cys Gln Ala Glu
            100                 105                 110

Asn Val Ser Ile Leu Leu Gly Pro Gly Ala Asn Ile Lys Arg Ser Pro
        115                 120                 125

Leu Cys Gly Arg Asn Phe Glu Tyr Phe Pro Glu Asp Pro Tyr Leu Ser
    130                 135                 140

Ser Glu Leu Ala Ala Ser His Ile Lys Gly Val Gln Ser Gln Gly Val
145                 150                 155                 160

Gly Ala Cys Leu Lys His Phe Ala Ala Asn Asn Gln Glu His Arg Arg
                165                 170                 175

Met Thr Val Asp Thr Ile Val Asp Glu Arg Thr Leu Arg Glu Ile Tyr
            180                 185                 190

Phe Ala Ser Phe Glu Asn Ala Val Lys Lys Ala Arg Pro Trp Val Val
        195                 200                 205

Met Cys Ala Tyr Asn Lys Leu Asn Gly Glu Tyr Cys Ser Glu Asn Arg
    210                 215                 220

Tyr Leu Leu Thr Glu Val Leu Lys Asn Glu Trp Met His Asp Gly Phe
225                 230                 235                 240

Val Val Ser Asp Trp Gly Ala Val Asn Asp Arg Val Ser Gly Leu Asp
                245                 250                 255

Ala Gly Leu Asp Leu Glu Met Pro Thr Ser His Gly Ile Thr Asp Lys
            260                 265                 270

Lys Ile Val Glu Ala Val Lys Ser Gly Lys Leu Ser Glu Asn Ile Leu
        275                 280                 285

Asn Arg Ala Val Glu Arg Ile Leu Lys Val Ile Ile Met Ala Leu Glu
    290                 295                 300

Asn Lys Lys Glu Asn Ala Gln Tyr Glu Gln Asp Ala His His Arg Leu
305                 310                 315                 320

Ala Arg Gln Ala Ala Ala Glu Ser Met Val Leu Leu Lys Asn Glu Asp
                325                 330                 335

Asp Val Leu Pro Leu Lys Lys Ser Gly Thr Ile Ala Leu Ile Gly Ala
            340                 345                 350

Phe Val Lys Lys Pro Arg Tyr Gln Gly Ser Gly Ser Ser His Ile Thr

        355                 360                 365

Pro Thr Arg Leu Asp Asp Ile Tyr Glu Glu Ile Lys Lys Ala Gly Ala
    370                 375                 380

Asp Lys Val Asn Leu Val Tyr Ser Glu Gly Tyr Arg Leu Glu Asn Asp
385                 390                 395                 400

Gly Ile Asp Glu Glu Leu Ile Asn Glu Ala Lys Lys Ala Ala Ser Ser
                405                 410                 415

Ser Asp Val Ala Val Val Phe Ala Gly Leu Pro Asp Glu Tyr Glu Ser
            420                 425                 430

Glu Gly Phe Asp Arg Thr His Met Ser Ile Pro Glu Asn Gln Asn Arg
        435                 440                 445

Leu Ile Glu Ala Val Ala Glu Val Gln Ser Asn Ile Val Val Val Leu
    450                 455                 460

Leu Asn Gly Ser Pro Val Glu Met Pro Trp Ile Asp Lys Val Lys Ser
465                 470                 475                 480

Val Leu Glu Ala Tyr Leu Gly Gly Gln Ala Leu Gly Gly Arg Trp Arg
                485                 490                 495

Met Cys Tyr Ser Val Lys Ser Ile Val Gly Lys Leu Ala Glu Thr Phe
            500                 505                 510

Pro Val Lys Leu Ser His Asn Pro Ser Tyr Leu Asn Phe Pro Gly Glu
        515                 520                 525

Asp Asp Arg Val Glu Tyr Lys Glu Gly Leu Phe Val Gly Tyr Arg Tyr
    530                 535                 540

Tyr Asp Thr Lys Gly Ile Glu Pro Leu Phe Pro Phe Gly His Gly Leu
545                 550                 555                 560

Ser Tyr Thr Lys Phe Glu Tyr Ser Asp Ile Ser Val Asp Lys Lys Asp
                565                 570                 575

Val Ser Asp Asn Ser Ile Ile Asn Val Ser Val Lys Val Lys Asn Val
            580                 585                 590

Gly Lys Met Ala Gly Lys Glu Ile Val Gln Leu Tyr Val Lys Asp Val
        595                 600                 605

Lys Ser Ser Val Arg Arg Pro Glu Lys Glu Leu Lys Gly Phe Glu Lys
    610                 615                 620

Val Phe Leu Asn Pro Gly Glu Glu Lys Thr Val Thr Phe Thr Leu Asp

625                 630                 635                 640

Lys Arg Ala Phe Ala Tyr Tyr Asn Thr Gln Ile Lys Asp Trp His Val
                645                 650                 655

Glu Ser Gly Glu Phe Leu Ile Leu Ile Gly Arg Ser Ser Arg Asp Ile
            660                 665                 670

Val Leu Lys Glu Ser Val Arg Val Asn Ser Thr Val Lys Ile Arg Lys
        675                 680                 685

Arg Phe Thr Val Asn Ser Ala Val Glu Asp Val Met Ser Asp Ser Ser
    690                 695                 700

Ala Ala Ala Val Leu Gly Pro Val Leu Lys Glu Ile Thr Asp Ala Leu
705                 710                 715                 720

Gln Ile Asp Met Asp Asn Ala His Asp Met Met Ala Ala Asn Ile Lys
                725                 730                 735

Asn Met Pro Leu Arg Ser Leu Val Gly Tyr Ser Gln Gly Arg Leu Ser
            740                 745                 750

Glu Glu Met Leu Glu Glu Leu Val Asp Lys Ile Asn Asn Val Asp Cys
        755                 760                 765

Asn Gly Asp Gly Lys Val Asn Ser Thr Asp Ala Val Ala Leu Lys Arg
    770                 775                 780

Tyr Ile Leu Arg Ser Gly Ile Ser Ile Asn Thr Asp Asn Ala Asp Val
785                 790                 795                 800

Asn Ala Asp Gly Arg Val Asn Ser Thr Asp Leu Ala Ile Leu Lys Arg
                805                 810                 815

Tyr Ile Leu Lys Glu Leu Gly Thr Pro Gly Arg Pro Ala Ala Lys Leu
            820                 825                 830

Asn


<210>  5
<211>  2274
<212>  DNA
<213>  Clostridium thermocellum


<220>
<223>  CelS-d1

<400>  5
atgagaggat ctcaccatca ccatcaccat gggatccgca tgcagagaat ggtaaaaagc      60

agaaagattt ctattctgtt ggcagttgca atgctggtat ccataatgat acccacaact     120

gcattcgcag gtcctacaaa ggcacctaca aaagatggga catcttataa ggatcttttc 180
cttgaactct acggaaaaat taaagatcct aagaacggat atttcagccc agacgaggga 240
attccttatc actcaattga aacattgatc gttgaagcgc cggactacgg tcacgttact 300
accagtgagg ctttcagcta ttatgtatgg cttgaagcaa tgtatggaaa tctcacaggc 360
aactggtccg gagtagaaac agcatggaaa gttatggagg attggataat tcctgacagc 420
acagagcagc cgggtatgtc ttcttacaat ccaaacagcc ctgccacata tgctgacgaa 480
tatgaggatc cttcatacta tccttcagag ttgaagtttg ataccgtaag agttggatcc 540
gaccctgtac acaacgacct tgtatccgca tacggtccta acatgtacct catgcactgg 600
ttgatggacg ttgacaactg gtacggtttt ggtacaggaa cacgggcaac attcataaac 660
accttccaaa gaggtgaaca ggaatccaca tgggaaacca ttcctcatcc gtcaatagaa 720
gagttcaaat acggcggacc gaacggattc cttgatttgt ttacaaagga cagatcatat 780
gcaaaacagt ggcgttatac aaacgctcct gacgcagaag gccgtgctat acaggctgtt 840
tactgggcaa acaaatgggc aaaggagcag ggtaaaggtt ctgccgttgc ttccgttgta 900
tccaaggctg caaagatggg tgacttcttg agaaacgaca tgttcgacaa atacttcatg 960
aagatcggtg cacaggacaa gactcctgct accggttatg acagtgcaca ctaccttatg 1020
gcctggtata ctgcatgggg tggtggaatt ggtgcatcct gggcatggaa gatcggatgc 1080
agccacgcac acttcggata tcagaaccca ttccagggat gggtaagtgc aacacagagc 1140
gactttgctc ctaaatcatc caacggtaag agagactgga caacaagcta caagagacag 1200
cttgaattct atcagtggtt gcagtcggct gaaggtggta ttgccggtgg agcaaccaac 1260
tcctggaacg gtagatatga gaaatatcct gctggtacgt caacgttcta tggtatggca 1320
tatgttccgc atcctgtata cgctgacccg ggtagtaacc agtggttcgg attccaggca 1380
tggtcaatgc agcgtgtaat ggagtactac ctcgaaacag gagattcatc agttaagaat 1440
ttgattaaga agtgggtcga ctgggtaatg agcgaaatta agctctatga cgatggaaca 1500
tttgcaattc ctagcgacct cgagtggtca ggtcagcctg atacatggac cggaacatac 1560
acaggcaacc cgaacctcca tgtaagagta acttcttacg gtactgacct tggtgttgca 1620
ggttcacttg caaatgctct tgcaacttat gccgcagcta cagaaagatg ggaaggaaaa 1680
cttgatacaa aagcaagaga catggctgct gaactggtta accgtgcatg gtacaacttc 1740
tactgctctg aaggaaaagg tgttgttact gaggaagcac gtgctgacta caaacgtttc 1800
tttgagcagg aagtatacgt tccggcaggt tggagcggta ctatgccgaa cggtgacaag 1860
attcagcctg gtattaagtt catagacatc cgtacaaaat atagacaaga tccttactac 1920
gatatagtat atcaggcata cttgagaggc gaagctcctg tattgaatta tcaccgcttc 1980
tggcatgaag ttgaccttgc agttgcaatg ggtgtattgg ctacatactt cccggatatg 2040
acatataaag tacctggtac tccttctact aaattatacg gcgacgtcaa tgatgacgga 2100
aaagttaact caactgacgc tgtagcattg aagagatatg ttttgagatc aggtataagc 2160

atcaacactg acaatgccga tttgaatgaa gacggcagag ttaattcaac tgacttagga   2220

attttgaaga gatatattct caaagaaata gatacattgc cgtacaagaa ctaa         2274

<210>  6
<211>  757
<212>  PRT
<213>  Clostridium thermocellum

<220>
<223> CelS-d1

<400>  6

Met Arg Gly Ser His His His His His His Gly Ile Arg Met Gln Arg
1               5                   10                  15

Met Val Lys Ser Arg Lys Ile Ser Ile Leu Leu Ala Val Ala Met Leu
            20                  25                  30

Val Ser Ile Met Ile Pro Thr Thr Ala Phe Ala Gly Pro Thr Lys Ala
        35                  40                  45

Pro Thr Lys Asp Gly Thr Ser Tyr Lys Asp Leu Phe Leu Glu Leu Tyr
    50                  55                  60

Gly Lys Ile Lys Asp Pro Lys Asn Gly Tyr Phe Ser Pro Asp Glu Gly
65                  70                  75                  80

Ile Pro Tyr His Ser Ile Glu Thr Leu Ile Val Glu Ala Pro Asp Tyr
                85                  90                  95

Gly His Val Thr Thr Ser Glu Ala Phe Ser Tyr Tyr Val Trp Leu Glu
            100                 105                 110

Ala Met Tyr Gly Asn Leu Thr Gly Asn Trp Ser Gly Val Glu Thr Ala
        115                 120                 125

Trp Lys Val Met Glu Asp Trp Ile Ile Pro Asp Ser Thr Glu Gln Pro
    130                 135                 140

Gly Met Ser Ser Tyr Asn Pro Asn Ser Pro Ala Thr Tyr Ala Asp Glu
145                 150                 155                 160

Tyr Glu Asp Pro Ser Tyr Tyr Pro Ser Glu Leu Lys Phe Asp Thr Val
                165                 170                 175

Arg Val Gly Ser Asp Pro Val His Asn Asp Leu Val Ser Ala Tyr Gly
            180                 185                 190

Pro Asn Met Tyr Leu Met His Trp Leu Met Asp Val Asp Asn Trp Tyr
        195                 200                 205

Gly Phe Gly Thr Gly Thr Arg Ala Thr Phe Ile Asn Thr Phe Gln Arg
    210                 215                 220

Gly Glu Gln Glu Ser Thr Trp Glu Thr Ile Pro His Pro Ser Ile Glu
225                 230                 235                 240

Glu Phe Lys Tyr Gly Gly Pro Asn Gly Phe Leu Asp Leu Phe Thr Lys
                245                 250                 255

Asp Arg Ser Tyr Ala Lys Gln Trp Arg Tyr Thr Asn Ala Pro Asp Ala
            260                 265                 270

Glu Gly Arg Ala Ile Gln Ala Val Tyr Trp Ala Asn Lys Trp Ala Lys
        275                 280                 285

Glu Gln Gly Lys Gly Ser Ala Val Ala Ser Val Val Ser Lys Ala Ala
    290                 295                 300

Lys Met Gly Asp Phe Leu Arg Asn Asp Met Phe Asp Lys Tyr Phe Met
305                 310                 315                 320

Lys Ile Gly Ala Gln Asp Lys Thr Pro Ala Thr Gly Tyr Asp Ser Ala
                325                 330                 335

His Tyr Leu Met Ala Trp Tyr Thr Ala Trp Gly Gly Gly Ile Gly Ala
            340                 345                 350

Ser Trp Ala Trp Lys Ile Gly Cys Ser His Ala His Phe Gly Tyr Gln
        355                 360                 365

Asn Pro Phe Gln Gly Trp Val Ser Ala Thr Gln Ser Asp Phe Ala Pro
    370                 375                 380

Lys Ser Ser Asn Gly Lys Arg Asp Trp Thr Thr Ser Tyr Lys Arg Gln
385                 390                 395                 400

Leu Glu Phe Tyr Gln Trp Leu Gln Ser Ala Glu Gly Gly Ile Ala Gly
                405                 410                 415

Gly Ala Thr Asn Ser Trp Asn Gly Arg Tyr Glu Lys Tyr Pro Ala Gly
            420                 425                 430

Thr Ser Thr Phe Tyr Gly Met Ala Tyr Val Pro His Pro Val Tyr Ala
        435                 440                 445

Asp Pro Gly Ser Asn Gln Trp Phe Gly Phe Gln Ala Trp Ser Met Gln
    450                 455                 460

Arg Val Met Glu Tyr Tyr Leu Glu Thr Gly Asp Ser Ser Val Lys Asn
465                 470                 475                 480

Leu Ile Lys Lys Trp Val Asp Trp Val Met Ser Glu Ile Lys Leu Tyr
                485                 490                 495

Asp Asp Gly Thr Phe Ala Ile Pro Ser Asp Leu Glu Trp Ser Gly Gln
            500                 505                 510

Pro Asp Thr Trp Thr Gly Thr Tyr Thr Gly Asn Pro Asn Leu His Val
        515                 520                 525

Arg Val Thr Ser Tyr Gly Thr Asp Leu Gly Val Ala Gly Ser Leu Ala
    530                 535                 540

Asn Ala Leu Ala Thr Tyr Ala Ala Ala Thr Glu Arg Trp Glu Gly Lys
545                 550                 555                 560

Leu Asp Thr Lys Ala Arg Asp Met Ala Ala Glu Leu Val Asn Arg Ala
                565                 570                 575

Trp Tyr Asn Phe Tyr Cys Ser Glu Gly Lys Gly Val Val Thr Glu Glu
            580                 585                 590

Ala Arg Ala Asp Tyr Lys Arg Phe Phe Glu Gln Glu Val Tyr Val Pro
        595                 600                 605

Ala Gly Trp Ser Gly Thr Met Pro Asn Gly Asp Lys Ile Gln Pro Gly
    610                 615                 620

Ile Lys Phe Ile Asp Ile Arg Thr Lys Tyr Arg Gln Asp Pro Tyr Tyr
625                 630                 635                 640

Asp Ile Val Tyr Gln Ala Tyr Leu Arg Gly Glu Ala Pro Val Leu Asn
                645                 650                 655

Tyr His Arg Phe Trp His Glu Val Asp Leu Ala Val Ala Met Gly Val
            660                 665                 670

Leu Ala Thr Tyr Phe Pro Asp Met Thr Tyr Lys Val Pro Gly Thr Pro
        675                 680                 685

Ser Thr Lys Leu Tyr Gly Asp Val Asn Asp Asp Gly Lys Val Asn Ser
    690                 695                 700

Thr Asp Ala Val Ala Leu Lys Arg Tyr Val Leu Arg Ser Gly Ile Ser
705                 710                 715                 720

Ile Asn Thr Asp Asn Ala Asp Leu Asn Glu Asp Gly Arg Val Asn Ser
                725                 730                 735

Thr Asp Leu Gly Ile Leu Lys Arg Tyr Ile Leu Lys Glu Ile Asp Thr
            740                 745                 750

Leu Pro Tyr Lys Asn
        755

<210>  7
<211>  2655
<212>  DNA
<213>  Clostridium thermocellum

<220>
<223> CelK-d1

<400>  7
atgagaggat ctcaccatca ccatcaccat gggatccgca tgcgagctct ggaagacaag      60

tcttcaaagt tgccagatta taaaaacgac cttttgtatg aaagaacatt cgacgaaggt     120

ctttgctttc cgtggcatac ttgcgaagac agtggaggaa aatgtgattt cgctgttgtt     180

gatgttccag gagagcctgg gaacaaagct ttccgcttga cagtaattga caaaggacaa     240

aacaagtgga gtgtccagat gagacacaga ggtattaccc tcgagcaagg acatacatac     300

acggtaaggt ttacgatttg gtctgacaaa tcctgtaggg tttatgctaa aattggtcag     360

atgggtgaac cctatactga atattggaac aataactgga atccattcaa ccttacacca     420

ggacagaagc ttacagttga acagaatttt acaatgaact atcctactga tgacacatgc     480

gagttcacat tccatttggg tggagaactt gctgcaggta caccttacta tgtttacctt     540

gatgatgtat ctctctacga tcctaggttt gtaaagcctg ttgaatatgt acttccgcag     600

ccggatgtac gtgttaacca ggtaggatac ttgccgtttg caaagaagta tgctactgtt     660

gtatcttctt caaccagccc gcttaagtgg cagcttctca attcggcaaa tcaggttgtt     720

ttggaaggta atacaatacc aaaaggactt gacaaagatt cacaggatta tgtacattgg     780

atagatttct ccaactttaa gactgaagga aaaggttatt acttcaagct tccgactgta     840

aacagcgata caaattacag ccatcctttc gatatcagtg ctgatattta ctccaagatg     900

aaatttgatg cattggcatt cttctatcac aagagaagcg gtattcctat tgaaatgccg     960

tatgcaggag gagaacagtg gaccagacct gcaggacata ttggaattga gccgaacaag    1020

ggagatacaa atgttcctac atggcctcag gatgatgaat atgcaggaag acctcaaaaa    1080

tattatacaa aagatgtaac cggtggatgg tatgatgccg gtgaccacgg taaatatgtt    1140

gtaaacggcg gtatagctgt ttggacattg atgaacatgt atgaaagggc aaaaatcaga    1200

ggcatagcta atcaaggtgc ttataaagac ggtggaatga acataccgga gagaaataac    1260

ggttatccgg acattcttga tgaagcaaga tgggaaattg agttctttaa gaaaatgcag    1320

gtaactgaaa aagaggatcc ttccatagcc ggaatggtac accacaaaat tcacgacttc    1380

agatggactg ctttgggtat gttgcctcac gaagatcccc agccacgtta cttaaggccg    1440

gtaagtacgg ctgcgacttt gaactttgcg gcaactttgg cacaaagtgc acgtctttgg    1500

aaagattatg atccgacttt tgctgctgac tgtttggaaa aggctgaaat agcatggcag    1560

gcggcattaa agcatcctga tatttatgct gagtatactc ccggtagcgg tggtcccgga    1620

ggcggaccat acaatgacga ctatgtcgga gacgaattct actgggcagc ctgcgaactt 1680

tatgtaacaa caggaaaaga cgaatataag aattacctga tgaattcacc tcactatctt 1740

gaaatgcctg caaagatggg tgaaaacggt ggagcaaacg gagaagacaa cggattgtgg 1800

ggatgcttca cctggggaac tactcaagga ttgggaacca ttactcttgc attggttgaa 1860

aacggattgc ctgctacaga cattcaaaag gcaagaaaca atatagctaa agctgctgac 1920

agatggcttg agaatattga agagcaaggt tacagactgc cgatcaaaca ggcggaggat 1980

gagagaggcg gttatccatg gggttcaaac tccttcattt tgaaccagat gatagttatg 2040

ggatacgcat atgactttac aggcaacagc aagtatcttg acggaatgca ggatggtatg 2100

agctacctgt tgggaagaaa cggactggat cagtcctatg taacagggta tggtgagcgt 2160

ccacttcaga atcctcatga cagattctgg acgccgcaga caagtaagaa attccctgct 2220

ccacctccgg gtataattgc cggtggtccg aactcccgtt tcgaagaccc gacaataact 2280

gcagcagtta agaaggatac accgccgcag aagtgctaca ttgaccatac agactcatgg 2340

tcaaccaacg agataactgt taactggaat gctccgtttg catgggttac agcttatctc 2400

gatgaaattg acttaataac accgccagga ggagtagacc cagaagaacc ggaggttatt 2460

tatggtgact gcaatggcga cggaaaagtt aattcaactg acgctgtggc attgaagaga 2520

tatatcttga gatcaggtat aagcatcaac actgataatg ctgatgtaaa tgctgatggc 2580

agagttaact ctacagactt ggcaatattg aagagatata ttcttaaaga gatagatgta 2640

ttgccacata aataa 2655

<210> 8
<211> 884
<212> PRT
<213> Clostridium thermocellum

<220>
<223> CelK-d1

<400> 8

Met Arg Gly Ser His His His His His His Gly Ile Arg Met Arg Ala
1               5                   10                  15

Leu Glu Asp Lys Ser Ser Lys Leu Pro Asp Tyr Lys Asn Asp Leu Leu
            20                  25                  30

Tyr Glu Arg Thr Phe Asp Glu Gly Leu Cys Phe Pro Trp His Thr Cys
        35                  40                  45

Glu Asp Ser Gly Gly Lys Cys Asp Phe Ala Val Val Asp Val Pro Gly
    50                  55                  60

Glu Pro Gly Asn Lys Ala Phe Arg Leu Thr Val Ile Asp Lys Gly Gln
65                  70                  75                  80

Asn Lys Trp Ser Val Gln Met Arg His Arg Gly Ile Thr Leu Glu Gln
                85                  90                  95

Gly His Thr Tyr Thr Val Arg Phe Thr Ile Trp Ser Asp Lys Ser Cys
            100                 105                 110

Arg Val Tyr Ala Lys Ile Gly Gln Met Gly Glu Pro Tyr Thr Glu Tyr
        115                 120                 125

Trp Asn Asn Asn Trp Asn Pro Phe Asn Leu Thr Pro Gly Gln Lys Leu
    130                 135                 140

Thr Val Glu Gln Asn Phe Thr Met Asn Tyr Pro Thr Asp Asp Thr Cys
145                 150                 155                 160

Glu Phe Thr Phe His Leu Gly Gly Glu Leu Ala Ala Gly Thr Pro Tyr
                165                 170                 175

Tyr Val Tyr Leu Asp Asp Val Ser Leu Tyr Asp Pro Arg Phe Val Lys
            180                 185                 190

Pro Val Glu Tyr Val Leu Pro Gln Pro Asp Val Arg Val Asn Gln Val
        195                 200                 205

Gly Tyr Leu Pro Phe Ala Lys Lys Tyr Ala Thr Val Val Ser Ser Ser
    210                 215                 220

Thr Ser Pro Leu Lys Trp Gln Leu Leu Asn Ser Ala Asn Gln Val Val
225                 230                 235                 240

Leu Glu Gly Asn Thr Ile Pro Lys Gly Leu Asp Lys Asp Ser Gln Asp
                245                 250                 255

Tyr Val His Trp Ile Asp Phe Ser Asn Phe Lys Thr Glu Gly Lys Gly
            260                 265                 270

Tyr Tyr Phe Lys Leu Pro Thr Val Asn Ser Asp Thr Asn Tyr Ser His
        275                 280                 285

Pro Phe Asp Ile Ser Ala Asp Ile Tyr Ser Lys Met Lys Phe Asp Ala
    290                 295                 300

Leu Ala Phe Phe Tyr His Lys Arg Ser Gly Ile Pro Ile Glu Met Pro
305                 310                 315                 320

Tyr Ala Gly Gly Glu Gln Trp Thr Arg Pro Ala Gly His Ile Gly Ile
                325                 330                 335

Glu Pro Asn Lys Gly Asp Thr Asn Val Pro Thr Trp Pro Gln Asp Asp
            340                 345                 350

Glu Tyr Ala Gly Arg Pro Gln Lys Tyr Tyr Thr Lys Asp Val Thr Gly
        355                 360                 365

Gly Trp Tyr Asp Ala Gly Asp His Gly Lys Tyr Val Val Asn Gly Gly
    370                 375                 380

Ile Ala Val Trp Thr Leu Met Asn Met Tyr Glu Arg Ala Lys Ile Arg
385                 390                 395                 400

Gly Ile Ala Asn Gln Gly Ala Tyr Lys Asp Gly Gly Met Asn Ile Pro
                405                 410                 415

Glu Arg Asn Asn Gly Tyr Pro Asp Ile Leu Asp Glu Ala Arg Trp Glu
            420                 425                 430

Ile Glu Phe Phe Lys Lys Met Gln Val Thr Glu Lys Glu Asp Pro Ser
        435                 440                 445

Ile Ala Gly Met Val His His Lys Ile His Asp Phe Arg Trp Thr Ala
    450                 455                 460

Leu Gly Met Leu Pro His Glu Asp Pro Gln Pro Arg Tyr Leu Arg Pro
465                 470                 475                 480

Val Ser Thr Ala Ala Thr Leu Asn Phe Ala Ala Thr Leu Ala Gln Ser
                485                 490                 495

Ala Arg Leu Trp Lys Asp Tyr Asp Pro Thr Phe Ala Ala Asp Cys Leu
            500                 505                 510

Glu Lys Ala Glu Ile Ala Trp Gln Ala Ala Leu Lys His Pro Asp Ile
        515                 520                 525

Tyr Ala Glu Tyr Thr Pro Gly Ser Gly Gly Pro Gly Gly Gly Pro Tyr
    530                 535                 540

Asn Asp Asp Tyr Val Gly Asp Glu Phe Tyr Trp Ala Ala Cys Glu Leu
545                 550                 555                 560

Tyr Val Thr Thr Gly Lys Asp Glu Tyr Lys Asn Tyr Leu Met Asn Ser
                565                 570                 575

Pro His Tyr Leu Glu Met Pro Ala Lys Met Gly Glu Asn Gly Gly Ala
            580                 585                 590

Asn Gly Glu Asp Asn Gly Leu Trp Gly Cys Phe Thr Trp Gly Thr Thr
        595                 600                 605

Gln Gly Leu Gly Thr Ile Thr Leu Ala Leu Val Glu Asn Gly Leu Pro
    610                 615                 620

Ala Thr Asp Ile Gln Lys Ala Arg Asn Asn Ile Ala Lys Ala Ala Asp
625                 630                 635                 640

Arg Trp Leu Glu Asn Ile Glu Glu Gln Gly Tyr Arg Leu Pro Ile Lys
                645                 650                 655

Gln Ala Glu Asp Glu Arg Gly Gly Tyr Pro Trp Gly Ser Asn Ser Phe
            660                 665                 670

Ile Leu Asn Gln Met Ile Val Met Gly Tyr Ala Tyr Asp Phe Thr Gly
        675                 680                 685

Asn Ser Lys Tyr Leu Asp Gly Met Gln Asp Gly Met Ser Tyr Leu Leu
    690                 695                 700

Gly Arg Asn Gly Leu Asp Gln Ser Tyr Val Thr Gly Tyr Gly Glu Arg
705                 710                 715                 720

Pro Leu Gln Asn Pro His Asp Arg Phe Trp Thr Pro Gln Thr Ser Lys
                725                 730                 735

Lys Phe Pro Ala Pro Pro Pro Gly Ile Ile Ala Gly Gly Pro Asn Ser
            740                 745                 750

Arg Phe Glu Asp Pro Thr Ile Thr Ala Ala Val Lys Lys Asp Thr Pro
        755                 760                 765

Pro Gln Lys Cys Tyr Ile Asp His Thr Asp Ser Trp Ser Thr Asn Glu
    770                 775                 780

Ile Thr Val Asn Trp Asn Ala Pro Phe Ala Trp Val Thr Ala Tyr Leu
785                 790                 795                 800

Asp Glu Ile Asp Leu Ile Thr Pro Pro Gly Gly Val Asp Pro Glu Glu
                805                 810                 815

Pro Glu Val Ile Tyr Gly Asp Cys Asn Gly Asp Gly Lys Val Asn Ser
            820                 825                 830

Thr Asp Ala Val Ala Leu Lys Arg Tyr Ile Leu Arg Ser Gly Ile Ser
        835                 840                 845

Ile Asn Thr Asp Asn Ala Asp Val Asn Ala Asp Gly Arg Val Asn Ser
    850                 855                 860

Thr Asp Leu Ala Ile Leu Lys Arg Tyr Ile Leu Lys Glu Ile Asp Val
865                 870                 875                 880

Leu Pro His Lys

<210> 9
<211> 2181
<212> DNA
<213> Clostridium thermocellum

<220>
<223> CelR-d1

<400> 9
atgagaggat ctcaccatca ccatcaccat acggatcctg tttttgcagc agactataac 60

tatggagaag cactccaaaa agcaattatg ttctatgaat ttcaaatgtc cggaaagctt 120

cccgacaaca tccgtaacaa ctggcgcggt gattcatgtc tcggagacgg aagcgatgta 180

ggtcttgacc tcacaggagg ttggtttgac gccggtgacc atgtaaaatt caatctgcct 240

atggcttaca cagccactat gcttgcatgg gctgtgtatg agtacaagga cgcgttacaa 300

aaaagcggtc aattgggcta tttaatggat cagattaaat gggcatcgga ctacttcata 360

agatgccatc ccgaaaaata tgtatattat tatcaagtgg gtaacggtga catggaccac 420

agatggtggg tgccggcaga atgtatagat gttcaggcac caagaccgtc ttacaaagta 480

gatctgtcaa atcccggttc cacagttact gcgggtacag ctgccgcact tgctgcaact 540

gccttggtat tcaaaggcac tgatccggca tatgccgctc tgtgcatacg tcatgcagaa 600

gaactctttg attttgctga aaccactatg agtgataaag gatataccgc agcattgaat 660

ttctacacat ctcacagtgg atggtatgac gagctttcct gggcaggtgc atggatttat 720

cttgcagacg gtgacgaaac ttatcttgaa aaagctgaaa agtatgtgga taaatggcca 780

atcgaaagcc agacaactta cattgcttat tcatggggtc actgctggga cgacgttcac 840

tacggagcag cacttctttt ggcaaagatt acaaacaaat ccttatacaa agaagcgata 900

gaaagacacc tggactattg gacagttgga tttaatggtc agagagtcag atatacacca 960

aagggtcttg ctcacctcac tgactggggt gtattaagac atgccactac tactgcattc 1020

cttgcatgtg tttattccga ctggtcagaa tgtccaaggg aaaaagccaa tatttacata 1080

gattttgcca agaaacaggc tgactatgcc ttaggcagca gcggcagaag ttatgtagtc 1140

ggatttggtg taaatcctcc gcagcatccg caccacagaa ctgcccacag ctcatggtgt 1200

gacagtcaaa aagttcctga ataccacaga cacgttcttt acggagcact cgtaggcgga 1260

cctgatgcca gcgatgctta tgttgatgat ataggaaact atgtaacaaa tgaggttgcc 1320

tgcgactaca atgccggttt tgtaggattg ctcgccaaga tgtatgaaaa atatggcgga 1380

aaccccatac caaacttcat ggctatagaa gaaaaaacaa atgaagaaat ttatgttgaa 1440

gctaccgcca attcaaataa cggtgtcgaa ttgaaaacat acctttacaa taaatccgga 1500

tggccggcaa gagtttgcga caagctttcc ttcagatatt tcatggacct tacggaatat 1560

gtatccgccg gatacaatcc taatgatata actgtttcta taatttacag tgcagcacca 1620

actgcaaaaa tttcaaaacc aatactttat gacgcatcca aaaacatata ttattgcgaa 1680

atcgatctct ccggtaccaa gatattcccc ggaagcaact cagaccacca gaaagaaacc 1740

caatttagaa tacagcctcc tgcaggcgca ccttgggaca acaccaacga cttctcctat 1800

cagggaatca agaaaaacgg tgaagttgta aaagaaatgc ctgtttatga agacggagtt 1860

ctcatattcg gtgtagaacc caatggtacc ggtcctgcaa caccaacgcc gaaaccgtcc 1920

gtaaatcctt caccttcacc tacgccaaca tcggatattc tttacggtga catcaatctg 1980

gacggaaaaa ttaactcttc agatgttaca ctgttaaaaa gatatattgt gaagtccata 2040

gatgttttcc caaccgctga tccggaacgg agcttaatag catcagatgt aaacggagac 2100

ggaagggtaa actctacaga ctattcatac cttaaacgtt atgtcttgaa aatcatacca 2160

accatacccg gaaattcatg a 2181

<210> 10
<211> 726
<212> PRT
<213> Clostridium thermocellum

<220>
<223> CelR-d1

<400> 10

Met Arg Gly Ser His His His His His His Thr Asp Pro Val Phe Ala
1               5                   10                  15

Ala Asp Tyr Asn Tyr Gly Glu Ala Leu Gln Lys Ala Ile Met Phe Tyr
            20                  25                  30

Glu Phe Gln Met Ser Gly Lys Leu Pro Asp Asn Ile Arg Asn Asn Trp
        35                  40                  45

Arg Gly Asp Ser Cys Leu Gly Asp Gly Ser Asp Val Gly Leu Asp Leu
    50                  55                  60

Thr Gly Gly Trp Phe Asp Ala Gly Asp His Val Lys Phe Asn Leu Pro
65                  70                  75                  80

Met Ala Tyr Thr Ala Thr Met Leu Ala Trp Ala Val Tyr Glu Tyr Lys
                85                  90                  95

Asp Ala Leu Gln Lys Ser Gly Gln Leu Gly Tyr Leu Met Asp Gln Ile
            100                 105                 110

Lys Trp Ala Ser Asp Tyr Phe Ile Arg Cys His Pro Glu Lys Tyr Val
        115                 120                 125

Tyr Tyr Tyr Gln Val Gly Asn Gly Asp Met Asp His Arg Trp Trp Val
    130                 135                 140

Pro Ala Glu Cys Ile Asp Val Gln Ala Pro Arg Pro Ser Tyr Lys Val
145                 150                 155                 160

Asp Leu Ser Asn Pro Gly Ser Thr Val Thr Ala Gly Thr Ala Ala Ala
                165                 170                 175

Leu Ala Ala Thr Ala Leu Val Phe Lys Gly Thr Asp Pro Ala Tyr Ala
            180                 185                 190

Ala Leu Cys Ile Arg His Ala Glu Glu Leu Phe Asp Phe Ala Glu Thr
        195                 200                 205

Thr Met Ser Asp Lys Gly Tyr Thr Ala Ala Leu Asn Phe Tyr Thr Ser
    210                 215                 220

His Ser Gly Trp Tyr Asp Glu Leu Ser Trp Ala Gly Ala Trp Ile Tyr
225                 230                 235                 240

Leu Ala Asp Gly Asp Glu Thr Tyr Leu Glu Lys Ala Glu Lys Tyr Val
                245                 250                 255

Asp Lys Trp Pro Ile Glu Ser Gln Thr Thr Tyr Ile Ala Tyr Ser Trp
            260                 265                 270

Gly His Cys Trp Asp Asp Val His Tyr Gly Ala Ala Leu Leu Leu Ala
        275                 280                 285

Lys Ile Thr Asn Lys Ser Leu Tyr Lys Glu Ala Ile Glu Arg His Leu
    290                 295                 300

Asp Tyr Trp Thr Val Gly Phe Asn Gly Gln Arg Val Arg Tyr Thr Pro
305                 310                 315                 320

Lys Gly Leu Ala His Leu Thr Asp Trp Gly Val Leu Arg His Ala Thr
                325                 330                 335

Thr Thr Ala Phe Leu Ala Cys Val Tyr Ser Asp Trp Ser Glu Cys Pro
            340                 345                 350

Arg Glu Lys Ala Asn Ile Tyr Ile Asp Phe Ala Lys Lys Gln Ala Asp
        355                 360                 365

Tyr Ala Leu Gly Ser Ser Gly Arg Ser Tyr Val Val Gly Phe Gly Val
    370                 375                 380

Asn Pro Pro Gln His Pro His His Arg Thr Ala His Ser Ser Trp Cys
385                 390                 395                 400

Asp Ser Gln Lys Val Pro Glu Tyr His Arg His Val Leu Tyr Gly Ala
                405                 410                 415

Leu Val Gly Gly Pro Asp Ala Ser Asp Ala Tyr Val Asp Asp Ile Gly
            420                 425                 430

Asn Tyr Val Thr Asn Glu Val Ala Cys Asp Tyr Asn Ala Gly Phe Val
        435                 440                 445

Gly Leu Leu Ala Lys Met Tyr Glu Lys Tyr Gly Gly Asn Pro Ile Pro
    450                 455                 460

Asn Phe Met Ala Ile Glu Glu Lys Thr Asn Glu Glu Ile Tyr Val Glu
465                 470                 475                 480

Ala Thr Ala Asn Ser Asn Asn Gly Val Glu Leu Lys Thr Tyr Leu Tyr
                485                 490                 495

Asn Lys Ser Gly Trp Pro Ala Arg Val Cys Asp Lys Leu Ser Phe Arg
            500                 505                 510

Tyr Phe Met Asp Leu Thr Glu Tyr Val Ser Ala Gly Tyr Asn Pro Asn
        515                 520                 525

Asp Ile Thr Val Ser Ile Ile Tyr Ser Ala Ala Pro Thr Ala Lys Ile
    530                 535                 540

Ser Lys Pro Ile Leu Tyr Asp Ala Ser Lys Asn Ile Tyr Tyr Cys Glu
545                 550                 555                 560

Ile Asp Leu Ser Gly Thr Lys Ile Phe Pro Gly Ser Asn Ser Asp His
                565                 570                 575

Gln Lys Glu Thr Gln Phe Arg Ile Gln Pro Pro Ala Gly Ala Pro Trp
            580                 585                 590

Asp Asn Thr Asn Asp Phe Ser Tyr Gln Gly Ile Lys Lys Asn Gly Glu
        595                 600                 605

Val Val Lys Glu Met Pro Val Tyr Glu Asp Gly Val Leu Ile Phe Gly
    610                 615                 620

Val Glu Pro Asn Gly Thr Gly Pro Ala Thr Pro Thr Pro Lys Pro Ser
625                 630                 635                 640

Val Asn Pro Ser Pro Ser Pro Thr Pro Thr Ser Asp Ile Leu Tyr Gly
                645                 650                 655

Asp Ile Asn Leu Asp Gly Lys Ile Asn Ser Ser Asp Val Thr Leu Leu
            660                 665                 670

Lys Arg Tyr Ile Val Lys Ser Ile Asp Val Phe Pro Thr Ala Asp Pro
        675                 680                 685

Glu Arg Ser Leu Ile Ala Ser Asp Val Asn Gly Asp Gly Arg Val Asn
    690                 695                 700

Ser Thr Asp Tyr Ser Tyr Leu Lys Arg Tyr Val Leu Lys Ile Ile Pro
705                 710                 715                 720

Thr Ile Pro Gly Asn Ser
                725
<210>  11
<211>  4845
<212>  DNA
<213>  Clostridium thermocellum

<220>
<223> CelJ-d1

<400>  11
atgagaggat ctcaccatca ccatcaccat gggatccgca tgccaaagag aagattatcg      60

ctacttttgg tacttgccat aatgtttacg atggtcgttc cacagatatc tgcaagtgcc     120

gaaacagttg ctcctgaagg ctacaggaag cttttggatg tacaaatttt caaggattcg     180

cctgtagtcg gatggtcagg aagcggtatg ggcgagcttg aaactatcgg cgataccctt     240

ccggttgata ccacagttac atataacggt ttgccgactt taagactgaa tgtccagaca     300

accgttcagt caggatggtg gatttctctt cttacattaa gaggatggaa cacccatgac     360

ctttcccagt atgtcgaaaa cggttatctt gagtttgaca tcaagggtaa ggaaggcgga     420

gaagactttg ttattggttt cagggacaag gtttatgaac gcgtttacgg acttgaaatt     480

gatgttacca cagtaatatc aaattatgta acggtaacta cggactggca gcatgttaag     540

attcctttga gagacctgat gaagattaat aacggatttg atccttcatc agttacatgc     600

ctggtgttct caaaaagata tgcagatccg tttacagtat ggttcagtga tataaagatt     660

acatcagaag acaatgaaaa gtccgctcct gcaatcaagg taaaccagct tggctttatt     720

cctgaagctg aaaaatacgc tttggttaca ggttttgcag aagagctcgc agtatcggaa     780

ggtgacgaat ttgccgttat aaatgctgcg gacaattctg ttgcttatac cggaaaatta     840

actcttgtaa cagaatatga acctcttgat tccggagaaa aaatacttaa ggcagatttc     900

agcgacttga ctgtacctgg caaatactac attagtattg aaggtcttga caattcaccc     960

aagtttgaaa tcggtgaagg tatttacggt ccactggttg ttgacgctgc aagatatttc    1020

tattatcagc gtcagggtat agaacttgaa gagccttatg cgcagggata tccccgcaag    1080

gacgttactc ctcaggacgc atatgctgta tttgcatccg gaaagaagga tccgattgac    1140

ataacaaagg gttggtatga cgcaggagac ttcggtaagt atgtaaatgc cggagcaacc    1200

ggtgtttccg atttgttctg ggcatatgaa atgttccctt cccagtttgt tgacggtcag    1260

ttcaatattc ctgaaagcgg aaacggtgta ccggacatcc ttgacgaagc tcgctgggag    1320

cttgaatgga tgctgaaaat gcaggacaaa gaaagcggag gattctatcc cagagttcaa    1380

tctgacaatg acgaaaacat aaaatcaaga ataatcaggg atcagaacgg ctgtaccact    1440

gatgatactg catgtgccgc cggaatactt gctcatgcat acttgattta caaggatatt    1500

gaccctgatt ttgcacaaga gtgcctggat gcggcaataa atgcatggaa attccttgaa    1560

aagaatcctg aaaacattgt ttcacctccg ggtccataca acgtatatga cgacagcgga 1620
gacagactct gggctgcagc ttcgctgtac agagctaccg gtgaagaggt ttatcataca 1680
tactttaaac aaaactacaa atcttttgca caaaagttcg aaagcccgac tgcatatgct 1740
catacatggg gtgatatgtg gcttacggca ttcctttcgt atttgaaagc tgaaaacaag 1800
gatcaggaag ttgtagactg gattgataca gagtttggaa tctggcttga aaacatactc 1860
acaagatatg agaacaatcc atggaagaat gcaattgttc ccggaaacta cttctgggga 1920
atcaacatgc aggttatgaa tgttccgatg gatgctatca taggttcaca gcttcttgga 1980
aaatacagtg acagaataga aaaattaggt tttggttcac ttaactggct gcttggtaca 2040
aatccgcttc gcttcagctt tgtatcagga tatggagagg attctgtaaa aggagtattc 2100
agcaatattt acaatacgga cggcaagcag ggaattccga aaggatacat gcctggtgga 2160
ccaaatgctt atgaaggtgc aggcctgtca aggtttgcag caaaatgcta caccagaagt 2220
accggtgact gggtagccaa cgaacataca gtatattgga actcagcttt ggtatttatg 2280
gctgcttttg caaaccaggg ttcagaggtt aatccgggac ctgcgccgga accgggagta 2340
actccgaatc ctacagaacc tgcaaaagtg gttgacatca ggatagatac ttctgctgaa 2400
agaaagccaa tcagcccgta tatatacgga agcaatcagg aacttgatgc aacagttact 2460
gcaaagaggt tcggcggaaa cagaactaca ggatacaact gggaaaacaa cttctcaaat 2520
gcaggaagtg actggctgca ttacagtgat acataccttt tggaggacgg cggagttcct 2580
aagggagagt ggagtacacc tgcttctgta gttaccacgt tccatgacaa ggcacttagc 2640
aaaaatgttc cttacacact tatcactctt caggcagcag gttatgtttc cgcagacgga 2700
aacggaccgg tttcccagga agaaactgca ccgtcttcaa gatggaagga agttaagttt 2760
gaaaagggag cacctttctc acttacaccg gacacagaag atgattatgt ttacatggat 2820
gagtttgtaa actatcttgt aaacaaatac ggaaatgcat ccacacctac aggaataaag 2880
ggttattcaa tagataacga gccggcattg tggagtcata ctcatccgag aattcatccg 2940
gacaatgtaa ctgccaaaga gcttattgaa aaatctgtag ctctttccaa ggcggttaaa 3000
aaggtagatc catatgcaga aatattcgga cctgctttgt acggatttgc cgcatatgag 3060
acacttcagt cagctcctga ctggggaact gaaggagaag gatacaggtg gtttatagat 3120
tattacctcg ataagatgaa aaaggcttct gatgaagaag gaaagagact tttggacgta 3180
cttgacgtac actggtatcc ggaagccagg ggcggcggtg aaagaatatg ctttggagcc 3240
gatccaagaa atattgagac aaacaaagca agattgcagg cgcccagaac attgtgggat 3300
cctacatata ttgaagacag ctggatagga caatggaaga aggatttcct cccgatatta 3360
cctaatcttt tggattccat tgaaaaatat tatccgggaa cgaagcttgc tataactgaa 3420
tatgactatg gcggaggaaa tcatattaca ggcggtattg ctcaagccga tgttcttggt 3480
atattcggta aatacggtgt ttaccttgca acattctggg gagatgcaag caataactat 3540
actgaggccg gtataaacct ttataccaac tacgacggca aaggcggcaa atttggagat 3600

acatccgtaa aatgtgaaac gtccgacata gaagtaagct ctgcttatgc atccattgtc 3660

ggtgaagatg acagcaaact ccatatcatt cttttgaaca agaactatga ccagccgacg 3720

acattcaatt tctcaattga cagcagcaag aactacacaa taggaaatgt atgggcattt 3780

gacagaggaa gctccaatat tactcaaaga actcctatag tgaacataaa ggacaatacc 3840

ttcacatata cagtaccggc tttgacagcg tgccatattg tgcttgaagc tgcggagccc 3900

gtagtgtacg gagacttgaa caatgactct aaagtaaacg cagtagacat tatgatgctc 3960

aaacgatata ttctcggaat aatagataat ataaatctga cagcagctga catttatttt 4020

gacggtgttg taaattcaag tgactataat ataatgaaga gatatttgtt aaaggcaata 4080

gaagatattc cttatgttcc ggaaaaccag gcacctaaag caatatttac tttctcgccc 4140

gaagacccgg ttactgacga gaatgtagtg ttcaatgcat caaattcaat agatgaagac 4200

ggaacaattg cctattatgc atgggatttc ggtgacggat atgaaggaac ttcaacaaca 4260

ccgactatta cctataagta taaaaacccc ggaacataca aagtaaaact gattgttaca 4320

gacaaccagg gggcttcaag ttcgtttaca gctaccataa aagtaacctc agctaccggg 4380

gacaattcca aattcaactt tgaagacggc acgctgggag gatttacaac atccggaaca 4440

aatgctacgg gtgttgttgt gaacactact gaaaaagcat tcaaaggcga aagaggtctt 4500

aaatggactg taacaagcga aggagaagga actgcagaat tgaaacttga cggaggtact 4560

attgtagttc ccggtaccac tatgacgttt agaatctgga taccttccgg tgcgcctatt 4620

gctgccatcc agccgtatat tatgcctcat acacctgatt ggtcggaagt cctctggaat 4680

tcgacatgga aaggatacac catggtgaag accgatgact ggaatgaaat taccctgaca 4740

ctgccggaag acgtggatcc gacttggccg cagcagatgg gtatacaggt acagaccata 4800

gatgaaggtg aattcactat ctatgtagat gctattgact ggtaa 4845

<210> 12
<211> 1614
<212> PRT
<213> Clostridium thermocellum

<220>
<223> CelJ-d1

<400> 12

Met Arg Gly Ser His His His His His His Gly Ile Arg Met Pro Lys
1               5                   10                  15

Arg Arg Leu Ser Leu Leu Leu Val Leu Ala Ile Met Phe Thr Met Val
            20                  25                  30

Val Pro Gln Ile Ser Ala Ser Ala Glu Thr Val Ala Pro Glu Gly Tyr
        35                  40                  45

Arg Lys Leu Leu Asp Val Gln Ile Phe Lys Asp Ser Pro Val Val Gly

50                  55                  60

Trp Ser Gly Ser Gly Met Gly Glu Leu Glu Thr Ile Gly Asp Thr Leu
65                  70                  75                  80

Pro Val Asp Thr Thr Val Thr Tyr Asn Gly Leu Pro Thr Leu Arg Leu
                85                  90                  95

Asn Val Gln Thr Thr Val Gln Ser Gly Trp Trp Ile Ser Leu Leu Thr
            100                 105                 110

Leu Arg Gly Trp Asn Thr His Asp Leu Ser Gln Tyr Val Glu Asn Gly
        115                 120                 125

Tyr Leu Glu Phe Asp Ile Lys Gly Lys Glu Gly Gly Glu Asp Phe Val
    130                 135                 140

Ile Gly Phe Arg Asp Lys Val Tyr Glu Arg Val Tyr Gly Leu Glu Ile
145                 150                 155                 160

Asp Val Thr Thr Val Ile Ser Asn Tyr Val Thr Val Thr Thr Asp Trp
                165                 170                 175

Gln His Val Lys Ile Pro Leu Arg Asp Leu Met Lys Ile Asn Asn Gly
            180                 185                 190

Phe Asp Pro Ser Ser Val Thr Cys Leu Val Phe Ser Lys Arg Tyr Ala
        195                 200                 205

Asp Pro Phe Thr Val Trp Phe Ser Asp Ile Lys Ile Thr Ser Glu Asp
    210                 215                 220

Asn Glu Lys Ser Ala Pro Ala Ile Lys Val Asn Gln Leu Gly Phe Ile
225                 230                 235                 240

Pro Glu Ala Glu Lys Tyr Ala Leu Val Thr Gly Phe Ala Glu Glu Leu
                245                 250                 255

Ala Val Ser Glu Gly Asp Glu Phe Ala Val Ile Asn Ala Ala Asp Asn
            260                 265                 270

Ser Val Ala Tyr Thr Gly Lys Leu Thr Leu Val Thr Glu Tyr Glu Pro
        275                 280                 285

Leu Asp Ser Gly Glu Lys Ile Leu Lys Ala Asp Phe Ser Asp Leu Thr
    290                 295                 300

Val Pro Gly Lys Tyr Tyr Ile Ser Ile Glu Gly Leu Asp Asn Ser Pro
305                 310                 315                 320

Lys Phe Glu Ile Gly Glu Gly Ile Tyr Gly Pro Leu Val Val Asp Ala

                325                 330                 335

Ala Arg Tyr Phe Tyr Tyr Gln Arg Gln Gly Ile Glu Leu Glu Glu Pro
            340                 345                 350

Tyr Ala Gln Gly Tyr Pro Arg Lys Asp Val Thr Pro Gln Asp Ala Tyr
        355                 360                 365

Ala Val Phe Ala Ser Gly Lys Lys Asp Pro Ile Asp Ile Thr Lys Gly
    370                 375                 380

Trp Tyr Asp Ala Gly Asp Phe Gly Lys Tyr Val Asn Ala Gly Ala Thr
385                 390                 395                 400

Gly Val Ser Asp Leu Phe Trp Ala Tyr Glu Met Phe Pro Ser Gln Phe
                405                 410                 415

Val Asp Gly Gln Phe Asn Ile Pro Glu Ser Gly Asn Gly Val Pro Asp
            420                 425                 430

Ile Leu Asp Glu Ala Arg Trp Glu Leu Glu Trp Met Leu Lys Met Gln
        435                 440                 445

Asp Lys Glu Ser Gly Gly Phe Tyr Pro Arg Val Gln Ser Asp Asn Asp
    450                 455                 460

Glu Asn Ile Lys Ser Arg Ile Ile Arg Asp Gln Asn Gly Cys Thr Thr
465                 470                 475                 480

Asp Asp Thr Ala Cys Ala Ala Gly Ile Leu Ala His Ala Tyr Leu Ile
                485                 490                 495

Tyr Lys Asp Ile Asp Pro Asp Phe Ala Gln Glu Cys Leu Asp Ala Ala
            500                 505                 510

Ile Asn Ala Trp Lys Phe Leu Glu Lys Asn Pro Glu Asn Ile Val Ser
        515                 520                 525

Pro Pro Gly Pro Tyr Asn Val Tyr Asp Asp Ser Gly Asp Arg Leu Trp
    530                 535                 540

Ala Ala Ala Ser Leu Tyr Arg Ala Thr Gly Glu Glu Val Tyr His Thr
545                 550                 555                 560

Tyr Phe Lys Gln Asn Tyr Lys Ser Phe Ala Gln Lys Phe Glu Ser Pro
                565                 570                 575

Thr Ala Tyr Ala His Thr Trp Gly Asp Met Trp Leu Thr Ala Phe Leu
            580                 585                 590

Ser Tyr Leu Lys Ala Glu Asn Lys Asp Gln Glu Val Val Asp Trp Ile

595                 600                 605

Asp Thr Glu Phe Gly Ile Trp Leu Glu Asn Ile Leu Thr Arg Tyr Glu
    610                 615                 620

Asn Asn Pro Trp Lys Asn Ala Ile Val Pro Gly Asn Tyr Phe Trp Gly
625                 630                 635                 640

Ile Asn Met Gln Val Met Asn Val Pro Met Asp Ala Ile Ile Gly Ser
                645                 650                 655

Gln Leu Leu Gly Lys Tyr Ser Asp Arg Ile Glu Lys Leu Gly Phe Gly
            660                 665                 670

Ser Leu Asn Trp Leu Leu Gly Thr Asn Pro Leu Arg Phe Ser Phe Val
        675                 680                 685

Ser Gly Tyr Gly Glu Asp Ser Val Lys Gly Val Phe Ser Asn Ile Tyr
    690                 695                 700

Asn Thr Asp Gly Lys Gln Gly Ile Pro Lys Gly Tyr Met Pro Gly Gly
705                 710                 715                 720

Pro Asn Ala Tyr Glu Gly Ala Gly Leu Ser Arg Phe Ala Ala Lys Cys
                725                 730                 735

Tyr Thr Arg Ser Thr Gly Asp Trp Val Ala Asn Glu His Thr Val Tyr
            740                 745                 750

Trp Asn Ser Ala Leu Val Phe Met Ala Ala Phe Ala Asn Gln Gly Ser
        755                 760                 765

Glu Val Asn Pro Gly Pro Ala Pro Glu Pro Gly Val Thr Pro Asn Pro
    770                 775                 780

Thr Glu Pro Ala Lys Val Val Asp Ile Arg Ile Asp Thr Ser Ala Glu
785                 790                 795                 800

Arg Lys Pro Ile Ser Pro Tyr Ile Tyr Gly Ser Asn Gln Glu Leu Asp
                805                 810                 815

Ala Thr Val Thr Ala Lys Arg Phe Gly Gly Asn Arg Thr Thr Gly Tyr
            820                 825                 830

Asn Trp Glu Asn Asn Phe Ser Asn Ala Gly Ser Asp Trp Leu His Tyr
        835                 840                 845

Ser Asp Thr Tyr Leu Leu Glu Asp Gly Gly Val Pro Lys Gly Glu Trp
    850                 855                 860

Ser Thr Pro Ala Ser Val Val Thr Thr Phe His Asp Lys Ala Leu Ser

865                    870                    875                    880

Lys Asn Val Pro Tyr Thr Leu Ile Thr Leu Gln Ala Ala Gly Tyr Val
                885                    890                    895

Ser Ala Asp Gly Asn Gly Pro Val Ser Gln Glu Glu Thr Ala Pro Ser
            900                    905                    910

Ser Arg Trp Lys Glu Val Lys Phe Glu Lys Gly Ala Pro Phe Ser Leu
        915                    920                    925

Thr Pro Asp Thr Glu Asp Asp Tyr Val Tyr Met Asp Glu Phe Val Asn
    930                    935                    940

Tyr Leu Val Asn Lys Tyr Gly Asn Ala Ser Thr Pro Thr Gly Ile Lys
945                    950                    955                    960

Gly Tyr Ser Ile Asp Asn Glu Pro Ala Leu Trp Ser His Thr His Pro
                965                    970                    975

Arg Ile His Pro Asp Asn Val Thr Ala Lys Glu Leu Ile Glu Lys Ser
            980                    985                    990

Val Ala Leu Ser Lys Ala Val Lys  Lys Val Asp Pro Tyr  Ala Glu Ile
        995                    1000                    1005

Phe Gly  Pro Ala Leu Tyr Gly  Phe Ala Ala Tyr Glu  Thr Leu Gln
    1010                    1015                    1020

Ser Ala  Pro Asp Trp Gly Thr  Glu Gly Glu Gly Tyr  Arg Trp Phe
    1025                    1030                    1035

Ile Asp  Tyr Tyr Leu Asp Lys  Met Lys Lys Ala Ser  Asp Glu Glu
    1040                    1045                    1050

Gly Lys  Arg Leu Leu Asp Val  Leu Asp Val His Trp  Tyr Pro Glu
    1055                    1060                    1065

Ala Arg  Gly Gly Gly Glu Arg  Ile Cys Phe Gly Ala  Asp Pro Arg
    1070                    1075                    1080

Asn Ile  Glu Thr Asn Lys Ala  Arg Leu Gln Ala Pro  Arg Thr Leu
    1085                    1090                    1095

Trp Asp  Pro Thr Tyr Ile Glu  Asp Ser Trp Ile Gly  Gln Trp Lys
    1100                    1105                    1110

Lys Asp  Phe Leu Pro Ile Leu  Pro Asn Leu Leu Asp  Ser Ile Glu
    1115                    1120                    1125

Lys Tyr  Tyr Pro Gly Thr Lys  Leu Ala Ile Thr Glu  Tyr Asp Tyr

1130                 1135                 1140

Gly Gly  Gly Asn His Ile Thr  Gly Gly Ile Ala Gln  Ala Asp Val
    1145                 1150                 1155

Leu Gly  Ile Phe Gly Lys Tyr  Gly Val Tyr Leu Ala  Thr Phe Trp
    1160                 1165                 1170

Gly Asp  Ala Ser Asn Asn Tyr  Thr Glu Ala Gly Ile  Asn Leu Tyr
    1175                 1180                 1185

Thr Asn  Tyr Asp Gly Lys Gly  Gly Lys Phe Gly Asp  Thr Ser Val
    1190                 1195                 1200

Lys Cys  Glu Thr Ser Asp Ile  Glu Val Ser Ser Ala  Tyr Ala Ser
    1205                 1210                 1215

Ile Val  Gly Glu Asp Asp Ser  Lys Leu His Ile Ile  Leu Leu Asn
    1220                 1225                 1230

Lys Asn  Tyr Asp Gln Pro Thr  Thr Phe Asn Phe Ser  Ile Asp Ser
    1235                 1240                 1245

Ser Lys  Asn Tyr Thr Ile Gly  Asn Val Trp Ala Phe  Asp Arg Gly
    1250                 1255                 1260

Ser Ser  Asn Ile Thr Gln Arg  Thr Pro Ile Val Asn  Ile Lys Asp
    1265                 1270                 1275

Asn Thr  Phe Thr Tyr Thr Val  Pro Ala Leu Thr Ala  Cys His Ile
    1280                 1285                 1290

Val Leu  Glu Ala Ala Glu Pro  Val Val Tyr Gly Asp  Leu Asn Asn
    1295                 1300                 1305

Asp Ser  Lys Val Asn Ala Val  Asp Ile Met Met Leu  Lys Arg Tyr
    1310                 1315                 1320

Ile Leu  Gly Ile Ile Asp Asn  Ile Asn Leu Thr Ala  Ala Asp Ile
    1325                 1330                 1335

Tyr Phe  Asp Gly Val Val Asn  Ser Ser Asp Tyr Asn  Ile Met Lys
    1340                 1345                 1350

Arg Tyr  Leu Leu Lys Ala Ile  Glu Asp Ile Pro Tyr  Val Pro Glu
    1355                 1360                 1365

Asn Gln  Ala Pro Lys Ala Ile  Phe Thr Phe Ser Pro  Glu Asp Pro
    1370                 1375                 1380

Val Thr  Asp Glu Asn Val Val  Phe Asn Ala Ser Asn  Ser Ile Asp

1385                    1390                    1395

Glu Asp Gly Thr Ile Ala Tyr Tyr Ala Trp Asp Phe Gly Asp Gly
    1400                    1405                    1410

Tyr Glu Gly Thr Ser Thr Thr Pro Thr Ile Thr Tyr Lys Tyr Lys
    1415                    1420                    1425

Asn Pro Gly Thr Tyr Lys Val Lys Leu Ile Val Thr Asp Asn Gln
    1430                    1435                    1440

Gly Ala Ser Ser Ser Phe Thr Ala Thr Ile Lys Val Thr Ser Ala
    1445                    1450                    1455

Thr Gly Asp Asn Ser Lys Phe Asn Phe Glu Asp Gly Thr Leu Gly
    1460                    1465                    1470

Gly Phe Thr Thr Ser Gly Thr Asn Ala Thr Gly Val Val Val Asn
    1475                    1480                    1485

Thr Thr Glu Lys Ala Phe Lys Gly Glu Arg Gly Leu Lys Trp Thr
    1490                    1495                    1500

Val Thr Ser Glu Gly Glu Gly Thr Ala Glu Leu Lys Leu Asp Gly
    1505                    1510                    1515

Gly Thr Ile Val Val Pro Gly Thr Thr Met Thr Phe Arg Ile Trp
    1520                    1525                    1530

Ile Pro Ser Gly Ala Pro Ile Ala Ala Ile Gln Pro Tyr Ile Met
    1535                    1540                    1545

Pro His Thr Pro Asp Trp Ser Glu Val Leu Trp Asn Ser Thr Trp
    1550                    1555                    1560

Lys Gly Tyr Thr Met Val Lys Thr Asp Asp Trp Asn Glu Ile Thr
    1565                    1570                    1575

Leu Thr Leu Pro Glu Asp Val Asp Pro Thr Trp Pro Gln Gln Met
    1580                    1585                    1590

Gly Ile Gln Val Gln Thr Ile Asp Glu Gly Glu Phe Thr Ile Tyr
    1595                    1600                    1605

Val Asp Ala Ile Asp Trp
    1610

<210>  13
<211>  1809
<212>  DNA
<213>  Clostridium thermocellum

<220>
<223> CelT-d1

<400> 13
atgagaggat ctcaccatca ccatcaccat acggatccag ggattgtctc tttcaacacc 60
gtaagcacca gtgccgccgg agaatacaat tatgcaaagg cgctgcagta ttccatgttc 120
ttctatgatg cgaacatgtg cggtacaggt gttgacgaga acagcctttt gtcatggaga 180
ggagactgcc acgtatatga tgcaagactt cctctggatt cccagaacac caacatgtcc 240
gatggtttta taagcagcaa cagaagtgtg cttgaccctg acggagacgg caaagttgac 300
gtgtcaggcg gttttcatga cgccggcgac catgtgaagt ttggtttgcc tgaggcttat 360
gccgcttcaa cagtgggttg gggttactat gaatttaaag accagttccg tgcaacggga 420
caggccgtcc atgctgaagt aattttaaga tacttcaatg actattttat gagatgtact 480
ttcagagacg cttccggaaa tgttgtggcg ttctgtcatc aggtgggcga cggagatatc 540
gaccatgcat tttggggtgc tccggaaaat gacaccatgt tcagaagagg ttggtttatt 600
accaaagaaa agcctggaac tgacattatt tcggcaacag cagcttcttt agcaataaac 660
tacatgaatt ttaaagacac agaccctcaa tatgcggcaa aaagccttga ttatgcaaaa 720
gctttgtttg attttgcgga gaaaaatcca aaaggggtag ttcagggaga ggacggacca 780
aaaggttatt atggttcaag caaatggcag gatgactact gctgggctgc cgcatggctt 840
tatttggcaa cgcagaatga gcactatttg gatgaagcat ttaaatatta tgattattat 900
gctccgccgg gatggataca ttgctggaat gacgtgtggt cgggaaccgc atgtattttg 960
gcggaaataa atgatttgta cgacaaggac agccagaatt tcgaagacag gtataaaaga 1020
gcttccaata agaatcagtg ggagcagata gacttctgga aacccataca agatttgctt 1080
gacaagtggt cgggtggcgg tattacagtt acaccgggcg gatacgtttt cctcaatcag 1140
tggggttctg caagatacaa tactgccgct cagctgatag ctcttgttta tgacaagcat 1200
catggtgaca caccgtcaaa atatgctaac tgggcacggt cgcagatgga ttatctgttg 1260
ggtaaaaacc cgttgaatcg ctgctatgtt gtaggctaca gcagcaattc ggtcaaatac 1320
ccgcaccaca gagcggcttc cggactgaaa gatgccaatg attcttctcc gcacaaatat 1380
gtgttgtatg gtgccctggt cggagggccg gatgcaagtg accagcatgt ggatagaaca 1440
aatgattata tttacaatga ggttgccatt gactataatg ccgcttttgt gggagcatgt 1500
gcaggtcttt acagattctt cggggattct tcaatgcaga tagacccgtc aatgccgtcg 1560
cataacgtac ctgtaccacc gacacccaca cctcctgata cgcaaattgt atatggagat 1620
ttgaacggcg accagaaagt gacttccaca gactatacga tgctcaagag gtatttgatg 1680
aaaagcattg ataggtttaa tacttccgaa caagctgcgg atttgaacag agacggcaaa 1740
atcaattcca cggacttgac aatattgaaa agatatttgc tttacagcat accgtctctc 1800
cctatataa 1809

<210>  14
<211>  602
<212>  PRT
<213>  Clostridium thermocellum


<220>
<223> CelT-d1

<400>  14

Met Arg Gly Ser His His His His His His Thr Asp Pro Gly Ile Val
1               5                   10                  15


Ser Phe Asn Thr Val Ser Thr Ser Ala Ala Gly Glu Tyr Asn Tyr Ala
            20                  25                  30


Lys Ala Leu Gln Tyr Ser Met Phe Phe Tyr Asp Ala Asn Met Cys Gly
        35                  40                  45


Thr Gly Val Asp Glu Asn Ser Leu Leu Ser Trp Arg Gly Asp Cys His
    50                  55                  60


Val Tyr Asp Ala Arg Leu Pro Leu Asp Ser Gln Asn Thr Asn Met Ser
65                  70                  75                  80


Asp Gly Phe Ile Ser Ser Asn Arg Ser Val Leu Asp Pro Asp Gly Asp
                85                  90                  95


Gly Lys Val Asp Val Ser Gly Gly Phe His Asp Ala Gly Asp His Val
            100                 105                 110


Lys Phe Gly Leu Pro Glu Ala Tyr Ala Ala Ser Thr Val Gly Trp Gly
        115                 120                 125


Tyr Tyr Glu Phe Lys Asp Gln Phe Arg Ala Thr Gly Gln Ala Val His
    130                 135                 140


Ala Glu Val Ile Leu Arg Tyr Phe Asn Asp Tyr Phe Met Arg Cys Thr
145                 150                 155                 160


Phe Arg Asp Ala Ser Gly Asn Val Val Ala Phe Cys His Gln Val Gly
                165                 170                 175


Asp Gly Asp Ile Asp His Ala Phe Trp Gly Ala Pro Glu Asn Asp Thr
            180                 185                 190


Met Phe Arg Arg Gly Trp Phe Ile Thr Lys Glu Lys Pro Gly Thr Asp
        195                 200                 205


Ile Ile Ser Ala Thr Ala Ala Ser Leu Ala Ile Asn Tyr Met Asn Phe
    210                 215                 220


Lys Asp Thr Asp Pro Gln Tyr Ala Ala Lys Ser Leu Asp Tyr Ala Lys

225                 230                 235                 240

Ala Leu Phe Asp Phe Ala Glu Lys Asn Pro Lys Gly Val Val Gln Gly
                245                 250                 255

Glu Asp Gly Pro Lys Gly Tyr Tyr Gly Ser Ser Lys Trp Gln Asp Asp
            260                 265                 270

Tyr Cys Trp Ala Ala Ala Trp Leu Tyr Leu Ala Thr Gln Asn Glu His
        275                 280                 285

Tyr Leu Asp Glu Ala Phe Lys Tyr Tyr Asp Tyr Tyr Ala Pro Pro Gly
    290                 295                 300

Trp Ile His Cys Trp Asn Asp Val Trp Ser Gly Thr Ala Cys Ile Leu
305                 310                 315                 320

Ala Glu Ile Asn Asp Leu Tyr Asp Lys Asp Ser Gln Asn Phe Glu Asp
                325                 330                 335

Arg Tyr Lys Arg Ala Ser Asn Lys Asn Gln Trp Glu Gln Ile Asp Phe
            340                 345                 350

Trp Lys Pro Ile Gln Asp Leu Leu Asp Lys Trp Ser Gly Gly Gly Ile
        355                 360                 365

Thr Val Thr Pro Gly Gly Tyr Val Phe Leu Asn Gln Trp Gly Ser Ala
    370                 375                 380

Arg Tyr Asn Thr Ala Ala Gln Leu Ile Ala Leu Val Tyr Asp Lys His
385                 390                 395                 400

His Gly Asp Thr Pro Ser Lys Tyr Ala Asn Trp Ala Arg Ser Gln Met
                405                 410                 415

Asp Tyr Leu Leu Gly Lys Asn Pro Leu Asn Arg Cys Tyr Val Val Gly
            420                 425                 430

Tyr Ser Ser Asn Ser Val Lys Tyr Pro His His Arg Ala Ala Ser Gly
        435                 440                 445

Leu Lys Asp Ala Asn Asp Ser Ser Pro His Lys Tyr Val Leu Tyr Gly
    450                 455                 460

Ala Leu Val Gly Gly Pro Asp Ala Ser Asp Gln His Val Asp Arg Thr
465                 470                 475                 480

Asn Asp Tyr Ile Tyr Asn Glu Val Ala Ile Asp Tyr Asn Ala Ala Phe
                485                 490                 495

Val Gly Ala Cys Ala Gly Leu Tyr Arg Phe Phe Gly Asp Ser Ser Met

500                 505                 510

Gln Ile Asp Pro Ser Met Pro Ser His Asn Val Pro Val Pro Pro Thr
        515                 520                 525

Pro Thr Pro Pro Asp Thr Gln Ile Val Tyr Gly Asp Leu Asn Gly Asp
    530                 535                 540

Gln Lys Val Thr Ser Thr Asp Tyr Thr Met Leu Lys Arg Tyr Leu Met
545                 550                 555                 560

Lys Ser Ile Asp Arg Phe Asn Thr Ser Glu Gln Ala Ala Asp Leu Asn
                565                 570                 575

Arg Asp Gly Lys Ile Asn Ser Thr Asp Leu Thr Ile Leu Lys Arg Tyr
            580                 585                 590

Leu Leu Tyr Ser Ile Pro Ser Leu Pro Ile
        595                 600

<210>  15
<211>  2400
<212>  DNA
<213>  Clostridium thermocellum

<220>
<223> CelE-d1

<400>  15
atgagaggat cgcatcacca tcaccatcac ggatccccgg taaaaggctt tcaggtatcg      60

ggaacaaagc ttttggatgc aagcggaaac gagcttgtaa tgaggggcat gcgtgatatt     120

tcagcaatag atttggttaa agaaataaaa atcggatgga atttgggaaa tactttggat     180

gctcctacag agactgcctg gggaaatcca aggacaacca aggcaatgat agaaaaggta     240

agggaaatgg gctttaatgc cgtcagagtg cctgttacct gggatacgca catcggacct     300

gctccggact ataaaattga cgaagcatgg ctgaacagag ttgaggaagt ggtaaactat     360

gttcttgact gcggtatgta cgcgatcata aatcttcacc atgacaatac atggattata     420

cctacatatg ccaatgagca aaggagtaaa gaaaaacttg taaaagtttg ggaacaaata     480

gcaacccgtt ttaaagatta tgacgaccat ttgttgtttg agacaatgaa cgaaccgaga     540

gaagtaggtt cacctatgga atggatgggc ggaacgtatg aaaaccgaga tgtgataaac     600

agatttaatt tggcggttgt taataccatc agagcaagcg gcggaaataa cgataaaaga     660

ttcatactgg ttccgaccaa tgcggcaacc ggcctggatg ttgcattaaa cgaccttgtc     720

attccgaaca atgacagcag agtcatagta tccatacatg cttattcacc gtatttcttt     780

gctatggatg tcaacggaac ttcatattgg ggaagtgact atgacaaggc ttctcttaca     840

agtgaacttg atgctattta caacagattt gtgaaaaacg gaagggctgt aattatcgga     900

gaattcggaa ccattgacaa gaacaacctg tcttcaaggg tggctcatgc cgagcactat     960

gcaagagaag cagtttcaag aggaattgct gttttctggt gggataacgg ctattacaat 1020
ccgggtgatg cagagactta tgcattgctg aacagaaaaa ctctctcatg gtattatcct 1080
gaaattgtcc aggctcttat gagaggtgcc ggcgttgaac ctttagtttc accgactcct 1140
acacctacat taatgccgac cccctcgccc acggtgacag caaatatttt gtacggtgac 1200
gtaaacgggg acggaaaaat aaattctaca gactgtacaa tgctaaagag atatattttg 1260
cgtggcatag aagaattccc aagtcctagc ggaattatag ccgctgacgt aaatgcggat 1320
ctgaaaatca attccaccga cttggtattg atgaaaaaat atctactgcg ctcaatagac 1380
aaatttcctg cggaggattc tcaaacacct gatgaagaca atccgggcat tttgtataac 1440
ggaagattcg atttttcaga tccgaacggt ccgaaatgcg cctggtccgg cagcaatgtt 1500
gagctgaatt tttacggcac ggaagcaagt gtgactatca aatccggcgg tgagaactgg 1560
ttccaggcta ttgtagacgg caatcctctt cctccttttt cggttaacgc tactacctct 1620
accgtaaagc ttgtaagcgg tcttgcagaa ggagctcatc atcttgtatt gtggaagagg 1680
acagaggcat ccttgggaga agttcagttc cttgggtttg attttggttc aggaaagctt 1740
cttgccgcac cgaagccttt ggaaagaaag attgagttta tcggagactc catcacatgt 1800
gcatacggaa atgaaggaac aagcaaggag cagtctttta caccgaaaaa tgaaaacagc 1860
tatatgtctt atgcggcaat tacagcccgt aatttgaatg caagtgcaaa tatgattgcg 1920
tggtccggaa tcggacttac catgaactac ggcggagccc ccggacctct tataatggac 1980
cgttatcctt atacccttcc ttacagcgga gtcagatggg attttagcaa atatgtgcct 2040
caggttgttg taatcaatct tggtaccaat gatttttcta catcatttgc agataaaaca 2100
aagtttgtaa cggcatataa aaaccttata agtgaagttc gcaggaacta tccggatgcc 2160
catatattct gctgtgtcgg tccgatgctt tggggaacgg gcctggattt gtgccgcagt 2220
tatgttacgg aagttgtaaa tgattgtaac agaagcgggg atttaaaggt gtattttgtt 2280
gagtttccgc agcaggacgg aagcaccgga tacggagaag actggcatcc aagtattgcc 2340
acccaccagc tgatggctga gcggcttact gcggaaataa aaaacaagct tggatggtaa 2400

<210> 16
<211> 799
<212> PRT
<213> Clostridium thermocellum

<220>
<223> CelE-d1

<400> 16

Met Arg Gly Ser His His His His His His Gly Ser Pro Val Lys Gly
1               5                   10                  15

Phe Gln Val Ser Gly Thr Lys Leu Leu Asp Ala Ser Gly Asn Glu Leu
            20                  25                  30

Val Met Arg Gly Met Arg Asp Ile Ser Ala Ile Asp Leu Val Lys Glu
        35                  40                  45

Ile Lys Ile Gly Trp Asn Leu Gly Asn Thr Leu Asp Ala Pro Thr Glu
    50                  55                  60

Thr Ala Trp Gly Asn Pro Arg Thr Thr Lys Ala Met Ile Glu Lys Val
65                  70                  75                  80

Arg Glu Met Gly Phe Asn Ala Val Arg Val Pro Val Thr Trp Asp Thr
                85                  90                  95

His Ile Gly Pro Ala Pro Asp Tyr Lys Ile Asp Glu Ala Trp Leu Asn
            100                 105                 110

Arg Val Glu Glu Val Val Asn Tyr Val Leu Asp Cys Gly Met Tyr Ala
        115                 120                 125

Ile Ile Asn Leu His His Asp Asn Thr Trp Ile Ile Pro Thr Tyr Ala
    130                 135                 140

Asn Glu Gln Arg Ser Lys Glu Lys Leu Val Lys Val Trp Glu Gln Ile
145                 150                 155                 160

Ala Thr Arg Phe Lys Asp Tyr Asp Asp His Leu Leu Phe Glu Thr Met
                165                 170                 175

Asn Glu Pro Arg Glu Val Gly Ser Pro Met Glu Trp Met Gly Gly Thr
            180                 185                 190

Tyr Glu Asn Arg Asp Val Ile Asn Arg Phe Asn Leu Ala Val Val Asn
        195                 200                 205

Thr Ile Arg Ala Ser Gly Gly Asn Asn Asp Lys Arg Phe Ile Leu Val
    210                 215                 220

Pro Thr Asn Ala Ala Thr Gly Leu Asp Val Ala Leu Asn Asp Leu Val
225                 230                 235                 240

Ile Pro Asn Asn Asp Ser Arg Val Ile Val Ser Ile His Ala Tyr Ser
                245                 250                 255

Pro Tyr Phe Phe Ala Met Asp Val Asn Gly Thr Ser Tyr Trp Gly Ser
            260                 265                 270

Asp Tyr Asp Lys Ala Ser Leu Thr Ser Glu Leu Asp Ala Ile Tyr Asn
        275                 280                 285

Arg Phe Val Lys Asn Gly Arg Ala Val Ile Ile Gly Glu Phe Gly Thr
    290                 295                 300

Ile Asp Lys Asn Asn Leu Ser Ser Arg Val Ala His Ala Glu His Tyr
305                 310                 315                 320

Ala Arg Glu Ala Val Ser Arg Gly Ile Ala Val Phe Trp Trp Asp Asn
                325                 330                 335

Gly Tyr Tyr Asn Pro Gly Asp Ala Glu Thr Tyr Ala Leu Leu Asn Arg
            340                 345                 350

Lys Thr Leu Ser Trp Tyr Tyr Pro Glu Ile Val Gln Ala Leu Met Arg
        355                 360                 365

Gly Ala Gly Val Glu Pro Leu Val Ser Pro Thr Pro Thr Pro Thr Leu
    370                 375                 380

Met Pro Thr Pro Ser Pro Thr Val Thr Ala Asn Ile Leu Tyr Gly Asp
385                 390                 395                 400

Val Asn Gly Asp Gly Lys Ile Asn Ser Thr Asp Cys Thr Met Leu Lys
                405                 410                 415

Arg Tyr Ile Leu Arg Gly Ile Glu Glu Phe Pro Ser Pro Ser Gly Ile
            420                 425                 430

Ile Ala Ala Asp Val Asn Ala Asp Leu Lys Ile Asn Ser Thr Asp Leu
        435                 440                 445

Val Leu Met Lys Lys Tyr Leu Leu Arg Ser Ile Asp Lys Phe Pro Ala
    450                 455                 460

Glu Asp Ser Gln Thr Pro Asp Glu Asp Asn Pro Gly Ile Leu Tyr Asn
465                 470                 475                 480

Gly Arg Phe Asp Phe Ser Asp Pro Asn Gly Pro Lys Cys Ala Trp Ser
                485                 490                 495

Gly Ser Asn Val Glu Leu Asn Phe Tyr Gly Thr Glu Ala Ser Val Thr
            500                 505                 510

Ile Lys Ser Gly Gly Glu Asn Trp Phe Gln Ala Ile Val Asp Gly Asn
        515                 520                 525

Pro Leu Pro Pro Phe Ser Val Asn Ala Thr Thr Ser Thr Val Lys Leu
    530                 535                 540

Val Ser Gly Leu Ala Glu Gly Ala His His Leu Val Leu Trp Lys Arg
545                 550                 555                 560

Thr Glu Ala Ser Leu Gly Glu Val Gln Phe Leu Gly Phe Asp Phe Gly
                565                 570                 575

Ser Gly Lys Leu Leu Ala Ala Pro Lys Pro Leu Glu Arg Lys Ile Glu
            580                 585                 590

Phe Ile Gly Asp Ser Ile Thr Cys Ala Tyr Gly Asn Glu Gly Thr Ser
        595                 600                 605

Lys Glu Gln Ser Phe Thr Pro Lys Asn Glu Asn Ser Tyr Met Ser Tyr
    610                 615                 620

Ala Ala Ile Thr Ala Arg Asn Leu Asn Ala Ser Ala Asn Met Ile Ala
625                 630                 635                 640

Trp Ser Gly Ile Gly Leu Thr Met Asn Tyr Gly Gly Ala Pro Gly Pro
                645                 650                 655

Leu Ile Met Asp Arg Tyr Pro Tyr Thr Leu Pro Tyr Ser Gly Val Arg
            660                 665                 670

Trp Asp Phe Ser Lys Tyr Val Pro Gln Val Val Val Ile Asn Leu Gly
        675                 680                 685

Thr Asn Asp Phe Ser Thr Ser Phe Ala Asp Lys Thr Lys Phe Val Thr
    690                 695                 700

Ala Tyr Lys Asn Leu Ile Ser Glu Val Arg Arg Asn Tyr Pro Asp Ala
705                 710                 715                 720

His Ile Phe Cys Cys Val Gly Pro Met Leu Trp Gly Thr Gly Leu Asp
                725                 730                 735

Leu Cys Arg Ser Tyr Val Thr Glu Val Val Asn Asp Cys Asn Arg Ser
            740                 745                 750

Gly Asp Leu Lys Val Tyr Phe Val Glu Phe Pro Gln Gln Asp Gly Ser
        755                 760                 765

Thr Gly Tyr Gly Glu Asp Trp His Pro Ser Ile Ala Thr His Gln Leu
    770                 775                 780

Met Ala Glu Arg Leu Thr Ala Glu Ile Lys Asn Lys Leu Gly Trp
785                 790                 795

<210>  17
<211>  2985
<212>  DNA
<213>  Clostridium thermocellum

<220>
<223> CelQ-d1

<400>  17
atgagaggat ctcaccatca ccatcaccat gggatccgca tgcgagctcg gtaccccggg      60

tcgacggcat ttattcttcc tcaggggatt gtgtccgcag caggaagcta taactatgcg 120
gaagcacttc agaaagccat ttacttttat gagtgtcagc aggccggccc tctacctgaa 180
tggaaccgcg ttgagtggcg tggcgacgca acaatgaatg atgaggtact tggtggatgg 240
tatgacgcag gtgaccatgt caagtttaat ctgcctatgg cgtattcggc ggcaatgctt 300
ggctgggctc tttatgagta tggcgatgac attgaggcat cggggcagag acttcatctt 360
gaaaggaacc ttgcctttgc ccttgactat cttgttgcct gcgacagagg tgacagtgtc 420
gtttatcaga taggtgacgg tgccgctgac cataaatggt ggggttctgc ggaagttatt 480
gaaaaagaaa tgacaagacc ttactttgta ggaaagggat ccgccgttgt aggtcagatg 540
gctgcagctt tggctgtagg ttccatagtt cttaaaaatg atacatacct cagatatgcg 600
aagaagtatt tcgaacttgc agatgcaaca agaagtgaca gcacttatac tgctgcaaat 660
ggtttctaca gttcccacag cggattctgg gatgagctgt tgtgggcttc cacttggctc 720
tatcttgcaa caggtgatag aaattatctt gataaagctg agtcctatat tccaaaatta 780
aaccgtcaga atcagaccac agatatagaa tatcagtggg cacattgctg ggatgactgc 840
cactatggag caatgatctt gcttgcaaga gctacaggta aagaagagta tcacaaattt 900
gcacaaatgc atctggattg gtggacacct caaggttata acggaaagag agttgcatat 960
actcccggcg gacttgcgca tcttgatacc tggggaccgt tgagatatgc tacaactgaa 1020
gcattcctcg cttttgtata tgccgattca ataaatgacc cggctctcaa gcaaaaatat 1080
tataattttg cgaaaagcca gattgactat gcattgggtt caaatcctga caacagaagc 1140
tatgtagtcg gatttggaaa caatccgcca cagcgtcctc accacagaac cgctcatgga 1200
acttggttgg ataaaagaga tattccggaa aagcacagac atgtacttta cggtgctctg 1260
gtcggaggac ccggaagaga tgacagttat gaagacaata tagaggatta tgtaaaaaat 1320
gaagttgcct gcgactacaa tgcaggtttt gtaggcgcgc tctgcagatt gactgctgaa 1380
tacggcggaa ctcctcttgc gaacttcccg ccaccggaac aaagagatga tgagttcttc 1440
gtagaagcgg ctataaatca ggcaagtgat catttcactg aaataaaagc attgctcaac 1500
aaccgttcat cctggccggc aagacttatt aaggaccttt catacaacta ttatatggat 1560
ttgactgaag tttttgaggc aggttacagt gttgacgata ttaaagtaac aataggctat 1620
tgcgaaagcg gtatggatgt cgagatttcg ccgattactc atttgtatga caatatttat 1680
tacataaaaa tatcatatat cgacggaacc aatatttgtc cgataggtca ggaacagtat 1740
gccgctgagc ttcagttccg tattgcggca cctcaaggta ctaaattctg ggatccgaca 1800
aatgacttct catatcaggg acttaccaga gagttggcaa agacaaaata tatgcccgtt 1860
tttgacggag caacaaaaat ctttggagaa gttccaggcg gctttgaacc ggttccttca 1920
ccttcgccga ctcctgctca atataaagtc ggtgacttaa acggtgacgg agtggttaat 1980
tcaactgaca gtgtaatatt gaaaagacat ataattaaat tttctgaaat aacagatcca 2040
gttaaattga aagctgctga tcttaacgga gatggcaata taaactccag cgatgtttca 2100

ttaatgaaga gatatctgct ccgtataata gataaatttc cggtagaata gtttggcatt 2160

tgaaataagc ttaattagct gagcttggac tcctgttgat agatccagta atgacctcag 2220

aactccatct ggatttgttc agaacgctcg gttgccgccg ggcgtttttt attggtgaga 2280

atccaagcta gcttggcgag attttcagga gctaaggaag ctaaaatgga gaaaaaaatc 2340

actggatata ccaccgttga tatatcccaa tggcatcgta aagaacattt tgaggcattt 2400

cagtcagttg ctcaatgtac ctataaccag accgttcagc tggatattac ggccttttta 2460

aagaccgtaa agaaaaataa gcacaagttt tatccggcct ttattcacat tcttgcccgc 2520

ctgatgaatg ctcatccgga atttcgtatg gcaatgaaag acggtgagct ggtgatatgg 2580

gatagtgttc acccttgtta caccgttttc catgagcaaa ctgaaacgtt ttcatcgctc 2640

tggagtgaat accacgacga tttccggcag tttctacaca tatattcgca agatgtggcg 2700

tgttacggtg aaaacctggc ctatttccct aaagggttta ttgagaatat gtttttcgtc 2760

tcagccaatc cctgggtgag tttcaccagt tttgatttaa acgtggccaa tatggacaac 2820

ttcttcgccc ccgttttcac catgggcaaa tattatacgc aaggcgacaa ggtgctgatg 2880

ccgctggcga ttcaggttca tcatgccgtt tgtgatggct tccatgtcgg cagaatgctt 2940

aatgaattac aacagtactg cgatgagtgg cagggcgggg cgtaa 2985

<210> 18
<211> 987
<212> PRT
<213> Clostridium thermocellum

<220>
<223> CelQ-d1

<400> 18

Met Arg Gly Ser His His His His His His Gly Ile Arg Met Arg Ala
1               5                   10                  15

Arg Tyr Pro Gly Ser Thr Ala Phe Ile Leu Pro Gln Gly Ile Val Ser
            20                  25                  30

Ala Ala Gly Ser Tyr Asn Tyr Ala Glu Ala Leu Gln Lys Ala Ile Tyr
        35                  40                  45

Phe Tyr Glu Cys Gln Gln Ala Gly Pro Leu Pro Glu Trp Asn Arg Val
    50                  55                  60

Glu Trp Arg Gly Asp Ala Thr Met Asn Asp Glu Val Leu Gly Gly Trp
65                  70                  75                  80

Tyr Asp Ala Gly Asp His Val Lys Phe Asn Leu Pro Met Ala Tyr Ser
                85                  90                  95

Ala Ala Met Leu Gly Trp Ala Leu Tyr Glu Tyr Gly Asp Asp Ile Glu
            100                 105                 110

Ala Ser Gly Gln Arg Leu His Leu Glu Arg Asn Leu Ala Phe Ala Leu
        115                 120                 125

Asp Tyr Leu Val Ala Cys Asp Arg Gly Asp Ser Val Val Tyr Gln Ile
    130                 135                 140

Gly Asp Gly Ala Ala Asp His Lys Trp Trp Gly Ser Ala Glu Val Ile
145                 150                 155                 160

Glu Lys Glu Met Thr Arg Pro Tyr Phe Val Gly Lys Gly Ser Ala Val
                165                 170                 175

Val Gly Gln Met Ala Ala Ala Leu Ala Val Gly Ser Ile Val Leu Lys
            180                 185                 190

Asn Asp Thr Tyr Leu Arg Tyr Ala Lys Lys Tyr Phe Glu Leu Ala Asp
        195                 200                 205

Ala Thr Arg Ser Asp Ser Thr Tyr Thr Ala Ala Asn Gly Phe Tyr Ser
    210                 215                 220

Ser His Ser Gly Phe Trp Asp Glu Leu Leu Trp Ala Ser Thr Trp Leu
225                 230                 235                 240

Tyr Leu Ala Thr Gly Asp Arg Asn Tyr Leu Asp Lys Ala Glu Ser Tyr
                245                 250                 255

Ile Pro Lys Leu Asn Arg Gln Asn Gln Thr Thr Asp Ile Glu Tyr Gln
            260                 265                 270

Trp Ala His Cys Trp Asp Asp Cys His Tyr Gly Ala Met Ile Leu Leu
        275                 280                 285

Ala Arg Ala Thr Gly Lys Glu Glu Tyr His Lys Phe Ala Gln Met His
    290                 295                 300

Leu Asp Trp Trp Thr Pro Gln Gly Tyr Asn Gly Lys Arg Val Ala Tyr
305                 310                 315                 320

Thr Pro Gly Gly Leu Ala His Leu Asp Thr Trp Gly Pro Leu Arg Tyr
                325                 330                 335

Ala Thr Thr Glu Ala Phe Leu Ala Phe Val Tyr Ala Asp Ser Ile Asn
            340                 345                 350

Asp Pro Ala Leu Lys Gln Lys Tyr Tyr Asn Phe Ala Lys Ser Gln Ile
        355                 360                 365

Asp Tyr Ala Leu Gly Ser Asn Pro Asp Asn Arg Ser Tyr Val Val Gly
    370                 375                 380

Phe Gly Asn Asn Pro Pro Gln Arg Pro His His Arg Thr Ala His Gly
385                 390                 395                 400

Thr Trp Leu Asp Lys Arg Asp Ile Pro Glu Lys His Arg His Val Leu
                405                 410                 415

Tyr Gly Ala Leu Val Gly Gly Pro Gly Arg Asp Asp Ser Tyr Glu Asp
            420                 425                 430

Asn Ile Glu Asp Tyr Val Lys Asn Glu Val Ala Cys Asp Tyr Asn Ala
        435                 440                 445

Gly Phe Val Gly Ala Leu Cys Arg Leu Thr Ala Glu Tyr Gly Gly Thr
    450                 455                 460

Pro Leu Ala Asn Phe Pro Pro Pro Glu Gln Arg Asp Asp Glu Phe Phe
465                 470                 475                 480

Val Glu Ala Ala Ile Asn Gln Ala Ser Asp His Phe Thr Glu Ile Lys
                485                 490                 495

Ala Leu Leu Asn Asn Arg Ser Ser Trp Pro Ala Arg Leu Ile Lys Asp
            500                 505                 510

Leu Ser Tyr Asn Tyr Tyr Met Asp Leu Thr Glu Val Phe Glu Ala Gly
        515                 520                 525

Tyr Ser Val Asp Asp Ile Lys Val Thr Ile Gly Tyr Cys Glu Ser Gly
    530                 535                 540

Met Asp Val Glu Ile Ser Pro Ile Thr His Leu Tyr Asp Asn Ile Tyr
545                 550                 555                 560

Tyr Ile Lys Ile Ser Tyr Ile Asp Gly Thr Asn Ile Cys Pro Ile Gly
                565                 570                 575

Gln Glu Gln Tyr Ala Ala Glu Leu Gln Phe Arg Ile Ala Ala Pro Gln
            580                 585                 590

Gly Thr Lys Phe Trp Asp Pro Thr Asn Asp Phe Ser Tyr Gln Gly Leu
        595                 600                 605

Thr Arg Glu Leu Ala Lys Thr Lys Tyr Met Pro Val Phe Asp Gly Ala
    610                 615                 620

Thr Lys Ile Phe Gly Glu Val Pro Gly Gly Phe Glu Pro Val Pro Ser
625                 630                 635                 640

Pro Ser Pro Thr Pro Ala Gln Tyr Lys Val Gly Asp Leu Asn Gly Asp
                645                 650                 655

Gly Val Val Asn Ser Thr Asp Ser Val Ile Leu Lys Arg His Ile Ile
            660                 665                 670

Lys Phe Ser Glu Ile Thr Asp Pro Val Lys Leu Lys Ala Ala Asp Leu
        675                 680                 685

Asn Gly Asp Gly Asn Ile Asn Ser Ser Asp Val Ser Leu Met Lys Arg
    690                 695                 700

Tyr Leu Leu Arg Ile Ile Asp Lys Phe Pro Val Glu Phe Gly Ile Asn
705                 710                 715                 720

Lys Leu Asn Leu Ser Leu Asp Ser Cys Ile Gln Pro Gln Asn Ser Ile
                725                 730                 735

Trp Ile Cys Ser Glu Arg Ser Val Ala Ala Gly Arg Phe Leu Leu Val
            740                 745                 750

Arg Ile Gln Ala Ser Leu Ala Arg Phe Ser Gly Ala Lys Glu Ala Lys
        755                 760                 765

Met Glu Lys Lys Ile Thr Gly Tyr Thr Thr Val Asp Ile Ser Gln Trp
    770                 775                 780

His Arg Lys Glu His Phe Glu Ala Phe Gln Ser Val Ala Gln Cys Thr
785                 790                 795                 800

Tyr Asn Gln Thr Val Gln Leu Asp Ile Thr Ala Phe Leu Lys Thr Val
                805                 810                 815

Lys Lys Asn Lys His Lys Phe Tyr Pro Ala Phe Ile His Ile Leu Ala
            820                 825                 830

Arg Leu Met Asn Ala His Pro Glu Phe Arg Met Ala Met Lys Asp Gly
        835                 840                 845

Glu Leu Val Ile Trp Asp Ser Val His Pro Cys Tyr Thr Val Phe His
    850                 855                 860

Glu Gln Thr Glu Thr Phe Ser Ser Leu Trp Ser Glu Tyr His Asp Asp
865                 870                 875                 880

Phe Arg Gln Phe Leu His Ile Tyr Ser Gln Asp Val Ala Cys Tyr Gly
                885                 890                 895

Glu Asn Leu Ala Tyr Phe Pro Lys Gly Phe Ile Glu Asn Met Phe Phe
            900                 905                 910

Val Ser Ala Asn Pro Trp Val Ser Phe Thr Ser Phe Asp Leu Asn Val
        915                 920                 925

Ala Asn Met Asp Asn Phe Phe Ala Pro Val Phe Thr Met Gly Lys Tyr
    930                 935                 940

Tyr Thr Gln Gly Asp Lys Val Leu Met Pro Leu Ala Ile Gln Val His
945                 950                 955                 960

His Ala Val Cys Asp Gly Phe His Val Gly Arg Met Leu Asn Glu Leu
                965                 970                 975

Gln Gln Tyr Cys Asp Glu Trp Gln Gly Gly Ala
            980                 985

<210>  19
<211>  3696
<212>  DNA
<213>  Clostridium thermocellum

<220>
<223> CbhA-d1

<400>  19
atgagaggat ctcaccatca ccatcaccat acggatccgc atgcgagctc cgtgtttgcc      60

ttagaagata attcttcgac tttgccgccg tataaaaacg accttttgta tgagaggact     120

tttgatgagg gactttgtta tccatggcat acctgtgaag acagcggagg aaaatgctcc     180

tttgatgtgg tcgatgttcc ggggcagccc ggtaataaag catttgccgt tactgttctt     240

gacaaagggc aaaacagatg gagagttcag atgagacacc gtggtcttac tcttgaacag     300

ggacatacat atagagtacg gcttaagatt tgggcagatg cgtcctgtaa agtttatata     360

aaaataggac aaatggcgga gccctatgct gaatattgga acaacaagtg gagtccatac     420

acactgacag caggtaaggt attggaaatt gacgagacgt ttgttatgga caagccaact     480

gacgacacat gcgaatttac attccattta ggtggcgaat tggcagcaac tcctccatat     540

acagtttatc ttgatgatgt atccctttat gacccagaat atacgaagcc tgttgaatat     600

atacttccgc agcctgatgt acgtgtgaac caggttggct acctgccgga gggcaagaaa     660

gttgccactg tggtatgcaa ttcaactcag ccggtaaaat ggcagcttaa gaatgctgca     720

ggcgttgtag ttttggaagg ttataccgaa ccaaagggtc ttgacaaaga ctcgcaggat     780

tatgtacatt ggcttgattt ttccgatttt gcaaccgaag gaattggtta ctattttgaa     840

cttccgactg taaacagtcc tacaaactac agtcatccat ttgacattcg caaagacatc     900

tatactcaga tgaaatatga tgcattggca ttcttctatc acaagagaag cggtattcct     960

attgaaatgc cgtatgcagg aggagaacag tggaccagac ctgcaggaca tatcggaatt    1020

gagccgaaca agggagatac aaatgttcct acatggcctc aggatgatga gtatgcagga    1080

atacctcaga agaattatac aaaggatgta accggtggat ggtatgatgc cggtgaccac    1140

ggtaaatatg ttgtaaacgg cggtatagcc gtctggacat taatgaacat gtatgagagg    1200

gcaaaaatta gaggtcttga caactgggga ccatacaggg acggcggaat gaacataccg    1260

gagcagaata acggttatcc ggacattctt gatgaagcaa gatgggaaat tgagttcttt 1320
aagaaaatgc aggtaactga aaaagaggat ccttccatag ccggaatggt acaccacaaa 1380
attcacgact tcagatggac tgctttgggt atgttgcctc acgaagatcc ccagccacgt 1440
tacttaaggc cggtaagtac ggctgcgact ttgaactttg cggcaacttt ggcacaaagt 1500
gcacgtcttt ggaaagatta tgatccgact tttgctgctg actgtttgga aaaggctgaa 1560
atagcatggc aggcggcatt aaagcatcct gatatttatg ctgagtatac tcccggtagc 1620
ggtggtcccg gaggcggacc atacaatgac gactatgtcg gagacgaatt ctactgggca 1680
gcctgcgaac tttatgtaac aacaggaaaa gacgaatata agaattacct gatgaattca 1740
cctcactatc ttgaaatgcc tgcaaagatg ggtgaaaacg gtggagcaaa cggagaagac 1800
aacggattgt ggggatgctt cacctgggga actactcaag gattgggaac cattactctt 1860
gctttggttg aaaacggatt gcctgctaca gacattcaaa aggcaagaaa caatatagct 1920
aaagctgctg acagatggct tgagaatatt gaagagcaag gttacagact gccgatcaaa 1980
cgggcggagg atgagagagc cggttatcca tggggttcaa actccttgca ttttgaacca 2040
gatgacctag ttatgggata tgcctatgac tttacaggtg actcaaatat ctcgatggaa 2100
tgtttgaccg gcataagcta cctgttggga agaaacgcaa tggatcagtc ctatgtaaca 2160
gggtatggtg agcgtccgct tcagaatcct catgacaggt tctggacgcc gcagacaagt 2220
aagagattcc ctgctccacc tccgggtata atttccggcc gtccgaactc ccgtttcgag 2280
gacccgacaa taaatgcggc cgttaagaag gatacaccgc cacagaaatg ttttatcgac 2340
catacagact catggtcaac caacgagata actgttaact ggaatgctcc gtttgcatgg 2400
gttacagctt atcttgacga gcagtacaca gacagtgaaa ccgataaggt aactattgat 2460
tcgcctgttg caggagaaag atttgaagcg ggtaaagaca ttaatataag aactgttaaa 2520
tcaaaaactc ctgtaagcaa agtagagttt tacaatggag atacgcttat ttccagtgac 2580
acaactgcac cttacacagc aaagataaca ggagccgctg tcggagcata taaccttaaa 2640
gcggttgcag tgctgtctga cggaagaaga attgagtcac cggtaactcc tgtacttgtt 2700
aaggtaattg tgaaacctac tgtaaaactt actgcaccca agtcaaatgt tgtggcttat 2760
ggaaatgagt tcctgaagat tacagcaaca gccagtgact ctgacggcaa aatctccagg 2820
gttgatttcc ttgttgacgg tgaagtaatc ggttcagaca gggaagcacc ttatgaatat 2880
gagtggaaag ctgtggaagg caatcacgaa ataagtgtaa ttgcttatga tgatgacgat 2940
gcggcttcaa cacctgattc cgtaaaaata tttgtaaaac aggcacggga tgtaaaagta 3000
cagtatttgt gcgaaaatac gcaaacatcc actcaggaaa tcaagggtaa attcaatata 3060
gttaacacag gaaacagaga ttattcgctg aaagatatag tattaagata ctactttacc 3120
aaggagcaca attcacagct tcagtttatc tgctattata cacccatagg ctccggaaat 3180
ctcattccgt cctttggcgg ctcgggtgac gagcattatc tgcagctgga attcaaagat 3240
gtcaagctgc ctgccggcgg tcagactggg gaaatacagt ttgttataag atatgcagat 3300

aactccttcc atgatcagtc gaacgactat tcgttcgatc caactataaa agcgttccag 3360

gattatggca aggttaccct gtataagaat ggagaacttg tttggggaac gccgccgggc 3420

ggtacagaac ctgaagaacc ggaagagcct gaagaaccgg aagagcctgc gatagtttac 3480

ggcgactgta atgatgacgg caaagtaaat tcaacagacg tcgcagtaat gaagagatat 3540

ttaaagaaag aaaatgttaa tattaatctt gacaatgcag atgtgaatgc ggacggcaaa 3600

gttaactcaa cagacttctc aatacttaag agatatgtta tgaagaacat agaagaattg 3660

ccatatcgat aagataatct gaaattattt gtgtaa 3696

<210> 20
<211> 1223
<212> PRT
<213> Clostridium thermocellum

<220>
<223> CbhA-d1

<400> 20

Met Arg Gly Ser His His His His His His Thr Asp Pro His Ala Ser
1               5                   10                  15

Ser Val Phe Ala Leu Glu Asp Asn Ser Ser Thr Leu Pro Pro Tyr Lys
            20                  25                  30

Asn Asp Leu Leu Tyr Glu Arg Thr Phe Asp Glu Gly Leu Cys Tyr Pro
        35                  40                  45

Trp His Thr Cys Glu Asp Ser Gly Gly Lys Cys Ser Phe Asp Val Val
    50                  55                  60

Asp Val Pro Gly Gln Pro Gly Asn Lys Ala Phe Ala Val Thr Val Leu
65                  70                  75                  80

Asp Lys Gly Gln Asn Arg Trp Arg Val Gln Met Arg His Arg Gly Leu
                85                  90                  95

Thr Leu Glu Gln Gly His Thr Tyr Arg Val Arg Leu Lys Ile Trp Ala
            100                 105                 110

Asp Ala Ser Cys Lys Val Tyr Ile Lys Ile Gly Gln Met Ala Glu Pro
        115                 120                 125

Tyr Ala Glu Tyr Trp Asn Asn Lys Trp Ser Pro Tyr Thr Leu Thr Ala
    130                 135                 140

Gly Lys Val Leu Glu Ile Asp Glu Thr Phe Val Met Asp Lys Pro Thr
145                 150                 155                 160

Asp Asp Thr Cys Glu Phe Thr Phe His Leu Gly Gly Glu Leu Ala Ala
                165                 170                 175

Thr Pro Pro Tyr Thr Val Tyr Leu Asp Asp Val Ser Leu Tyr Asp Pro
            180                 185                 190

Glu Tyr Thr Lys Pro Val Glu Tyr Ile Leu Pro Gln Pro Asp Val Arg
        195                 200                 205

Val Asn Gln Val Gly Tyr Leu Pro Glu Gly Lys Lys Val Ala Thr Val
    210                 215                 220

Val Cys Asn Ser Thr Gln Pro Val Lys Trp Gln Leu Lys Asn Ala Ala
225                 230                 235                 240

Gly Val Val Val Leu Glu Gly Tyr Thr Glu Pro Lys Gly Leu Asp Lys
                245                 250                 255

Asp Ser Gln Asp Tyr Val His Trp Leu Asp Phe Ser Asp Phe Ala Thr
            260                 265                 270

Glu Gly Ile Gly Tyr Tyr Phe Glu Leu Pro Thr Val Asn Ser Pro Thr
        275                 280                 285

Asn Tyr Ser His Pro Phe Asp Ile Arg Lys Asp Ile Tyr Thr Gln Met
    290                 295                 300

Lys Tyr Asp Ala Leu Ala Phe Phe Tyr His Lys Arg Ser Gly Ile Pro
305                 310                 315                 320

Ile Glu Met Pro Tyr Ala Gly Gly Glu Gln Trp Thr Arg Pro Ala Gly
                325                 330                 335

His Ile Gly Ile Glu Pro Asn Lys Gly Asp Thr Asn Val Pro Thr Trp
            340                 345                 350

Pro Gln Asp Asp Glu Tyr Ala Gly Ile Pro Gln Lys Asn Tyr Thr Lys
        355                 360                 365

Asp Val Thr Gly Gly Trp Tyr Asp Ala Gly Asp His Gly Lys Tyr Val
    370                 375                 380

Val Asn Gly Gly Ile Ala Val Trp Thr Leu Met Asn Met Tyr Glu Arg
385                 390                 395                 400

Ala Lys Ile Arg Gly Leu Asp Asn Trp Gly Pro Tyr Arg Asp Gly Gly
                405                 410                 415

Met Asn Ile Pro Glu Gln Asn Asn Gly Tyr Pro Asp Ile Leu Asp Glu
            420                 425                 430

Ala Arg Trp Glu Ile Glu Phe Phe Lys Lys Met Gln Val Thr Glu Lys
        435                 440                 445

Glu Asp Pro Ser Ile Ala Gly Met Val His His Lys Ile His Asp Phe
    450                 455                 460

Arg Trp Thr Ala Leu Gly Met Leu Pro His Glu Asp Pro Gln Pro Arg
465                 470                 475                 480

Tyr Leu Arg Pro Val Ser Thr Ala Ala Thr Leu Asn Phe Ala Ala Thr
                485                 490                 495

Leu Ala Gln Ser Ala Arg Leu Trp Lys Asp Tyr Asp Pro Thr Phe Ala
            500                 505                 510

Ala Asp Cys Leu Glu Lys Ala Glu Ile Ala Trp Gln Ala Ala Leu Lys
        515                 520                 525

His Pro Asp Ile Tyr Ala Glu Tyr Thr Pro Gly Ser Gly Gly Pro Gly
    530                 535                 540

Gly Gly Pro Tyr Asn Asp Asp Tyr Val Gly Asp Glu Phe Tyr Trp Ala
545                 550                 555                 560

Ala Cys Glu Leu Tyr Val Thr Thr Gly Lys Asp Glu Tyr Lys Asn Tyr
                565                 570                 575

Leu Met Asn Ser Pro His Tyr Leu Glu Met Pro Ala Lys Met Gly Glu
            580                 585                 590

Asn Gly Gly Ala Asn Gly Glu Asp Asn Gly Leu Trp Gly Cys Phe Thr
        595                 600                 605

Trp Gly Thr Thr Gln Gly Leu Gly Thr Ile Thr Leu Ala Leu Val Glu
    610                 615                 620

Asn Gly Leu Pro Ala Thr Asp Ile Gln Lys Ala Arg Asn Asn Ile Ala
625                 630                 635                 640

Lys Ala Ala Asp Arg Trp Leu Glu Asn Ile Glu Glu Gln Gly Tyr Arg
                645                 650                 655

Leu Pro Ile Lys Arg Ala Glu Asp Glu Arg Ala Gly Tyr Pro Trp Gly
            660                 665                 670

Ser Asn Ser Leu His Phe Glu Pro Asp Asp Leu Val Met Gly Tyr Ala
        675                 680                 685

Tyr Asp Phe Thr Gly Asp Ser Asn Ile Ser Met Glu Cys Leu Thr Gly
    690                 695                 700

Ile Ser Tyr Leu Leu Gly Arg Asn Ala Met Asp Gln Ser Tyr Val Thr
705                 710                 715                 720

Gly Tyr Gly Glu Arg Pro Leu Gln Asn Pro His Asp Arg Phe Trp Thr
                725                 730                 735

Pro Gln Thr Ser Lys Arg Phe Pro Ala Pro Pro Pro Gly Ile Ile Ser
            740                 745                 750

Gly Arg Pro Asn Ser Arg Phe Glu Asp Pro Thr Ile Asn Ala Ala Val
        755                 760                 765

Lys Lys Asp Thr Pro Pro Gln Lys Cys Phe Ile Asp His Thr Asp Ser
    770                 775                 780

Trp Ser Thr Asn Glu Ile Thr Val Asn Trp Asn Ala Pro Phe Ala Trp
785                 790                 795                 800

Val Thr Ala Tyr Leu Asp Glu Gln Tyr Thr Asp Ser Glu Thr Asp Lys
                805                 810                 815

Val Thr Ile Asp Ser Pro Val Ala Gly Glu Arg Phe Glu Ala Gly Lys
            820                 825                 830

Asp Ile Asn Ile Arg Thr Val Lys Ser Lys Thr Pro Val Ser Lys Val
        835                 840                 845

Glu Phe Tyr Asn Gly Asp Thr Leu Ile Ser Ser Asp Thr Thr Ala Pro
    850                 855                 860

Tyr Thr Ala Lys Ile Thr Gly Ala Ala Val Gly Ala Tyr Asn Leu Lys
865                 870                 875                 880

Ala Val Ala Val Leu Ser Asp Gly Arg Arg Ile Glu Ser Pro Val Thr
                885                 890                 895

Pro Val Leu Val Lys Val Ile Val Lys Pro Thr Val Lys Leu Thr Ala
            900                 905                 910

Pro Lys Ser Asn Val Val Ala Tyr Gly Asn Glu Phe Leu Lys Ile Thr
        915                 920                 925

Ala Thr Ala Ser Asp Ser Asp Gly Lys Ile Ser Arg Val Asp Phe Leu
    930                 935                 940

Val Asp Gly Glu Val Ile Gly Ser Asp Arg Glu Ala Pro Tyr Glu Tyr
945                 950                 955                 960

Glu Trp Lys Ala Val Glu Gly Asn His Glu Ile Ser Val Ile Ala Tyr
                965                 970                 975

Asp Asp Asp Asp Ala Ala Ser Thr Pro Asp Ser Val Lys Ile Phe Val
            980                 985                 990

Lys Gln Ala Arg Asp Val Lys Val Gln Tyr Leu Cys Glu Asn Thr Gln
        995                 1000                1005

Thr Ser Thr Gln Glu Ile Lys Gly Lys Phe Asn Ile Val Asn Thr
    1010                1015                1020

Gly Asn Arg Asp Tyr Ser Leu Lys Asp Ile Val Leu Arg Tyr Tyr
    1025                1030                1035

Phe Thr Lys Glu His Asn Ser Gln Leu Gln Phe Ile Cys Tyr Tyr
    1040                1045                1050

Thr Pro Ile Gly Ser Gly Asn Leu Ile Pro Ser Phe Gly Gly Ser
    1055                1060                1065

Gly Asp Glu His Tyr Leu Gln Leu Glu Phe Lys Asp Val Lys Leu
    1070                1075                1080

Pro Ala Gly Gly Gln Thr Gly Glu Ile Gln Phe Val Ile Arg Tyr
    1085                1090                1095

Ala Asp Asn Ser Phe His Asp Gln Ser Asn Asp Tyr Ser Phe Asp
    1100                1105                1110

Pro Thr Ile Lys Ala Phe Gln Asp Tyr Gly Lys Val Thr Leu Tyr
    1115                1120                1125

Lys Asn Gly Glu Leu Val Trp Gly Thr Pro Pro Gly Gly Thr Glu
    1130                1135                1140

Pro Glu Glu Pro Glu Glu Pro Glu Glu Pro Glu Glu Pro Ala Ile
    1145                1150                1155

Val Tyr Gly Asp Cys Asn Asp Asp Gly Lys Val Asn Ser Thr Asp
    1160                1165                1170

Val Ala Val Met Lys Arg Tyr Leu Lys Lys Glu Asn Val Asn Ile
    1175                1180                1185

Asn Leu Asp Asn Ala Asp Val Asn Ala Asp Gly Lys Val Asn Ser
    1190                1195                1200

Thr Asp Phe Ser Ile Leu Lys Arg Tyr Val Met Lys Asn Ile Glu
    1205                1210                1215

Glu Leu Pro Tyr Arg
    1220

<210> 21
<211> 2106

<212>  DNA
<213>  Clostridium thermocellum

<220>
<223>  c3-c1-c1-d2

<400>  21
atgagaggat ctcaccatca ccatcaccat acggatccgc cggcgggtat tgcacgcgca      60

gataaagcct cgagcattga gcttaagttt gaccgcaata agggcgaagt tggagatata     120

cttattggta ccgtacgcat taacaatatc aagaatttcg caggctttca ggtaaacatt     180

gtatatgatc caaaagtctt aatggctgtt gaccctgaaa cggggaaaga atttacttct     240

tcaacatttc cgccaggccg cactgtactg aaaaacaatg cttacggccc aattcagatt     300

gcggacaatg atccggaaaa agggattctg aacttcgcgc ttgcatattc atatattgcg     360

ggctacaaag aaacaggcgt agcggaggaa agcggcatca ttgcgaaaat tggctttaaa     420

attctccaga aaaagagcac tgccgtaaaa ttccaggata cattaagcat gcccggcgct     480

atttcgggca cacagctgtt tgactgggac ggcgaagtta ttaccggcta tgaggtaatt     540

cagccggatg tgctgagttt gggtgacgag ccttatgaga caccgggcac ggatattccg     600

atttccgaca atccggcagc aactccgtca tccacgccgt cagttactcc ttcaccggat     660

ccgcccacca ggccatcggt accgacaaac acaccgacaa acacaccggc aaatacaccg     720

gtatcaggca atttgaaggt tgaattctac aacagcaatc cttcagatac tactaactca     780

atcaatcctc agttcaaggt tactaatacc ggaagcagtg caattgattt gtccaaactc     840

acattgagat attattatac agtagacgga cagaaagatc agaccttctg gtgtgaccat     900

gctgcaataa tcggcagtaa cggcagctac aacggaatta cttcaaatgt aaaaggaaca     960

tttgtaaaaa tgagttcctc aacaaataac gcagacacct accttgaaat aagctttaca    1020

ggcggaactc ttgaaccggg tgcacatgtt cagatacaag gtagatttgc aaagaatgac    1080

tggagtaact atacacagtc aaatgactac tcattcaagt ctgcttcaca gtttgttgaa    1140

tgggatcagg taacagcata cttgaacggt gttcttgtat ggggtaaaga acccggtggc    1200

agtgtagtac catcaacaca gcctgtaaca acaccacctg caacaacaaa accacctgca    1260

acaacaaaac cacctgcaac aacaataccg ccgtcagatg atccgaatgc aataaagatt    1320

aaggtggaca cagtaaatgc aaaaccggga gacacagtaa atatacctgt aagattcagt    1380

ggtataccat ccaagggaat agcaaactgt gactttgtat acagctatga cccgaatgta    1440

cttgagataa tagagataaa accgggagaa ttgatagttg acccgaatcc tgacaagagc    1500

tttgatactg cagtatatcc tgacagaaag ataatagtat tcctgtttgc agaagacagc    1560

ggaacaggag cgtatgcaat aactaaagac ggagtatttg ctacgatagt agcgaaagta    1620

aaatccggag cacctaacgg actcagtgta atcaaatttg tagaagtagg cggatttgcg    1680

aacaatgacc ttgtagaaca gaggacacag ttctttgacg gtggagtaaa tgttggagat    1740

acaacagtac ctacaacacc tacaacacct gtaacaacac cgacagattg ttcgagctca    1800

gccaatgtac cgtcacatgg tgtagtggta ttaaaagtac aagcaagctc cactgataca 1860

aatattgagt ttggtgatgt tgacggcaat ggcatgattg acgcattaga ttattcatta 1920

gtaaaacggt atttgctggg ccagatttct gattgtcctg attcaaaagg caagcttgct 1980

gctgatgttg atggcgacca gcaaattaca gcactggatt tttcattaat taagcaatac 2040

ttacttggga ctattaacaa atttcctgct caaacagcaa gtcgacctgc agccaagctt 2100

aattag 2106

<210> 22
<211> 701
<212> PRT
<213> Clostridium thermocellum

<220>
<223> c3-c1-c1-d2

<400> 22

Met Arg Gly Ser His His His His His His Thr Asp Pro Pro Ala Gly
1               5                   10                  15

Ile Ala Arg Ala Asp Lys Ala Ser Ser Ile Glu Leu Lys Phe Asp Arg
            20                  25                  30

Asn Lys Gly Glu Val Gly Asp Ile Leu Ile Gly Thr Val Arg Ile Asn
        35                  40                  45

Asn Ile Lys Asn Phe Ala Gly Phe Gln Val Asn Ile Val Tyr Asp Pro
    50                  55                  60

Lys Val Leu Met Ala Val Asp Pro Glu Thr Gly Lys Glu Phe Thr Ser
65                  70                  75                  80

Ser Thr Phe Pro Pro Gly Arg Thr Val Leu Lys Asn Asn Ala Tyr Gly
                85                  90                  95

Pro Ile Gln Ile Ala Asp Asn Asp Pro Glu Lys Gly Ile Leu Asn Phe
            100                 105                 110

Ala Leu Ala Tyr Ser Tyr Ile Ala Gly Tyr Lys Glu Thr Gly Val Ala
        115                 120                 125

Glu Glu Ser Gly Ile Ile Ala Lys Ile Gly Phe Lys Ile Leu Gln Lys
    130                 135                 140

Lys Ser Thr Ala Val Lys Phe Gln Asp Thr Leu Ser Met Pro Gly Ala
145                 150                 155                 160

Ile Ser Gly Thr Gln Leu Phe Asp Trp Asp Gly Glu Val Ile Thr Gly
                165                 170                 175

Tyr Glu Val Ile Gln Pro Asp Val Leu Ser Leu Gly Asp Glu Pro Tyr
            180                 185                 190

Glu Thr Pro Gly Thr Asp Ile Pro Ile Ser Asp Asn Pro Ala Ala Thr
        195                 200                 205

Pro Ser Ser Thr Pro Ser Val Thr Pro Ser Pro Asp Pro Pro Thr Arg
    210                 215                 220

Pro Ser Val Pro Thr Asn Thr Pro Thr Asn Thr Pro Ala Asn Thr Pro
225                 230                 235                 240

Val Ser Gly Asn Leu Lys Val Glu Phe Tyr Asn Ser Asn Pro Ser Asp
                245                 250                 255

Thr Thr Asn Ser Ile Asn Pro Gln Phe Lys Val Thr Asn Thr Gly Ser
            260                 265                 270

Ser Ala Ile Asp Leu Ser Lys Leu Thr Leu Arg Tyr Tyr Tyr Thr Val
        275                 280                 285

Asp Gly Gln Lys Asp Gln Thr Phe Trp Cys Asp His Ala Ala Ile Ile
    290                 295                 300

Gly Ser Asn Gly Ser Tyr Asn Gly Ile Thr Ser Asn Val Lys Gly Thr
305                 310                 315                 320

Phe Val Lys Met Ser Ser Ser Thr Asn Asn Ala Asp Thr Tyr Leu Glu
                325                 330                 335

Ile Ser Phe Thr Gly Gly Thr Leu Glu Pro Gly Ala His Val Gln Ile
            340                 345                 350

Gln Gly Arg Phe Ala Lys Asn Asp Trp Ser Asn Tyr Thr Gln Ser Asn
        355                 360                 365

Asp Tyr Ser Phe Lys Ser Ala Ser Gln Phe Val Glu Trp Asp Gln Val
    370                 375                 380

Thr Ala Tyr Leu Asn Gly Val Leu Val Trp Gly Lys Glu Pro Gly Gly
385                 390                 395                 400

Ser Val Val Pro Ser Thr Gln Pro Val Thr Thr Pro Pro Ala Thr Thr
                405                 410                 415

Lys Pro Pro Ala Thr Thr Lys Pro Pro Ala Thr Thr Ile Pro Pro Ser
            420                 425                 430

Asp Asp Pro Asn Ala Ile Lys Ile Lys Val Asp Thr Val Asn Ala Lys
        435                 440                 445

Pro Gly Asp Thr Val Asn Ile Pro Val Arg Phe Ser Gly Ile Pro Ser
    450                 455                 460

Lys Gly Ile Ala Asn Cys Asp Phe Val Tyr Ser Tyr Asp Pro Asn Val
465                 470                 475                 480

Leu Glu Ile Ile Glu Ile Lys Pro Gly Glu Leu Ile Val Asp Pro Asn
                485                 490                 495

Pro Asp Lys Ser Phe Asp Thr Ala Val Tyr Pro Asp Arg Lys Ile Ile
            500                 505                 510

Val Phe Leu Phe Ala Glu Asp Ser Gly Thr Gly Ala Tyr Ala Ile Thr
        515                 520                 525

Lys Asp Gly Val Phe Ala Thr Ile Val Ala Lys Val Lys Ser Gly Ala
    530                 535                 540

Pro Asn Gly Leu Ser Val Ile Lys Phe Val Glu Val Gly Gly Phe Ala
545                 550                 555                 560

Asn Asn Asp Leu Val Glu Gln Arg Thr Gln Phe Phe Asp Gly Gly Val
                565                 570                 575

Asn Val Gly Asp Thr Thr Val Pro Thr Thr Pro Thr Thr Pro Val Thr
            580                 585                 590

Thr Pro Thr Asp Cys Ser Ser Ser Ala Asn Val Pro Ser His Gly Val
        595                 600                 605

Val Val Leu Lys Val Gln Ala Ser Ser Thr Asp Thr Asn Ile Glu Phe
    610                 615                 620

Gly Asp Val Asp Gly Asn Gly Met Ile Asp Ala Leu Asp Tyr Ser Leu
625                 630                 635                 640

Val Lys Arg Tyr Leu Leu Gly Gln Ile Ser Asp Cys Pro Asp Ser Lys
                645                 650                 655

Gly Lys Leu Ala Ala Asp Val Asp Gly Asp Gln Gln Ile Thr Ala Leu
            660                 665                 670

Asp Phe Ser Leu Ile Lys Gln Tyr Leu Leu Gly Thr Ile Asn Lys Phe
        675                 680                 685

Pro Ala Gln Thr Ala Ser Arg Pro Ala Ala Lys Leu Asn
    690                 695                 700

<210>  23
<211>  1893
<212>  DNA
<213>  Clostridium thermocellum

<220>
<223> CBM-c1-c1-d3

<400> 23
atgagaggat ctcaccatca ccatcaccat acggatccgg tatcaggcaa tttgaaggtt 60
gaattctaca acagcaatcc ttcagatact actaactcaa tcaatcctca gttcaaggtt 120
actaataccg gaagcagtgc aattgatttg tccaaactca cattgagata ttattataca 180
gtagacggac agaaagatca gaccttctgg tgtgaccatg ctgcaataat cggcagtaac 240
ggcagctaca acggaattac ttcaaatgta aaaggaacat ttgtaaaaat gagttcctca 300
acaaataacg cagacaccta ccttgaaata agctttacag gcggaactct tgaaccgggt 360
gcacatgttc agatacaagg tagatttgca aagaatgact ggagtaacta tacacagtca 420
aatgactact cattcaagtc tgcttcacag tttgttgaat gggatcaggt aacagcatac 480
ttgaacggtg ttcttgtatg gggtaaagaa cccggtggca gtgtagtacc atcaacacag 540
cctgtaacaa caccacctgc aacaacaaaa ccacctgcaa caacaaaacc acctgcaaca 600
acaataccgc cgtcagatga tccgaatgca ataaagatta aggtggacac agtaaatgca 660
aaaccgggag acacagtaaa tatacctgta agattcagtg gtataccatc caagggaata 720
gcaaactgtg actttgtata cagctatgac ccgaatgtac ttgagataat agagataaaa 780
ccgggagaat tgatagttga cccgaatcct gacaagagct ttgatactgc agtatatcct 840
gacagaaaga taatagtatt cctgtttgca gaagacagcg gaacaggagc gtatgcaata 900
actaaagacg gagtatttgc tacgatagta gcgaaagtaa aatccggagc acctaacgga 960
ctcagtgtaa tcaaatttgt agaagtaggc ggatttgcga acaatgacct tgtagaacag 1020
aggacacagt tctttgacgg tggagtaaat gttggagata caacagtacc tacaacacct 1080
acaacacctg taacaacacc gacagatgat tcgaatgcag taaggattaa ggtggacaca 1140
gtaaatgcaa aaccgggaga cacagtaaga atacctgtaa gattcagcgg tataccatcc 1200
aagggaatag caaactgtga ctttgtatac agctatgacc cgaatgtact tgagataata 1260
gagatagaac cgggagacat aatagttgac ccgaatcctg acaagagctt tgatactgca 1320
gtatatcctg acagaaagat aatagtattc ctgtttgcgg aagacagcgg aacaggagcg 1380
tatgcaataa ctaaagacgg agtatttgct acgatagtag cgaaagtaaa atccggagca 1440
cctaacggac tcagtgtaat caaatttgta gaagtaggcg gatttgcgaa caatgacctt 1500
gtagaacaga agacacagtt ctttgacggt ggagtaaatg ttggagatac aacagaacct 1560
gcaacaccta caacacctgt aacaacaccg acaacaacag atgagctcgt aattgcaaat 1620
gttgtagtaa cgggcgatac ttcagtttca acttcacagg ctccaattat gatgtgggta 1680
ggcgacattg tgaaagacaa ttctatcaac ctgttggacg ttgcagaagt tatccgttgc 1740
ttcaacgcta ctaaaggcag cgcaaactac gtagaagaac ttgacattaa tcgcaacggc 1800
gcaattaaca tgcaagacat tatgattgtt cataagcact ttggcgctac atcaagtgat 1860

tacgtcgacc tgcagccaag cttaattagc tga                                    1893

<210>  24
<211>  630
<212>  PRT
<213>  Clostridium thermocellum

<220>
<223> CBM-c1-c1-d3

<400>  24

Met Arg Gly Ser His His His His His His Thr Asp Pro Val Ser Gly
1               5                   10                  15

Asn Leu Lys Val Glu Phe Tyr Asn Ser Asn Pro Ser Asp Thr Thr Asn
            20                  25                  30

Ser Ile Asn Pro Gln Phe Lys Val Thr Asn Thr Gly Ser Ser Ala Ile
        35                  40                  45

Asp Leu Ser Lys Leu Thr Leu Arg Tyr Tyr Tyr Thr Val Asp Gly Gln
    50                  55                  60

Lys Asp Gln Thr Phe Trp Cys Asp His Ala Ala Ile Ile Gly Ser Asn
65                  70                  75                  80

Gly Ser Tyr Asn Gly Ile Thr Ser Asn Val Lys Gly Thr Phe Val Lys
                85                  90                  95

Met Ser Ser Ser Thr Asn Asn Ala Asp Thr Tyr Leu Glu Ile Ser Phe
            100                 105                 110

Thr Gly Gly Thr Leu Glu Pro Gly Ala His Val Gln Ile Gln Gly Arg
        115                 120                 125

Phe Ala Lys Asn Asp Trp Ser Asn Tyr Thr Gln Ser Asn Asp Tyr Ser
    130                 135                 140

Phe Lys Ser Ala Ser Gln Phe Val Glu Trp Asp Gln Val Thr Ala Tyr
145                 150                 155                 160

Leu Asn Gly Val Leu Val Trp Gly Lys Glu Pro Gly Gly Ser Val Val
                165                 170                 175

Pro Ser Thr Gln Pro Val Thr Thr Pro Pro Ala Thr Thr Lys Pro Pro
            180                 185                 190

Ala Thr Thr Lys Pro Pro Ala Thr Thr Ile Pro Pro Ser Asp Asp Pro
        195                 200                 205

Asn Ala Ile Lys Ile Lys Val Asp Thr Val Asn Ala Lys Pro Gly Asp
    210                 215                 220

Thr Val Asn Ile Pro Val Arg Phe Ser Gly Ile Pro Ser Lys Gly Ile
225                 230                 235                 240

Ala Asn Cys Asp Phe Val Tyr Ser Tyr Asp Pro Asn Val Leu Glu Ile
                245                 250                 255

Ile Glu Ile Lys Pro Gly Glu Leu Ile Val Asp Pro Asn Pro Asp Lys
            260                 265                 270

Ser Phe Asp Thr Ala Val Tyr Pro Asp Arg Lys Ile Ile Val Phe Leu
        275                 280                 285

Phe Ala Glu Asp Ser Gly Thr Gly Ala Tyr Ala Ile Thr Lys Asp Gly
    290                 295                 300

Val Phe Ala Thr Ile Val Ala Lys Val Lys Ser Gly Ala Pro Asn Gly
305                 310                 315                 320

Leu Ser Val Ile Lys Phe Val Glu Val Gly Gly Phe Ala Asn Asn Asp
                325                 330                 335

Leu Val Glu Gln Arg Thr Gln Phe Phe Asp Gly Gly Val Asn Val Gly
            340                 345                 350

Asp Thr Thr Val Pro Thr Thr Pro Thr Thr Pro Val Thr Thr Pro Thr
        355                 360                 365

Asp Asp Ser Asn Ala Val Arg Ile Lys Val Asp Thr Val Asn Ala Lys
    370                 375                 380

Pro Gly Asp Thr Val Arg Ile Pro Val Arg Phe Ser Gly Ile Pro Ser
385                 390                 395                 400

Lys Gly Ile Ala Asn Cys Asp Phe Val Tyr Ser Tyr Asp Pro Asn Val
                405                 410                 415

Leu Glu Ile Ile Glu Ile Glu Pro Gly Asp Ile Ile Val Asp Pro Asn
            420                 425                 430

Pro Asp Lys Ser Phe Asp Thr Ala Val Tyr Pro Asp Arg Lys Ile Ile
        435                 440                 445

Val Phe Leu Phe Ala Glu Asp Ser Gly Thr Gly Ala Tyr Ala Ile Thr
    450                 455                 460

Lys Asp Gly Val Phe Ala Thr Ile Val Ala Lys Val Lys Ser Gly Ala
465                 470                 475                 480

Pro Asn Gly Leu Ser Val Ile Lys Phe Val Glu Val Gly Gly Phe Ala
                485                 490                 495

Asn Asn Asp Leu Val Glu Gln Lys Thr Gln Phe Phe Asp Gly Gly Val
            500                 505                 510

Asn Val Gly Asp Thr Thr Glu Pro Ala Thr Pro Thr Thr Pro Val Thr
        515                 520                 525

Thr Pro Thr Thr Thr Asp Glu Leu Val Ile Ala Asn Val Val Val Thr
    530                 535                 540

Gly Asp Thr Ser Val Ser Thr Ser Gln Ala Pro Ile Met Met Trp Val
545                 550                 555                 560

Gly Asp Ile Val Lys Asp Asn Ser Ile Asn Leu Leu Asp Val Ala Glu
                565                 570                 575

Val Ile Arg Cys Phe Asn Ala Thr Lys Gly Ser Ala Asn Tyr Val Glu
            580                 585                 590

Glu Leu Asp Ile Asn Arg Asn Gly Ala Ile Asn Met Gln Asp Ile Met
        595                 600                 605

Ile Val His Lys His Phe Gly Ala Thr Ser Ser Asp Tyr Val Asp Leu
    610                 615                 620

Gln Pro Ser Leu Ile Ser
625                 630

<210>  25
<211>  1767
<212>  DNA
<213>  Clostridium thermocellum

<220>
<223> c2-c1-c1

<400>  25
atgagaggat ctcaccatca ccatcaccat acggatccta aattgactat caatgtaggt      60

gaaagtggta atacaaatgg tcttaaggtt tcagtaggca cagctgttgg tgctcctggt     120

gatacagtaa cagttcctgt tacatttgct gatgtagcaa aagtaaacaa cgtaggaaca     180

tgtaacttct atcttggcta tgatgcaagt cttttggatg tagtatcagt agatgcaggt     240

ccaattgtta agaatgcagc agtaaacttc tcaagcagtg caagcaacgg cacaatcagc     300

ttcctgttct tggacaacac aatcactgat gaattgatta cttcagatgg tgtgttcgca     360

aatatcacat ttaagattaa gagtactgct acacaaggta caacaccaat taccttcaaa     420

gatggcggtg cttttggtga cggtactatg tcaaagattg cttcagttat taagacaagt     480

ggtagtgtag ttattagtcc agatcctaca aatgctctta aagtaacagt aggcacagca     540

gaaggtaatg ttggcgaaac agtaacagtt cctgttacat ttgcggatcc gcccaccagg     600

ccatcggtac cgacaaacac accgacaaac acaccggcaa atacaccggt atcaggcaat 660

ttgaaggttg aattctacaa cagcaatcct tcagatacta ctaactcaat caatcctcag 720

ttcaaggtta ctaataccgg aagcagtgca attgatttgt ccaaactcac attgagatat 780

tattatacag tagacggaca gaaagatcag accttctggt gtgaccatgc tgcaataatc 840

ggcagtaacg gcagctacaa cggaattact tcaaatgtaa aaggaacatt tgtaaaaatg 900

agttcctcaa caaataacgc agacacctac cttgaaataa gctttacagg cggaactctt 960

gaaccgggtg cacatgttca gatacaaggt agatttgcaa agaatgactg gagtaactat 1020

acacagtcaa atgactactc attcaagtct gcttcacagt ttgttgaatg ggatcaggta 1080

acagcatact tgaacggtgt tcttgtatgg ggtaaagaac ccggtggcag tgtagtacca 1140

tcaacacagc ctgtaacaac accacctgca acaacaaaac cacctgcaac aacaaaacca 1200

cctgcaacaa caataccgcc gtcagatgat ccgaatgcaa taaagattaa ggtggacaca 1260

gtaaatgcaa aaccgggaga cacagtaaat atacctgtaa gattcagtgg tataccatcc 1320

aagggaatag caaactgtga ctttgtatac agctatgacc cgaatgtact tgagataata 1380

gagataaaac cgggagaatt gatagttgac ccgaatcctg acaagagctt tgatactgca 1440

gtatatcctg acagaaagat aatagtattc ctgtttgcag aagacagcgg aacaggagcg 1500

tatgcaataa ctaaagacgg agtatttgct acgatagtag cgaaagtaaa atccggagca 1560

cctaacggac tcagtgtaat caaatttgta gaagtaggcg gatttgcgaa caatgacctt 1620

gtagaacaga ggacacagtt ctttgacggt ggagtaaatg ttggagatac aacagtacct 1680

acaacaccta caacacctgt aacaacaccg acagattgtt cgagctcggt accccgggtc 1740

gacctgcagc caagcttaat tagctga 1767

<210> 26
<211> 588
<212> PRT
<213> Clostridium thermocellum

<220>
<223> c2-c1-c1

<400> 26

Met Arg Gly Ser His His His His His His Thr Asp Pro Lys Leu Thr
1               5                   10                  15

Ile Asn Val Gly Glu Ser Gly Asn Thr Asn Gly Leu Lys Val Ser Val
            20                  25                  30

Gly Thr Ala Val Gly Ala Pro Gly Asp Thr Val Thr Val Pro Val Thr
        35                  40                  45

Phe Ala Asp Val Ala Lys Val Asn Asn Val Gly Thr Cys Asn Phe Tyr
    50                  55                  60

Leu Gly Tyr Asp Ala Ser Leu Leu Asp Val Val Ser Val Asp Ala Gly
65                  70                  75                  80

Pro Ile Val Lys Asn Ala Ala Val Asn Phe Ser Ser Ser Ala Ser Asn
                85                  90                  95

Gly Thr Ile Ser Phe Leu Phe Leu Asp Asn Thr Ile Thr Asp Glu Leu
            100                 105                 110

Ile Thr Ser Asp Gly Val Phe Ala Asn Ile Thr Phe Lys Ile Lys Ser
        115                 120                 125

Thr Ala Thr Gln Gly Thr Thr Pro Ile Thr Phe Lys Asp Gly Gly Ala
    130                 135                 140

Phe Gly Asp Gly Thr Met Ser Lys Ile Ala Ser Val Ile Lys Thr Ser
145                 150                 155                 160

Gly Ser Val Val Ile Ser Pro Asp Pro Thr Asn Ala Leu Lys Val Thr
                165                 170                 175

Val Gly Thr Ala Glu Gly Asn Val Gly Glu Thr Val Thr Val Pro Val
            180                 185                 190

Thr Phe Ala Asp Pro Pro Thr Arg Pro Ser Val Pro Thr Asn Thr Pro
        195                 200                 205

Thr Asn Thr Pro Ala Asn Thr Pro Val Ser Gly Asn Leu Lys Val Glu
    210                 215                 220

Phe Tyr Asn Ser Asn Pro Ser Asp Thr Thr Asn Ser Ile Asn Pro Gln
225                 230                 235                 240

Phe Lys Val Thr Asn Thr Gly Ser Ser Ala Ile Asp Leu Ser Lys Leu
                245                 250                 255

Thr Leu Arg Tyr Tyr Tyr Thr Val Asp Gly Gln Lys Asp Gln Thr Phe
            260                 265                 270

Trp Cys Asp His Ala Ala Ile Ile Gly Ser Asn Gly Ser Tyr Asn Gly
        275                 280                 285

Ile Thr Ser Asn Val Lys Gly Thr Phe Val Lys Met Ser Ser Ser Thr
    290                 295                 300

Asn Asn Ala Asp Thr Tyr Leu Glu Ile Ser Phe Thr Gly Gly Thr Leu
305                 310                 315                 320

Glu Pro Gly Ala His Val Gln Ile Gln Gly Arg Phe Ala Lys Asn Asp
                325                 330                 335

Trp Ser Asn Tyr Thr Gln Ser Asn Asp Tyr Ser Phe Lys Ser Ala Ser
            340                 345                 350

Gln Phe Val Glu Trp Asp Gln Val Thr Ala Tyr Leu Asn Gly Val Leu
        355                 360                 365

Val Trp Gly Lys Glu Pro Gly Gly Ser Val Val Pro Ser Thr Gln Pro
    370                 375                 380

Val Thr Thr Pro Pro Ala Thr Thr Lys Pro Pro Ala Thr Thr Lys Pro
385                 390                 395                 400

Pro Ala Thr Thr Ile Pro Pro Ser Asp Asp Pro Asn Ala Ile Lys Ile
                405                 410                 415

Lys Val Asp Thr Val Asn Ala Lys Pro Gly Asp Thr Val Asn Ile Pro
            420                 425                 430

Val Arg Phe Ser Gly Ile Pro Ser Lys Gly Ile Ala Asn Cys Asp Phe
        435                 440                 445

Val Tyr Ser Tyr Asp Pro Asn Val Leu Glu Ile Ile Glu Ile Lys Pro
    450                 455                 460

Gly Glu Leu Ile Val Asp Pro Asn Pro Asp Lys Ser Phe Asp Thr Ala
465                 470                 475                 480

Val Tyr Pro Asp Arg Lys Ile Ile Val Phe Leu Phe Ala Glu Asp Ser
                485                 490                 495

Gly Thr Gly Ala Tyr Ala Ile Thr Lys Asp Gly Val Phe Ala Thr Ile
            500                 505                 510

Val Ala Lys Val Lys Ser Gly Ala Pro Asn Gly Leu Ser Val Ile Lys
        515                 520                 525

Phe Val Glu Val Gly Gly Phe Ala Asn Asn Asp Leu Val Glu Gln Arg
    530                 535                 540

Thr Gln Phe Phe Asp Gly Gly Val Asn Val Gly Asp Thr Thr Val Pro
545                 550                 555                 560

Thr Thr Pro Thr Thr Pro Val Thr Thr Pro Thr Asp Cys Ser Ser Ser
                565                 570                 575

Val Pro Arg Val Asp Leu Gln Pro Ser Leu Ile Ser
            580                 585

## Description

### Background of the invention

### Field of the invention

The present invention provides an artificial cellulosome for enzymatic breakdown of resilient substrates. In particular, the present invention provides a complex comprising a backbone scaffold having at least four binding sites capable of binding the enzyme components, whereby at least two of the binding sites have essentially the same binding specificity; and at least three different enzyme components being randomly bound to the at least four binding sites. In addition, the present invention relates to a method for preparing the complex. Further, the present invention relates to the use of said complexes as well as the different enzyme components for enzymatic breakdown of resilient substrates, such as cellulose.

### Description of the related art

Cellulose is an abundant renewable source for biotechnology to produce biofuels and building blocks for the chemical industry. Cellulose from lignocellulosic biomass is set to become the largest source of sugar for industrial scale fermentation with the arrival of the upcoming second generation of White Biotechnology. Glucose for industrial fermentation is currently produced primarily from starch. The utilization of cellulose for sugar production would at least double the per hectare yield of agricultural products. In addition, the acreage of arable land could be increased because a greater variety of energy plants would be planted, including those which grow in mediocre soil, under unfavorable climatic conditions, as well as in dry, wet, cold or salt-rich environments.

Cellulose however is a recalcitrant material, refractory both to enzymatic as well as to chemico-physical degradation. Cellulose consists of long fibers of linear molecules without branches. It is chemically a highly homogenous ß-1,4-glucan which forms regular crystals of the form Iα and Iß¹. However, the crystals are not perfectly structured - they are more or less regularly interrupted by amorphous regions. The structural features of cellulose are therefore numerous and require various enzymes.

Present day technology for enzymatic hydrolysis of cellulose has to use large quantities of enzymes usually of fungal origin for the degradation of cellulosic material. These enzymes usually cannot be recycled. Hydrolysis is rather inefficient due to a number of reasons: heterogeneity of the material, complexation with hemicellulose, pectin and lignin, crystallinity of the cellulose, lack of accessibility for enzymes due to tight packaging in cell walls and crystals etc.. This increases the number of different enzyme activities needed for degradation as well as reduces the reaction velocity and therefore the yield of the process. By raising the reaction temperature, the reaction velocity can be increased, but at a cost to enzyme stability. This is especially intricate for the enzyme group of the cellulases which are intrinsically slowly reacting enzymes that have to cope with a crystalline, i.e. insoluble substrate. The diversity of cellulase actions on cellulose explain the multiplicity of the different enzymes involved in cellulase complexes: the structural heterogeneity of the cellulose fibre (crystalline or amorphous, edges or planes etc.), the type of crystal (Iα or Iß), the mode of activity (processive exo-glucanase, processive or non-processive endoglucanase). These enzymes have to function in harmony for efficient degradation of the crystalline substrate, although they all cleave an identical ß-1,4-glucosidic bond.

Commercially available cellulases generally used in the "White Biotechnology" contain a mixture of soluble enzymes of fungal origin. The most successful producers are *Trichoderma longibrachiatum, T. reesei (=Hypocrea jecorina), T. viride (= T. harzianum or Hypocrea atroviridis), Aspergillus niger, Phanerochaete chrysosporium, Chrysosporium lucknowense* and *Penicillium janthinellum.* Some mixtures of cellulases are prepared from two microorganisms such as from *T. longibrachiatum* and *A. niger,* or from *T. longibrachiatum* and *T. reesei.* These fungi produce high amounts of exoproteins in their culture fluid, partially after intensive strain development by selection and by genetic engineering. The cellulases comprise endo-glucanases, cellobiohydrolases (exo-glucanases) and ß-glucosidases, in some strains also a number of hemicellulases.

For this new technology a dramatic increase in demand for enzyme formulations is predicted for the hydrolysis of sustainably produced renewable sugar sources, such as lignocellulosic material. Other sectors of cellulase use are mainly food, textiles, detergents, paper industry and additives to animal feed. Although cellulases already have a big market, future fields of use are generally expected to be dominated by the production of cellulases for the emerging and potentially much bigger market of biomass degradation in the biofuel and bulk chemical biotechnology sector.

However, the different structural characteristics of cellulose crystals and their insoluble nature require the simultaneous presence of several different activities such as processive and non-processive cellulases. The single activities have a lower activity if present alone; only in combination the enzymes show high activity. This difference between single and multiple enzymes in a mixture is called synergism, where the activity of the mixture is higher than the sum of the single activities. However, this synergy is exerted by the fungal enzymes in a soluble mixture of single enzymes which are not "complexed", i.e. packed together by adsorption or in a polypeptide Synergy between soluble, single enzymes precludes the presence of at least two co-working activities at one site of the substrate. In soluble systems this is only possible with a high concentration of enzymes in the mixture. Examples of the limitations of fungal cellulases are the relatively high concentration necessary for enzymatic hydrolysis, the limited thermostability, and the high abortive binding rate. Such limitations have to be overcome for higher performance.

The commercially available cellulases are dominated by fungal enzymes. However, bacterial enzyme systems have also been investigated intensively. The soluble enzymes of *Thermomonospora bispora* or of other thermophilic aerobic bacteria have been discussed as additives for fungal cellulase mixes. Some anaerobic bacteria have been described whose extracellular enzyme systems have a higher specific activity and processivity on cellulose. All of these produce a large extracellular enzyme complex which binds the single enzymes on a scaffolding protein, the so called scaffoldin or Cip (cellulosome integrating protein). These complexes are held together by strong protein-protein interactions which are species specific. The complexes are called cellulosomes. Relatively few bacteria are known to produce cellulosomes. Their list comprises so far:

### In the Phylum Firmicutes:

Lachnospiraceae - *Butyrivibrio fibrisolvens, Ruminococcus flavefaciens, R. albus;*
Clostridiaceae - *Clostridium cellulovorans, C. cellobioparum, C. papyrosolvens, C. josui, C. cellulolyticum, C. thermocellum, C. sp. C7, Bacteroides sp. P-1, B. cellulosovens, Acetivibrio cellulolyticus*;

### In the Phylum Actinobacteria:

Nocardiopsaceae - *Thermobifida (Thermonospora) fusca;*

### In the phylum Fibrobacteres:

Fibrobacteriaceae - *Fibrobacter succinogenes.*

Some improvement of efficiency could be achieved with the strictly anaerobic, thermophilic bacterium *Clostridium thermocellum* which is the microorganism with the fastest growth rate on the recalcitrant substrate crystalline cellulose as it has one of the most efficient enzymatic cellulose degradation systems³. Without being bound to theory, some evidence is accumulating that this higher efficiency over other cellulolytic systems is due to the formation of a huge enzyme complex. The complex has a diameter of ∼18 nm and a mass in excess of 2x10⁶ Da ⁴. About 30 dockerin containing, cellulosome related genes have been more or less accidentally cloned by screening genomic libraries from C. *thermocellum* for enzymatically active clones^{5,6}. In addition, the scaffoldin protein CipA, which contains 9 type I cohesin modules to which enzymes and other protein components specifically dock by virtue of their type I dockerin modules⁷ was identified. Type II cohesin-dockerin interactions anchor the CipA protein to the cell wall bound proteins OIpB or SdbA and possibly to others^{8,9}. The non-enzymatic component CspP is presumably involved in structure formation of the huge complex¹⁰. However, not much is known about the structure of the complex and how it is assembled. Cellulosomes investigated from other bacteria also contain a scaffoldin protein, often with a different architecture.

72 cellulosomal genes were identified in the genomic sequence¹¹ of Clostridium thermocellum ATCC 27405¹². The most prevalent cellulosomal components were identified by proteome analysis of isolated cellulosomes and by mRNA analysis. However, it was by no means clear which of the components were indispensable to cellulose breakdown, and what role the complex formation could play.

Only the partial reconstitution of the cellulosome by construction of small tertiary complexes of a mini-scaffoldin combined with two recombinantly produced enzyme components was possible so far and showed a distinct synergistic effect¹⁴. The mutant isolated by another research group, called AD2, did not absorb to cellulose, but was not characterized in respect to its molecular mechanism, cellulose degrading ability and cellulosome formation^{15,16}. From mutagenized cultures of *C. thermocellum* non-cellulosome-forming mutants were isolated which did not adsorb to crystalline cellulose¹⁷.

*In vitro* assembly of the cellulosome and its single components would nevertheless be necessary to investigate the role of the single genes in cellulolysis and fiber degradation. Such attempts have however failed so far due to the insurmountable difficulty in taking the components apart in their native state - the tight binding in the complex prevents easy separation with mild, non-denaturing methods.

Enzymatic breakdown of insoluble and crystalline material such as crystalline cellulose and heterogenous hemicellulose is still inefficient, slow and requires a high enzyme concentration, which makes industrial exploitation costly and relatively ineffective with present day enzyme preparations.

It is therefore an object of the present invention to provide new enzyme formulations capable of enzymatic breakdown of resilient substrates, such as crystalline cellulose and heterogenous hemicellulose with higher effectivity.

It is another object of the present invention to provide new enzyme formulations as well as effective and cost efficient methods and uses of these enzyme formulations which overcome the drawbacks of the prior art enzyme systems, especially that lower enzyme concentration is needed for the enzymatic breakdown, the thermostability of the enzyme formulation is enhanced, and the binding rate of the enzyme is improved.

### Summary of the invention

According to the present invention, a strong enhancement of activity could be achieved by the complex of the invention as explained in more detail below. The enhancement of the activity of such an enzyme system responsible either for a continuous chain of catalytic events or a synergistic action on resilient substrates, including but not limited to crystalline cellulose and heterogeneous hemicellulose, is demonstrated herein. Surprisingly, the inventors could show that the complexes of the invention, when reconstituted *in vitro*, exhibit higher activity on crystalline cellulose than the native cellulosomes isolated from the bacterium.

The present inventors isolated mutants of *C. thermocellum* which did not form complexes and instead secreted native cellulosomal components in a non-complexed form. These proteins were initially used to reconstitute an artificial cellulosome having enhanced activity. With this mutant the role of synergism could now be investigated for the first time by *in vitro* reconstitution of the complexes.

Thus, the invention provides a complex comprising: (a) a backbone scaffold comprising at least four binding sites, wherein at least two of the binding sites having essentially the same binding specificity; and (b) an enzyme component bound to each of said four binding sites, wherein at least three of said enzyme components are different enzyme components. Preferably, the said enzyme components are randomly bound to the at least four binding sites.

The term "complex" as used herein means a coordination or association of components linked by chemical or biological interaction. Said complexes may be linked together to form a higher order complex (also designated herein with "artificial cellulosome") consisting of one or more cohesin containing backbone scaffolds, preferably cohesin containing scaffolding proteins (also designated herein with "mini-scaffoldin") and one or more dockerin containing enzymatic or non-enzymatic components, as explained in more detail below. Alternatively, the artificial cellulosome consists of one or more dockerin containing backbone scaffolds, preferably dockerin containing scaffolding proteins (also designated herein with "mini-scaffoldin") and one or more cohesin containing enzymatic or non-enzymatic components The term "backbone scaffold" as used herein relates to a support used as a complex backbone which provides for suitable binding sites for enzymatic or non-enzymatic protein components.

The term "having essentially the same binding specificity" when used in reference to the binding sites for the enzyme components refers to the specificity of binding between cohesin and dockerin modules, whereby only cohesin-dockerin pairs of identical binding specificity bind each other. This can be tested e.g. by mixing a pair of proteins and estimating the running behaviour in native gel electrophoresis.

In one embodiment, the invention relates the complex as defined herein, wherein the backbone scaffold is linear. The linear backbone scaffold may be of synthetic or biologic origin. A synthetic backbone scaffold may be for instance a synthetic polymer carrier or a linear organic polymer with functional groups capable of binding proteins. The proteins can be the enzymes to be included in the complex, or proteins containing one or more modules for taking part in cohesin-dockerin interaction (which bind the enzyme components). A biologic backbone scaffold may be a protein having naturally occurring binding sites for the enzyme components or binding sites fused thereto by genetic engineering.

In a further embodiment of the invention, enzyme components are bound to the linear backbone scaffold by a cohesin-dockerin interaction. In preferred embodiment of the invention, the backbone scaffold of the complex of the invention has at least four cohesin binding sites for dockerins.

Preferably the complex of the invention consists of one or more proteins, wherein the one or more proteins are linked together by chemical interaction or by a cohesin-dockerin interaction, whereby the binding specificity of the linking interaction is different from the binding specificity of the enzymes. More preferably, the one or more proteins are linked by a cohesin-dockerin interaction having a binding specificity which is different from the binding specificity of the cohesin-dockerin interaction binding the enzyme components.

The term "cohesin-dockerin interaction" as used herein refers to the interaction between a cohesin and a dockerin molecule. Dockerin is a protein module found in the cellulosome structure, the scaffoldin. The dockerin's binding partner is the cohesin module. This interaction is essential to the construction of the cellulosome complex. For binding the different enzyme components, the same cohesin-dockerin system is used in the complex of the invention. One or more of the complexes of the invention may be linked by cohesin-dockerin interaction; whereby this cohesin-dockerin pair has a different binding specificity than the cohesin-dockerin pair used for binding the enzyme's active site.

In a further embodiment, the backbone scaffold of the complex of the invention is derived from a non-catalytic scaffolding protein from cellulolytic, cellulosome forming microorganisms or genetically modified derivatives thereof.

"Cellulolytic, cellulosome forming microorganisms" as referred herein relates to those bacteria and fungi forming the extracellular complexes wherein enzymes are bound via cohesin-dockerin interaction to the complex scaffold. Further cellulolytic, cellulosome forming microorganisms which may be used in the present invention are: bacteria, such as *Acetivibrio cellulolyticus, Bacterioides cellulosolvens, Butyrivibrio fibrisolvens, Clostridium acetobutylicum, C. cellulolyticum, C. cellulovorans, C. cellobioparum, C. josui, C. papyrosolvens, C. thermocellum, C. sp C7, C. sp P-1, Fibrobacter succinogenes, Ruminococcus albus, R. flavefaciens,* and fungal microorganisms, *such as Piromyces sp. E2.*

In a further embodiment the backbone scaffold is derived from the non-catalytic scaffolding protein CipA from *Clostridium thermocellum* or genetically modified derivatives thereof.

The term "genetically modified derivative" as used herein means that the backbone scaffold of the complex of the invention is genetically modified, for example the backbone scaffold is derived from genetically modified derivatives of the CipA-protein of *C. thermocellum* or the backbone scaffold is genetically fused to a dockerin module or a His-tag sequence, or the number or order of the natural occurring modules (in CipA) is changed, or the nucleotide sequence is changed to introduce or eliminate restriction sites, to adapt the codon usage or to change amino acid residues in certain positions.

In a preferred embodiment the backbone scaffold of the complex of the invention comprises CBM-c1-c1-d3 as shown in SEQ ID NO: 24, c3-c1-c1-d2 as shown in SEQ ID NO: 22, c2-c1-c1 as shown in SEQ ID NO: 26, or derivatives thereof having more than 60 % amino acid sequence identity in their cohesin modules.

The term "sequence identity" known to the person skilled in the art designates the degree of relatedness between two or more nucleotide or polypeptide molecules, which is determined by the agreement between the sequences. The percentage "identity" is found from the percentage of identical regions in two or more sequences, taking account of gaps or other sequence features.

The identity of mutually related polypeptides can be determined by means of known procedures. As a rule, special computer programs with algorithms taking account of the special requirements are used. Preferred procedures for the determination of identity firstly generate the greatest agreement between the sequences studied. Computer programs for the determination of the homology between two sequences include, but are not limited to, the GCG program package, including GAP (Devereux J et al., (1984); Genetics Computer Group University of Wisconsin, Madison (WI); BLASTP, BLASTN and FASTA (Altschul S et al., (1990)). The BLAST X program can be obtained from the National Centre for Biotechnology Information (NCBI) and from other sources (BLAST Handbook, Altschul S et al., NCB NLM NIH Bethesda MD 20894; Altschul S et al.,1990). The well-known Smith Waterman algorithm can also be used for the determination of sequence identity.

Preferred parameters for the sequence comparison include the following:

| | |
|---|---|
| Algorithm: | Needleman S.B. and Wunsch, C.D. (1970) |
| | |
| Comparison matrix: | BLOSUM62 from Henikoff S. and Henikoff J.G. (1992). |
| | |
| Gap penalty: | 12Gap-length penalty: 2 |

The GAP program is also suitable for use with the above parameters. The above parameters are the standard parameters (default parameters) for amino acid sequence comparisons, in which gaps at the ends do not decrease the identity value. With very small sequences compared to the reference sequence, it can further be necessary to increase the expectancy value to up to 100,000 and in some cases to reduce the word length (word size) to down to 2.

Further model algorithms, gap opening penalties, gap extension penalties and comparison matrices including those named in the Program Handbook, Wisconsin Package, Version 9, September 1997, can be used. The choice will depend on the comparison to be performed and further on whether the comparison is performed between sequence pairs, where GAP or Best Fit are preferred, or between one sequence and a large sequence database, where FASTA or BLAST are preferred. An agreement of 60% determined with the aforesaid algorithms is described as 60% identity. The same applies for higher degrees of identity.

In preferred embodiments, the variants according to the invention the derivatives have more than 60 % amino acid sequence identity, preferably more than 70%, more preferably more than 80% or 90% amino acid sequence identity in their cohesin modules.

In a further embodiment of the invention, the backbone scaffold of the complex of the invention comprises a carbohydrate binding module (CBM). Preferably, the carbohydrate binding module is a carbohydrate binding module (CBM3) from the *cipA* gene of *Clostridium thermocellum* that is integrated into or attached to the linear backbone scaffold.

The term "carbohydrate binding module (CBM)" as used herein refers to a substrate binding module. The CBM may either be introduced into the complex ("mini-scaffoldins") of the invention or the CBM is present in the enzyme component, or alternatively may be genetically bound to the mini-scaffoldins via fusion to a dockerin module. The CBM may also be bound to the mini-scaffoldins by genetic modification or chemical reaction with a functional group of the backbone scaffold. The CBM elicits a "targeting effect", i.e. enhancement of binding between complex and substrate, which is particularly advantageous for insoluble substrates. CBMs are defined as a discretely folded non-catalytic polypeptide module, binding to a polysaccharide or a complex carbohydrate. They can be found or genetically engineered to be modularly fused to enzymes or scaffolding proteins, or as a genetic fusion bound to a backbone scaffold via cohesin-dockerin interaction or other means. In the preferred embodiment they bind to crystalline cellulose and belong to CBM family 3 (CBM3). (see: http://www.cazy.org/Carbohydrate-Binding-Modules.html) The binding to a polysaccharide can among other methods be determined by retardation of the protein in native gel electrophoresis in which the polysaccharide is homogeneously distributed in the gel.

In a further embodiment, the invention relates to a complex as defined herein, wherein the enzyme component comprises a dockerin module and a catalytic module of an enzyme.

The term "module" describes a separately folding moiety within a polypeptide which can be used in a "Lego" like fashion to assemble proteins with new characteristics by genetic engineering or natural recombination. The "catalytic module of an enzyme" as used herein refers to a protein module which contributes the catalytic activity to a polypeptide. All enzymes of the cellulosome are multimodular enzymes and consist of catalytic and non-catalytic modules, at least of a catalytic module and a dockerin module. A non-catalytic module may be a dockerin, cohesin, CBM, S-layer homologous module, or a module with yet unknown function (often called X-module).

In a further embodiment, the invention relates a complex as defined herein, wherein the enzyme components are selected from the group consisting of: processive or non-processive endo-β-1,4-glucanases, processive exo-ß-1,4-glucanases and glycosidases from polysaccharolytic or saccharolytic microorganisms or genetically modified derivatives thereof.

The enzyme components combined in the complex of the invention comprise *inter alia* cellulases from the cellulosome of *Clostridium thermocellum,* for example the components CbhA, CeIA, CeIE, CeIJ, CeIK, CeIR, CeIS or CeIT of the thermophilic bacterium *Clostridium thermocellum,* or thermostable β-glycosidases, for example β-glucosidase BgIB from the thermophilic bacterium *Thermotoga neapolitana.*

Exchanging by other activities, taking out or adding enzyme components or changing their molar ratio can extend or enhance the activity of the complexes for other substrates. The new components may include β-glucosidases, hemicellulases (xylanases, mannanases, arabinofuranosidases, glucuronidases, xylan-esterases etc.), pectinases, pectin lyases, amylases and other enzymes for lignocellulosic biomass hydrolysis, other polysaccharides, or the combination of enzymes for a biochemical synthesis pathway.

"Polysaccharolytic microorganisms" as used herein refer to hydrolytic microorganisms capable of degrading polysaccharides, such as amylolytic, pectinolytic, cellulolytic or hemicellulolytic microorganisms. "Saccharolytic microorganisms" as used herein refer to microorganisms using carbohydrates as primary source of carbon and energy.

In a further embodiment, the invention relates a complex as defined herein, wherein the at least three enzyme components are selected from the group consisting of cellulolytic and hemicellulolytic enzymes from other microorganisms.

Examples for polysaccharides are acetan, agar-agar, alginate, amylopectin, arabinan, arabinogalactan, arabinoxylan, carboxymethyl cellulose, cellulose, chitin, chitosan, chrysolaminarin, curdlan, cyclosophoran, dextran, dextrin, emulsan, fructan, galactan, galactomannan, gellan, α-glucan, β-glucan, glucuronan, glucuronoxylan, glycogen, N-acetyl-heparosan, hydroxyethyl cellulose, indican, inulin, kefiran, laminarin, lentinan, levan, lichenin, lichenan, lupin, mannan, pachyman, pectic galactan, pectin, pentosan, pleuran, polygalacturonic acid, pullulan, rhamnogalacturonan, schizophyllan, scleroglucan, starch, succinoglycan, welan, xanthan, xyloglucan, zymosan.

Examples of cellulolytic microorganisms are bacteria such as *Acetivibrio cellulolyticus, A. cellulosolvens, Anaerocellum thermophilum, Bacteroides cellulosolvens, Butyrivibrio fibrisolvens, Caldicellulosiruptor saccharolyticus, Cs. lactoaceticus, Cs. kristjansonii, Clostridium acetobutylicum, C. aldrichii, C. celerescens, C. cellobioparum, C. cellulofermentas, C. cellulolyticum, C. cellulosi, C. cellulovorans, C. chartatabidum, C. herbivorans, C. hungatei, C. josui, C. papyrosolvens, C. sp C7, C. sp P-1, C. stercorarium, C. thermocellum, C. thermocopriae, C. thermopapyrolyticum, Fibrobacter succinogenes, Eubacterium cellulolyticum, Ruminococcus albus, R. flavefaciens, R. succinogenes, Achromobacter piechaudii, Actinoplanes aurantiaca, Bacillus circulans, Bacillus megaterium, Bacillus pumilus, Caldibacillus cellulovorans, Cellulomonas biazotea, Cm. cartae, Cm. cellasea, Cm. cellulans, Cm. fimi, Cm. flavigena, Cm. gelida, Cm. iranensis, Cm. persica, Cm. uda, Cellvibrio fulvus, Cv. Gilvus, Cv. Mixtus, Cv. vulgaris, Curtobacterium falcumfaciens, Cytophaga sp., Flavobacterium johnsoniae, Microbispora bispora, Micromonospora melonosporea, Myxobacter sp. AL-1, Pseudomonas fluorescens, Ps. mendocina, Streptomyces alboguseolus, Sm. antibioticus, Sm. aureofaciens, Sm. cellulolyticus, Sm. flavogriseus, Sm. lividans, Sm. nitrosporeus, Sm. olivochromogenes, Sm. reticuli, Sm. rochei, Sm. thermovulgaris, Sm. viridosporus, Sporocytophaga myxcoccoides, Thermoactinomyces sp. XY, Thermobifida alba, Tb. cellulolytica, Tb. fusca, Thermonospora curvata, Xanthomonas sp. ;* fungi, such as *Anaeromyces mucronatus, Aspergillus niger, Caesomyces comunis, Cyllamyces aberensis, Hypocrea sp., Neocallimastix frontalis, Orpinomyces sp., Phanerochaete chrysosporium, Piptoporus cinnabarinus, Piromyces sp., Piromyces equi, Piromyces sp. E2, Rhizopus stolonifer, Serpula lacrymans, Sporotrichum pulverulentum, Trichoderma (=Hypocrea) harzianum, T. koningii, T. longibrachiatum, T. pseudokoninii, T. reesei, T. viride.*

In a further embodiment, the complex of the invention comprises at least three enzyme components derived from dockerin containing components of *Clostridium thermocellum* or from components of *Thermotoga maritima* having dockerin fused thereto. The complex of the invention may also comprise dockerin containing enzyme components or enzyme components having dockerin fused thereto from other bacteria.

In a preferred embodiment, the enzyme components comprise CeIK-d1 as shown in SEQ ID NO: 8, CeIR-d1 as shown in SEQ ID NO: 10, CeIT-d1 as shown in SEQ ID NO: 14, CeIE-d1 as shown in SEQ ID NO: 16, CeIS-d1 as shown in SEQ ID NO: 6 and BgIB-d1 as shown in SEQ ID NO: 4, or derivatives thereof or related genes from other bacteria having more than 50 %, preferably more than 60 or 70 %, more preferably 70 or 95 % amino acid sequence identity.

In a further embodiment, the complex of the invention comprises a backbone scaffold comprising the proteins CBM-c1-c1-d3 as shown in SEQ ID NO: 24, c3-c1-c1-d2 as shown in SEQ ID NO: 22, c2-c1-c1 as shown in SEQ ID NO: 26 and the enzyme components comprising CeIK-d1 as shown in SEQ ID NO: 8, CeIR-d1 as shown in SEQ ID NO: 10, CeIT-d1 as shown in SEQ ID NO: 14, CeIE-d1 as shown in SEO ID NO: 16, CeIS-d1 as shown in SEQ ID NO: 6 and BgIB-d1 as shown in SEQ ID NO: 4.

The invention further provides a method for preparing the complex as defined herein comprising the steps:
a) recombinantly producing the at least three enzyme components as defined herein,
b) recombinantly producing the backbone scaffold of any one of claims 1 to 8,
c) mixing the purified, partially purified or non-purified components of a) and b); and
d) randomly binding the enzyme components to the backbone scaffold.

The recombinant production of the enzyme components can be performed by gene cloning and modification techniques well known in the art, for example the engineered enzyme components may be fused to dockerin, cohesin and/or other non-catalytic modules, optionally followed by protein engineering of the components to enhance recombinant production, for example by optimizing the secretion signals, changing protein segments decreasing successful expression or secretion, or the codon usage. In a further step the backbone scaffolds ("mini-scaffoldins") of the invention were recombinantly produced, optionally fused cohesin modules and spontaneously combined to form the complex by mixing the enzyme components and the backbone scaffolds. The components may be purified, partially purified or non-purified, preferably partially purified or non-purified. Preferably, the molar ratio of cohesin and dockerin modules in the enzyme-backbone scaffold mixture is 1:1. The at least three enzyme components are mixed together in a molar ratio of 1:1 to 1:15, preferably 1:1 to 1:8. The enzyme components thereby randomly bind to the backbone scaffold. The dockerin and cohesin modules of the cohesin-dockerin interaction as defined herein are interchangeable, that means the cohesin modules may either be present in the backbone scaffold or in the enzyme component.

In a further embodiment, the invention provides a method for preparing the complex of the invention as defined herein above, wherein the total amount of backbone scaffolds in step c) and the total amount of enzyme components are mixed together in a molar ratio of 1 cohesin module to 1 enzyme component, and the at least three enzyme components are mixed together in a molar ratio of 1:1 to 1:15 to each other.

The "molar ratio" as far as it relates to the ratio of backbone scaffolds to enzyme components is calculated as molar ratio of 1 binding site, preferably a cohesin module comprised in the backbone scaffold to 1 binding site, preferably a dockerin module comprised in the enzyme component.

In a further embodiment, the invention provides a method for enzymatic hydrolysis of polysaccharide substrates comprising the steps of:
a) mixing the complex of the invention with insoluble cellulose; and optionally
b) isolating the degradation products.

Mixing the complex of the invention with insoluble cellulose is preferably performed in water environment at the optimal or near the optimal pH and temperature. The optimal or near optimal pH is 6,5 ± 0,5. The optimal temperature is in the range of 30-65 °C, preferably 55 °C.

In a further embodiment, the invention provides the use of the complex of the invention for enzymatic hydrolysis of polysaccharide substrates.

In a preferred embodiment of the invention, the polysaccharide substrate as described herein above is crystalline cellulose or a crystalline cellulose containing substrate.

### Brief description of the drawings

**Figure 1** shows colonies of a selected non-adsorbing culture of C. thermocellum. On a turbid agar surface colonies surrounded by a dark (= cleared) halo of hydrolyzed cellulose are visible. The ruler shows 1 and 2 cm markings.
**Figure 2** illustrates the approximate 3D-structure (curtesy H. Gilbert) of the recombinant scaffoldin constructs. Derivatives of the CipA-protein of *C. thermocellum* (top row: c1 = cohesin 1 etc.; CBM = carbohydrate binding module)
**Figure 3** shows the specific activity [mU/mg as glucose equivalents] of complexes with native soluble cellulosomal components from the mutant SM1 (SM901) and mini-scaffoldins as well as complete native scaffoldin CipA of *C. thermocellum.* Control: native cellulosome from *C.*
*thermocellum.* Determination of activity on 0.5% Avicel (T = 60 °C, pH 6.0). (Coh: Cohesin,
CBM: Carbohydrate-Binding-Module).
**Figure 4** illustrates the nanoparticle-linker scaffoldin-cellulase complex (NLSC). For simplicity the scaffoldin is shown with only 2 cohesins (instead of 9). The nanoparticles and the various molecules are not drawn to scale.
**Figure 5** shows the activity of various complexes with and without nanoparticle-binding. SM901: the mutant cellulosomal components without scaffoldin.
**Figure 6** compares pH-stability of free enzymes (SM901) and enzymes bound to nanoparticles (NP+SM901).

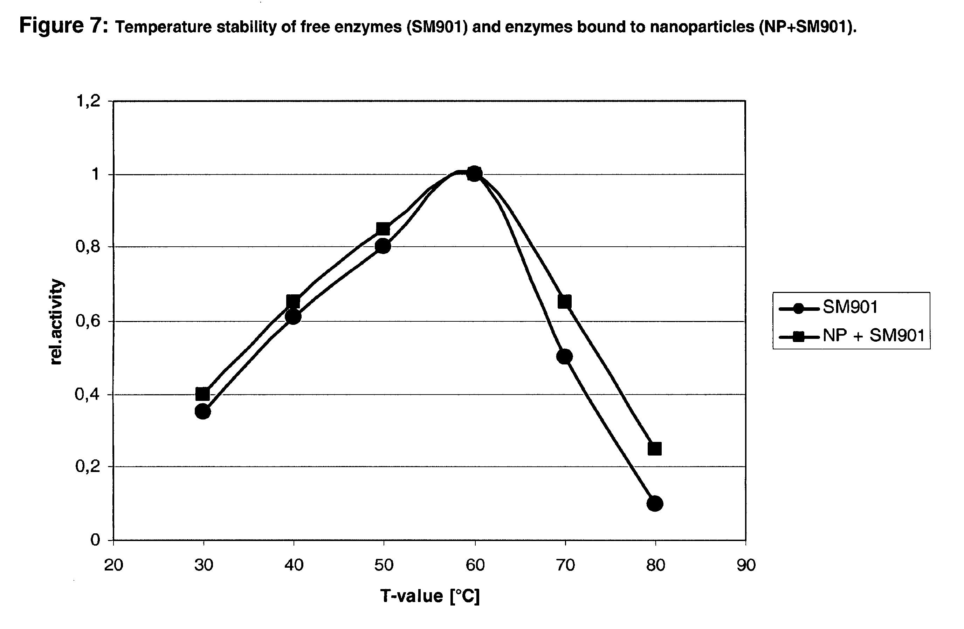

**Figure 7** compares the temperature stability of free enzymes (SM901) and enzymes bound to nanoparticles (NP+SM901).

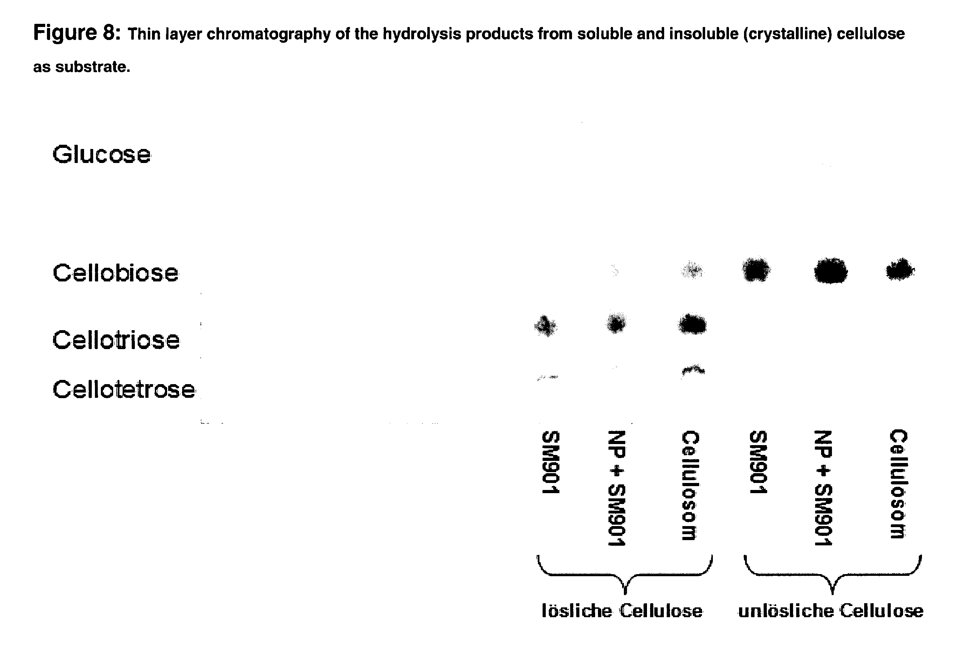

**Figure 8** shows a thin layer chromatography of the hydrolysis products from soluble (CMC) and insoluble (crystalline) cellulose (Avicel) as substrate. A comparison of soluble enzymes (SM901), artificial complexes and native cellulosome is shown.
**Figure 9** shows the activity of various mixtures of recombinant cellulosomal components with scaffolding protein. Crystalline cellulose is used as substrate.

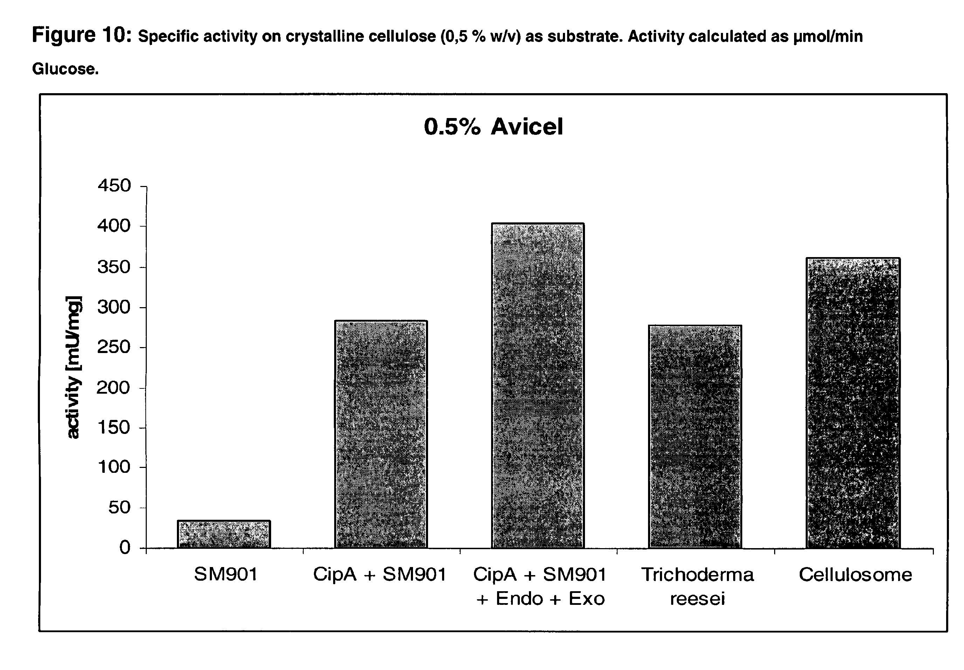

**Figure 10** shows the specific activity of the soluble cellulosomal components (SM901), the same enzymes in complex form (with scaffoldin), the synthetic mixture with recombinant components (SM901 + CipA + Endo + Exo [NTC]), a commercially available *Trichoderma reesei* enzyme preparation, and the native cellulosome from *Clostridium thermocellum.* Crystalline cellulose (0,5 % w/v) as substrate. Activity calculated as µmol/min as glucose equivalents.
**Figure 11** shows the nucleotide sequence and the amino acid sequence of the backbone scaffolds CBM-c1-c1-d3, c3-c1-c1-d2, c2-c1-c1 and the enzyme components CeIK-d1, CeIR-d1, CeIT-d1, CeIE-d1, CeIS-d1 and BgIB-d1.

### Examples

The invention will be further explained by the following Examples, which are intended to be purely exemplarily of the invention, and should not be considered as limiting the invention in any way.

The examples demonstrate the hydrolytic cellulose degradation by a system of 6 recombinantly expressed cellulases which are bound to a protein carrier, a backbone scaffold imitating the scaffoldin CipA (cellulosome integrating protein). The backbone scaffold carries cohesin binding modules which bind tightly and specifically to dockerin modules forming the C-terminus of the enzyme components. This is an *in vitro* assembled complex resembling the cellulosome of the thermophilic anaerobic bacterium *Clostridium thermocellum.* The cohesins and dockerins bind each other spontaneously. Such complexes, when reconstituted *in vitro* with the right components in the correct ratio, exhibit higher activity on crystalline cellulose than the native cellulosomes isolated from the bacterium.

### Example 1: Isolation of a non-cellulosome-forming mutant

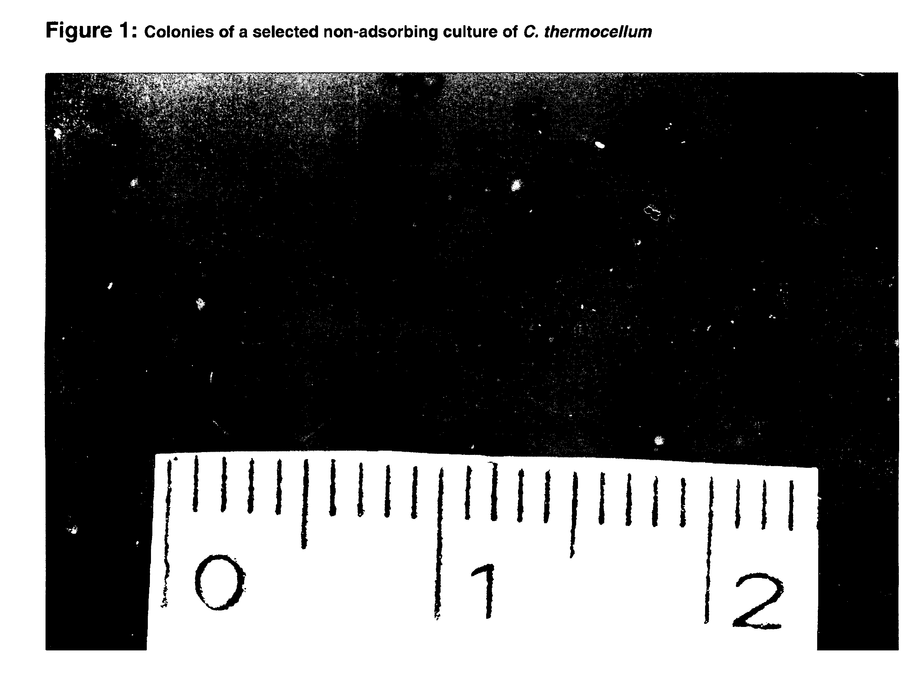

From mutagenized cultures of *C. thermocellum* mutants were isolated (Fig. 1). Six colonies with a reduced or absent ability to form clear halos in the cellulose around the colonies were randomly selected. One of the *C. thermocellum* mutants, SM1, had completely lost the ability to produce a scaffoldin protein or an active cohesin. Enzymes from wild type (having cellulosomes) and mutant SM1 (no cellulosomes; free enzymes) were tested on barley β-glucan, CMC (both control), and micro-crystalline cellulose MN300. The enzymatic activity on barley β-glucan and CMC were about 8.5 and 1.0 U mg⁻¹ protein respectively for both strains (Table 1). In contrast, specific activity on crystalline cellulose was dramatically reduced in the mutant SM1, up to 15 fold as compared to the wild type.

The mutant produced the cellulosomal components in approximately equal amounts compared to the wild type, with the exception of the CipA component (the scaffoldin CipA) which was completely missing. Supramolecular complexes were completely missing in the mutant. This indicates an inability of the mutant to properly form cellulosomes. The approximately 50 cellulosomal protein components consequently appeared as dispersed, soluble, non-complexed proteins which are produced in an amount and distribution similar to that observed for the wild type.

**Table 1: Enzymatic activity of concentrated culture supernatants of the mutant SM1 and the wildtype on barley β-glucan, CMC (both soluble) and MN300 cellulose (crystalline).**

| | **SM1** (U mg⁻¹ protein) | **Wt** (U mg⁻¹ protein) |
|---|---|---|
| β-Glucan | 7,9 ± 1,1 | 9,5 ± 0,9 |
| CMC | 1,1 ± 0,1 | 1,2 ± 0,1 |
| MN300 (x 100) | 0,3 ± 0,1 | 4,4 ± 0,1 |

### Example 2: Reconstitution of the cellulosome

### A: Preparation of enzyme components

The mutant SM01 and the mutant supernatant proteins (SM901) were selected to reconstitute an artificial cellulosome. All genes coding for presumable cellulase components were cloned and characterized for their biochemical parameters such as pH and temperature optimum and activity on different substrates. The five most prominent enzyme components with cellulase activity were selected from previous data on the composition of the cellulosome¹¹. In addition, β-glucosidases derived from a number of thermophilic saccharolytic bacteria were biochemically characterized. The β-glucosidase BgIB from *Thermotoga maritima* was selected due to its high thermostability and high activity on cellodextrins. The gene was fused to a downstream dockerin module, preferably the dockerin sequence of the *C. thermocellum* cellulase CelA. Optimal expressin conditions were determined. The enzymes containing catalytic and non-catalytic modules including a dockerin module formed thereby are herein designated with CeIK-d1, CeIR-d1, CeIT-d1, CeIE-d1, CeIS-d1 and BgIB-d1. The amino acid sequences of CeIK-d1, CeIR-d1, CeIT-d1, CeIE-d1, CeIS-d1 and BgIB-d1 are shown in SEQ ID NO: 4, 6, 8, 10, 14 and 16.

For easier purification they were cloned with an N-terminal His-tag to allow for an easy purification by affinity chromatography. The State of the art technology was used to clone the amplified DNA fragments in frame into a restriction site downstream of a promoter-operator sequence and a 6xHis sequence (e.g. pQE vector from Quiagen).

The stoichiometry of the components was kept in balance by calculating the number of cohesins and dockerins.

### B: Preparation of the backbone scaffolds

The mini-scaffoldins described hereafter were constructed by combining cohesin, dockerin and CBM sequences from the CipA gene of *C. thermocellum,* from *C. thermocellum* cellulosomal components and from *C. josui.* The sequences were optimized for the codon usage of *E. coli,* i.e. most rare codons for the scarcily expressed tRNA genes *argU, ileY,* and *leuw,* which recognize the AGA/AGG, AUA, and CUA codons, were displaced by synonymous codons. The sequences thus derived were synthesized and expressed in *E. coli* plasmid vectors, such as pQE, according to the art. In one embodiment the cohesins 3-4 (type I) and CBM3 from *cipA* were used as well as cohesin c3 (type II) and dockerin d3 from *olpB* of *C. thermocellum,* or from *cipA* c2 and dockerin d2 from cellulosomal components of *Clostridium josui.* The backbone scaffolds used in the Example are designated herein with CBM-c1-c1-d3, c3-c1-c1-d2, c2-c1-c1. The amino acid sequences of CBM-c1-c1-d3, c3-c1-c1-d2, c2-c1-c1 are shown in SEQ ID NO: 22, 24 and 26.

### C: Expression, purification and enrichment of dockerin-enzyme components

The enzyme components were initially produced by the mutant SM1. However, *C. thermocellum* can produce only a limited amount of exoprotein due to energy limitations in its anaerobic life style. Even strain development will not lead to a significant increase in the amount of exoproteins. To replace the native enzyme mixture of more than 30 components by an artificial mixture of cellulases, the major cellulosomal components were prepared from a recombinant host. For experimental simplicity *E. coli* was used as host. Other bacteria may be better suited for a low cost, high yield production of recombinant proteins. Any industrial producer strain with high yield for a given protein will be appropriate.

The enzyme components were isolated from the recombinant host and purified by His-tag affinity chromatography, or enriched by heat precipitation of *E. coli* proteins whereby the recombinant proteins remain in the soluble phase. Heat precipitation was performed by heating the protein solution to 65 °C for 10 min and removing the precipitated *E. coli* proteins by centrifugation. Enrichment was also successful by ultrafiltration (cutoff 10.000 Dalton).

### D: Complexation of enzyme components with the backbone scaffold

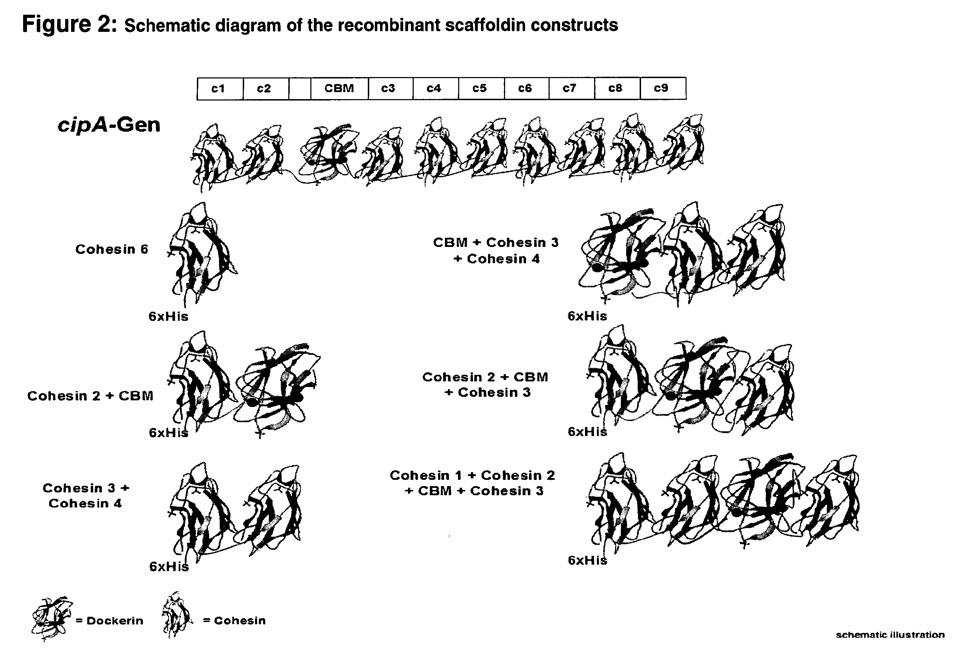

They were then bound to recombinant mini-scaffoldins consisting of various cohesins with or without a carbohydrate binding module, for example CBM-c1-c1. The complex can be reconstituted from the components by simple stoichiometric addition to the mixture of enzyme components (one dockerin bearing component per cohesin module present in the mixture) in the presence of calcium, in one embodiment 20 mM CaCl₂. Sample structures of the recombinant mini-scaffoldin constructs are depicted in Fig. 2. Complexation occurs by spontaneously combining the dockerin-enzyme components with mini-scaffoldins consisting of various cohesins via cohesin-dockerin interaction. They were then used to measure the effect of complexation, either with native enzymes or with enzymes isolated from recombinant hosts.

### Example 3: Activity of the artificial cellulosome complex

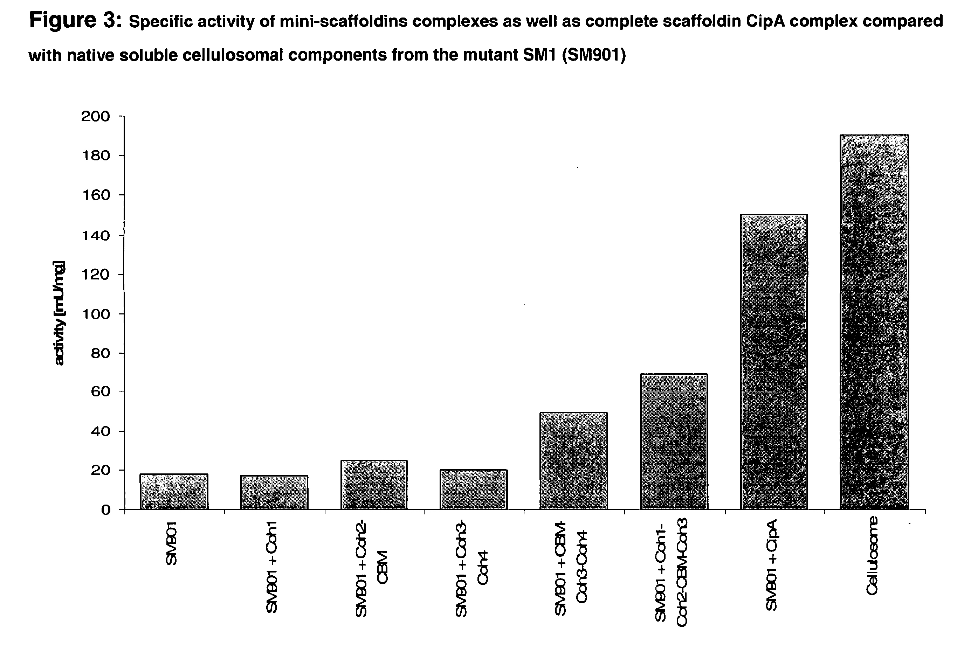

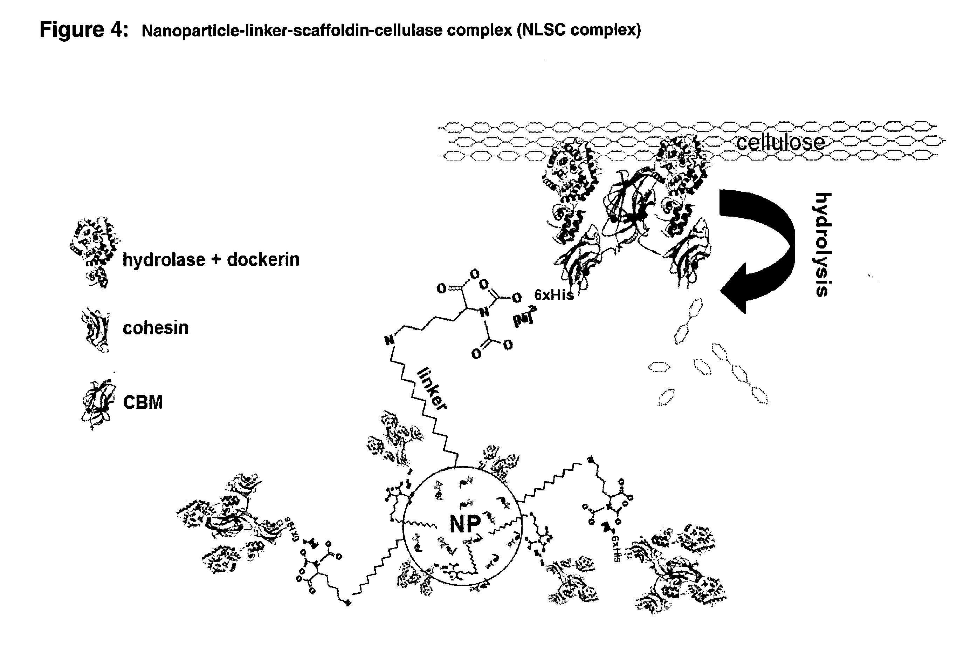

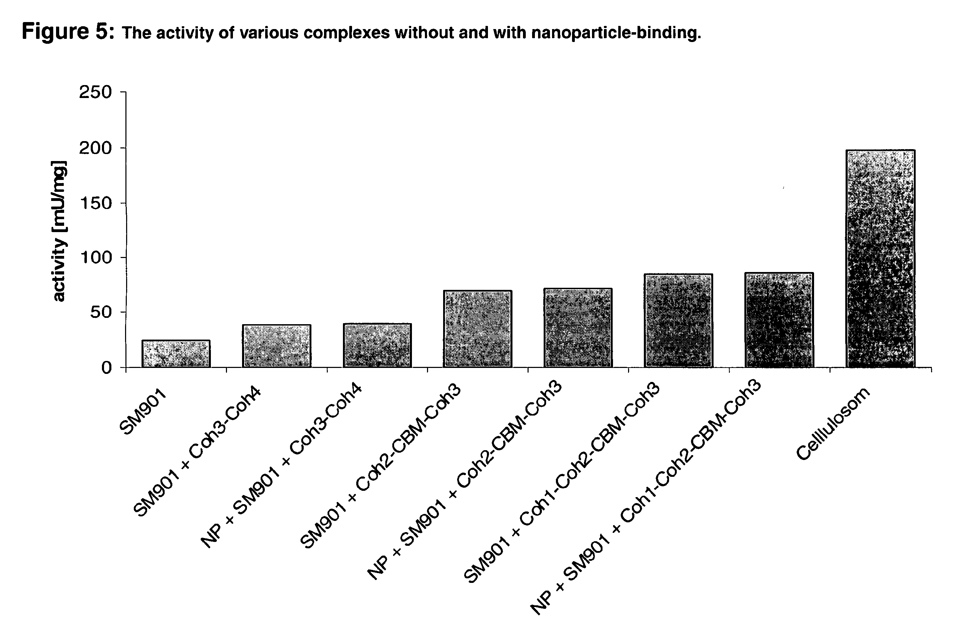

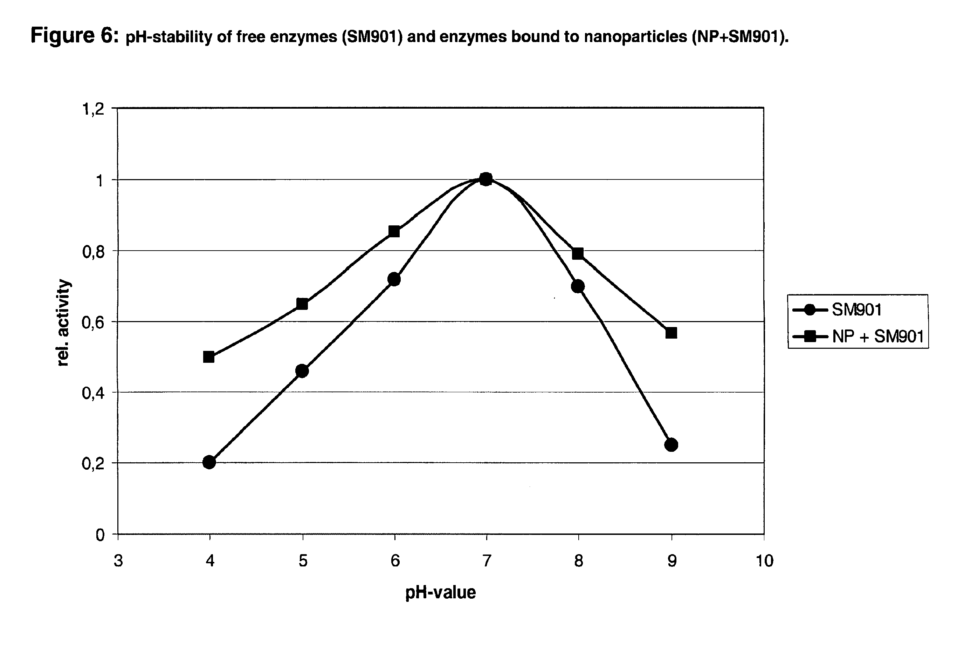

A mixture of such complexes with and without CBMs were now bound via an optimized aliphatic linker molecule on the surface of polystyrene nano-particles. Such structures are schematically shown in Fig. 3. The binding of various mini-scaffoldin complexes on hydrolysis of crystalline cellulose resulted in an increase in activity despite the sterical hindrance and a certain loss of degree of freedom of the enzyme components due to the dense covering of the nanoparticles (Fig. 3, 4). In addition, the pH range of the enzymes was broader if the proteins were bound to the particle (Fig. 5). This was also true for the temperature stability of the cellulases (Fig. 6). Both of these results are an important advantage for technical application.

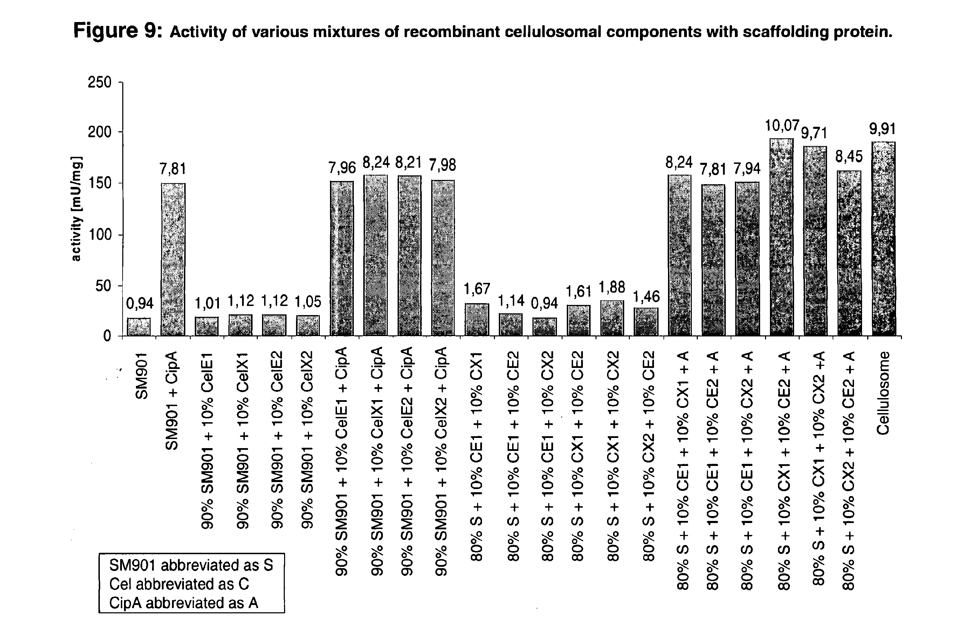

To test the feasibility of that approach, a part of the SM901 component mixture was replaced by one or more of the recombinant cellulases. Despite the decrease of SM901 components in the mixture, the result showed the activity of the complex on crystalline cellulose was slightly increasing when one recombinantly produced component was added. It increased significantly when a mixture of recombinant enzymes was added (Fig. 9). In certain mixtures the activity of synthetic mixtures was higher than that of the native cellulosome. By complete replacement and properly balanced stoichiometry, the synthetic complexes exhibit even higher activity.

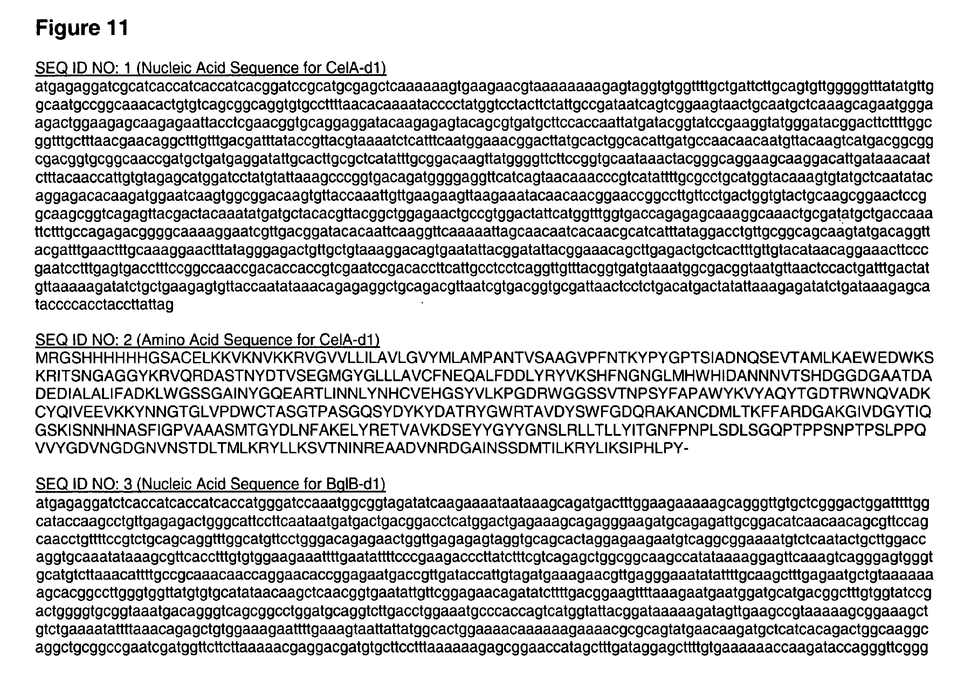

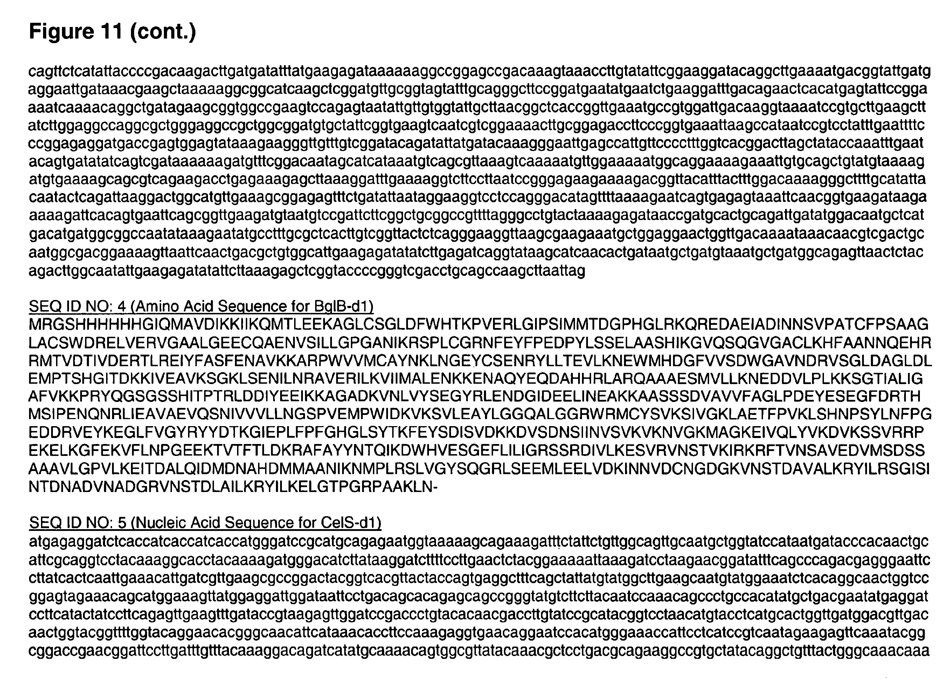

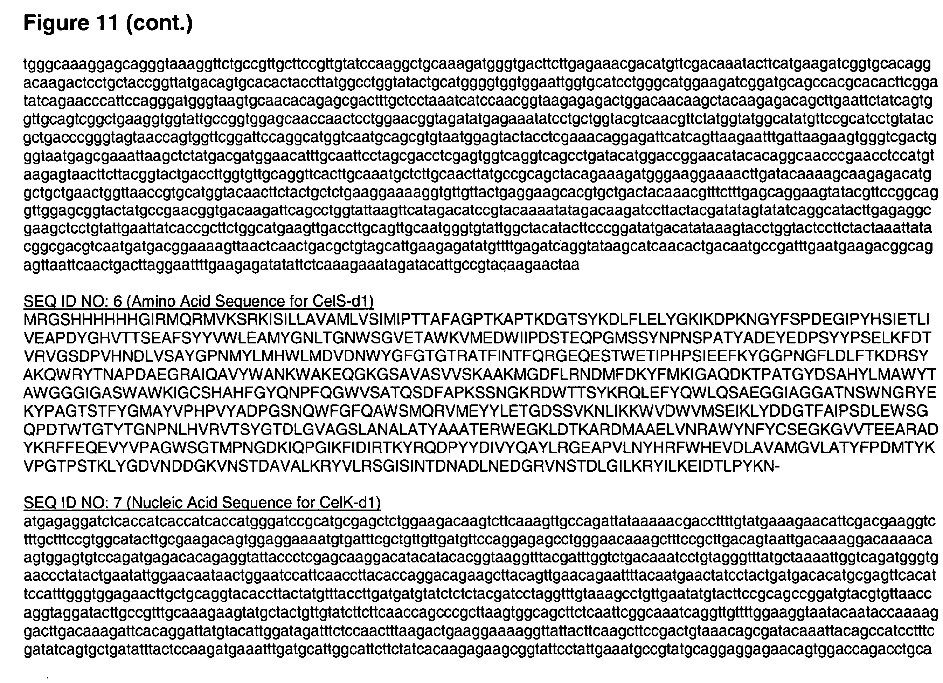

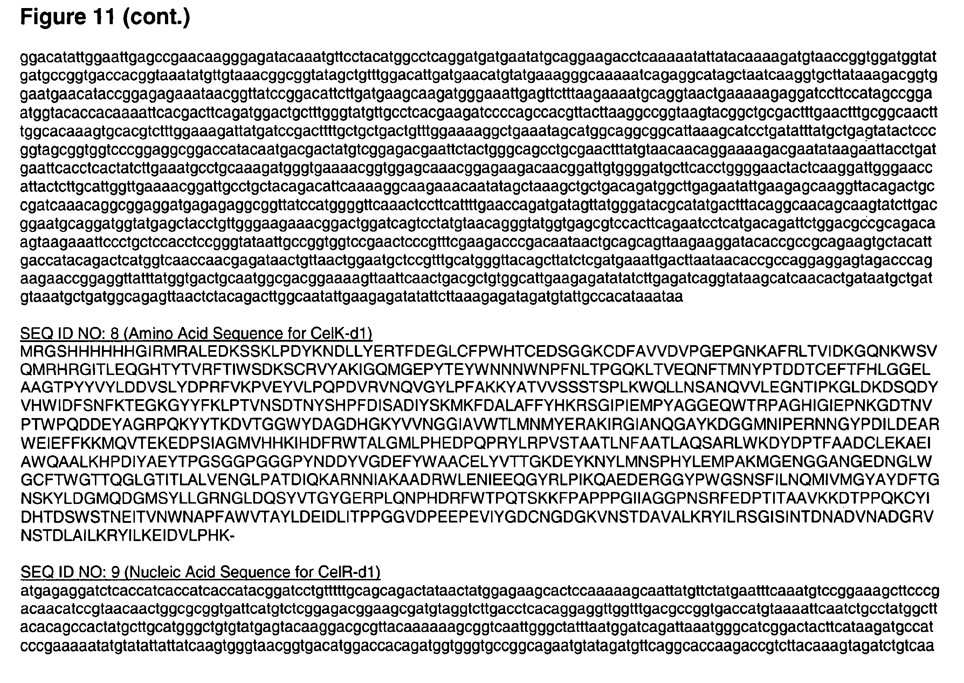

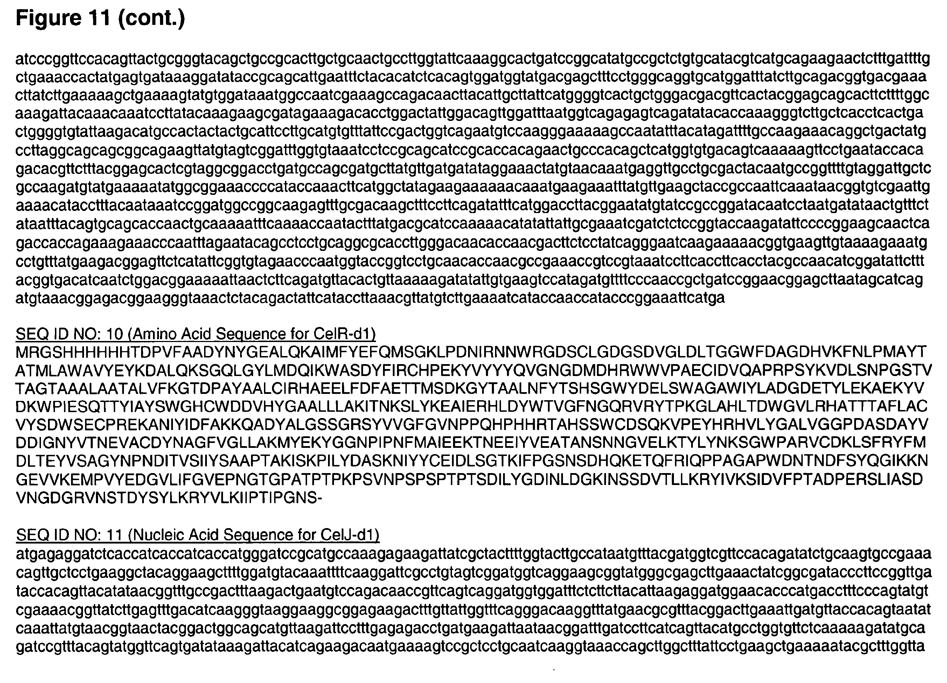

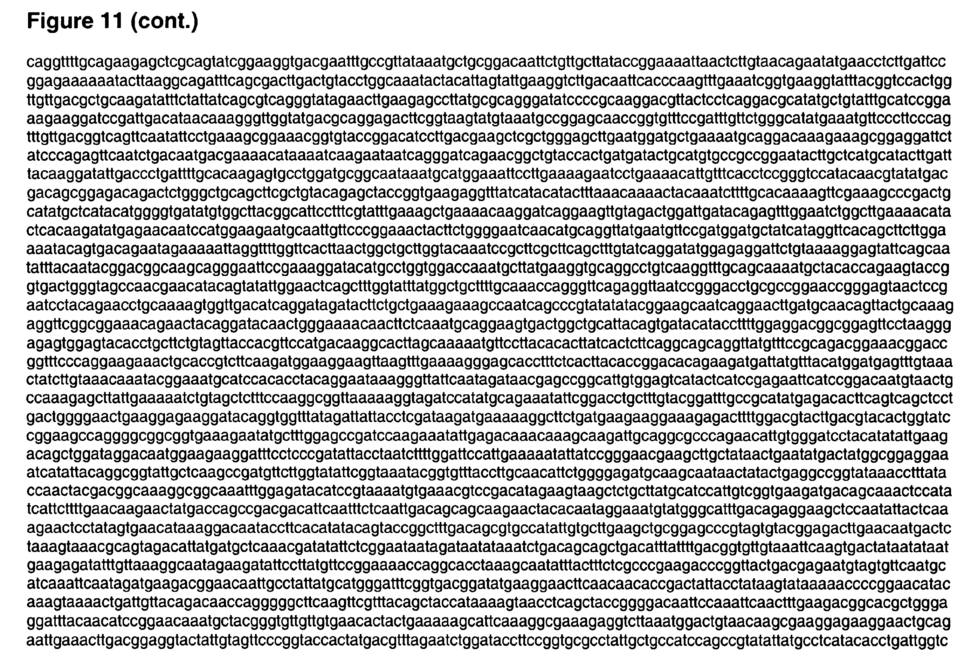

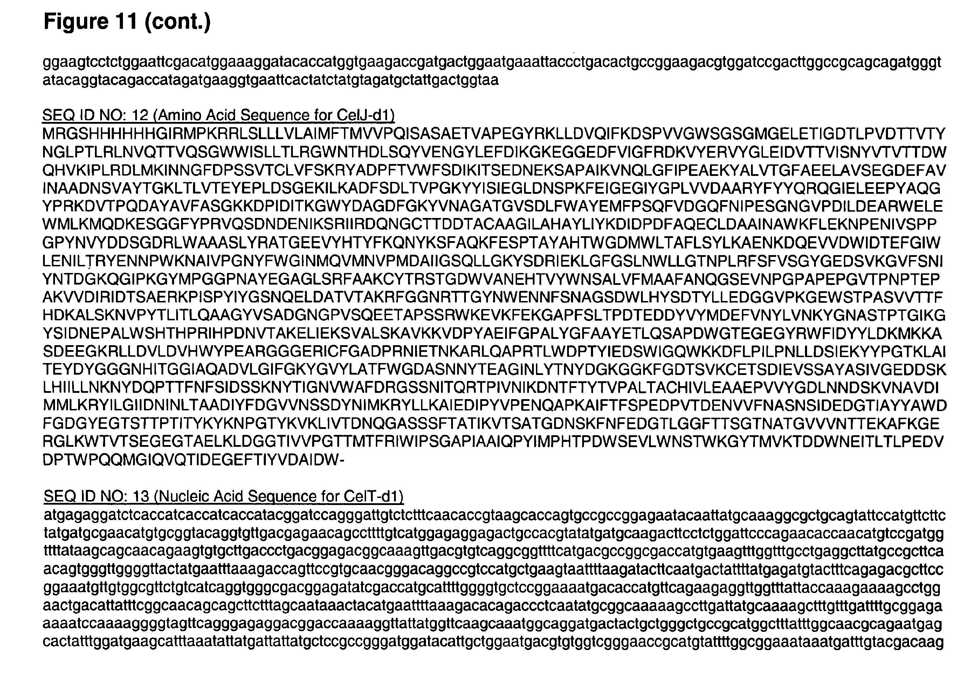

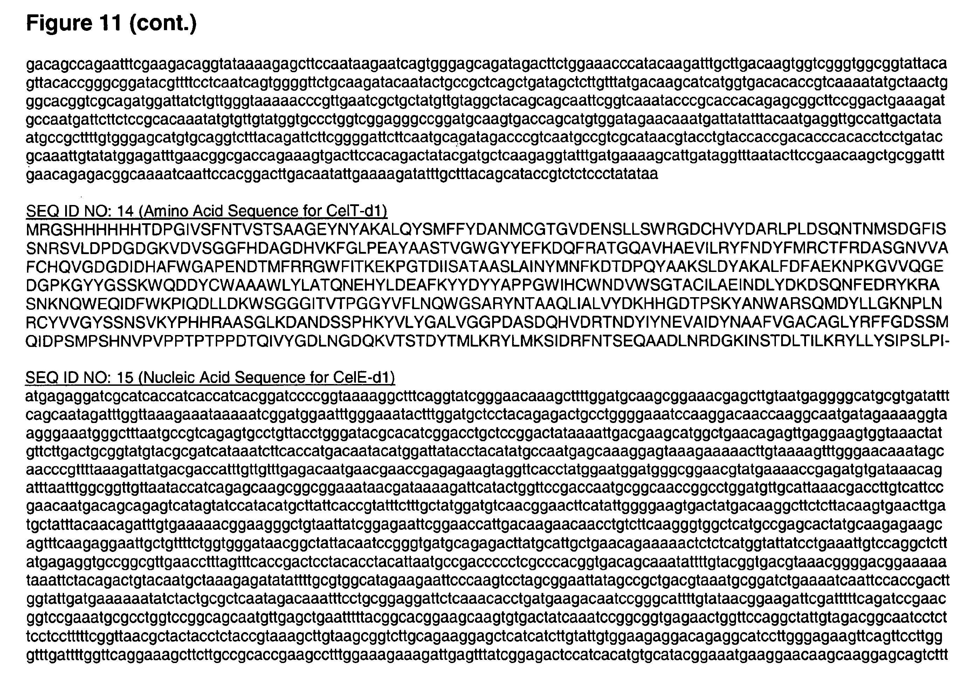

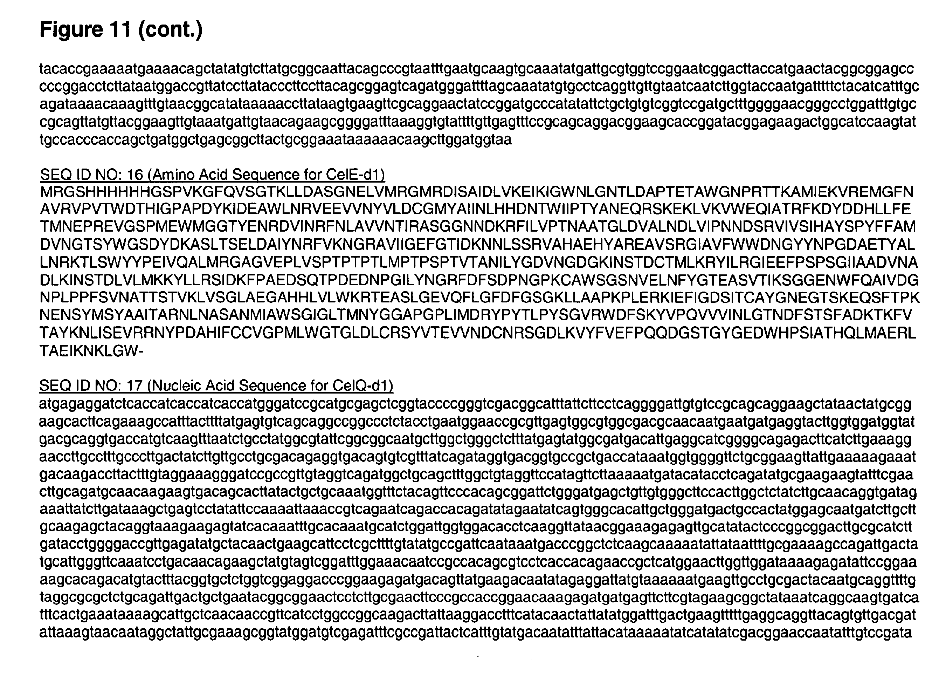

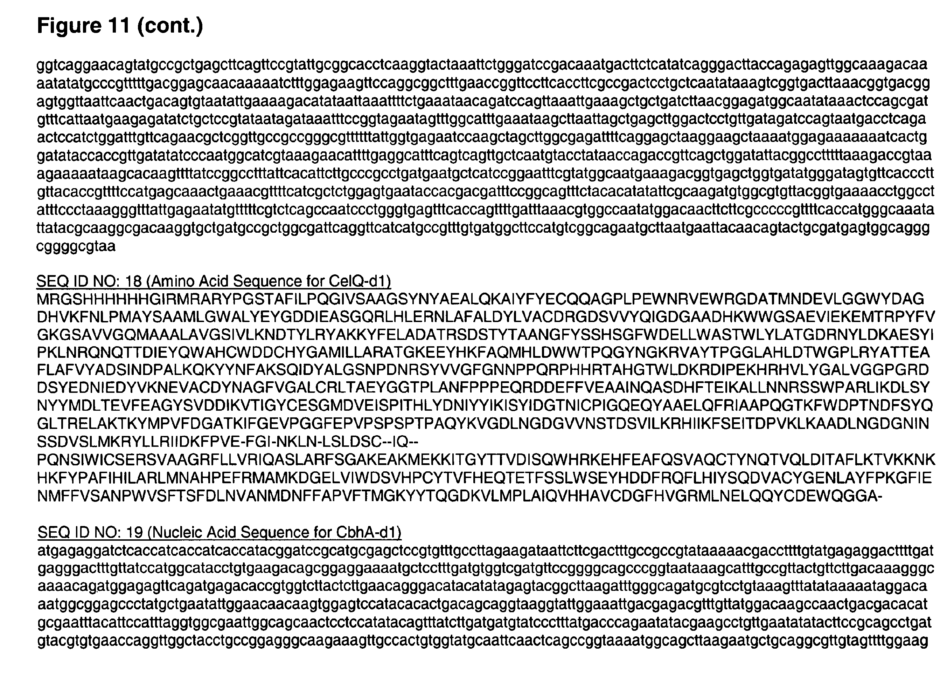

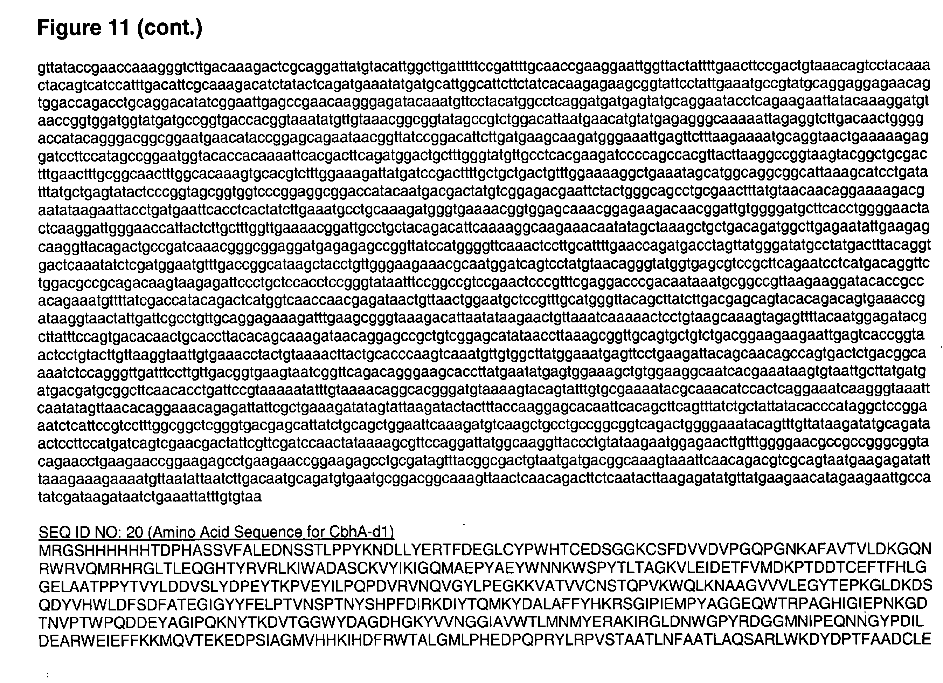

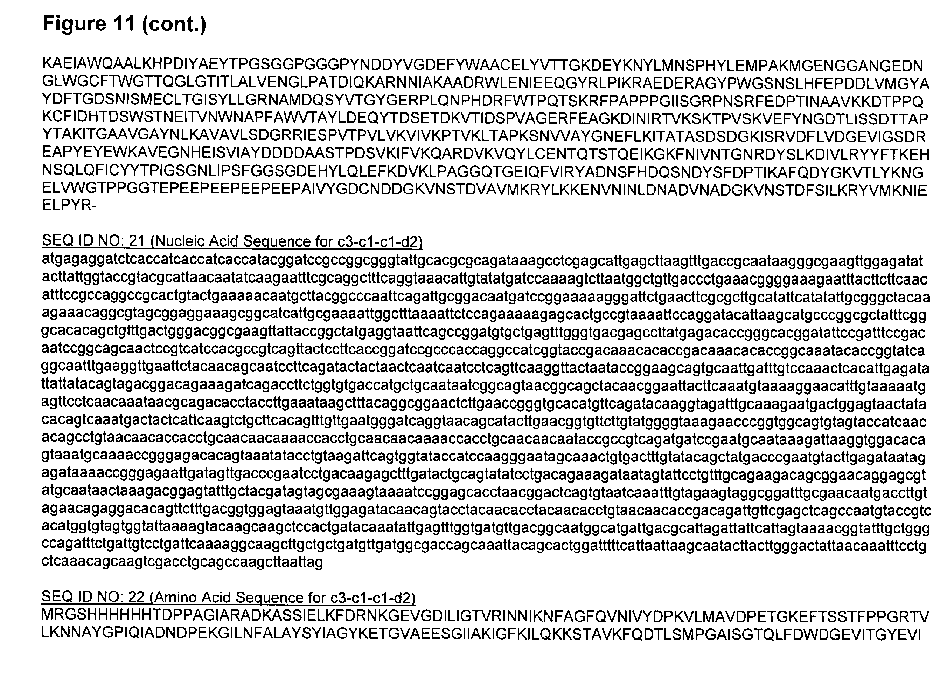

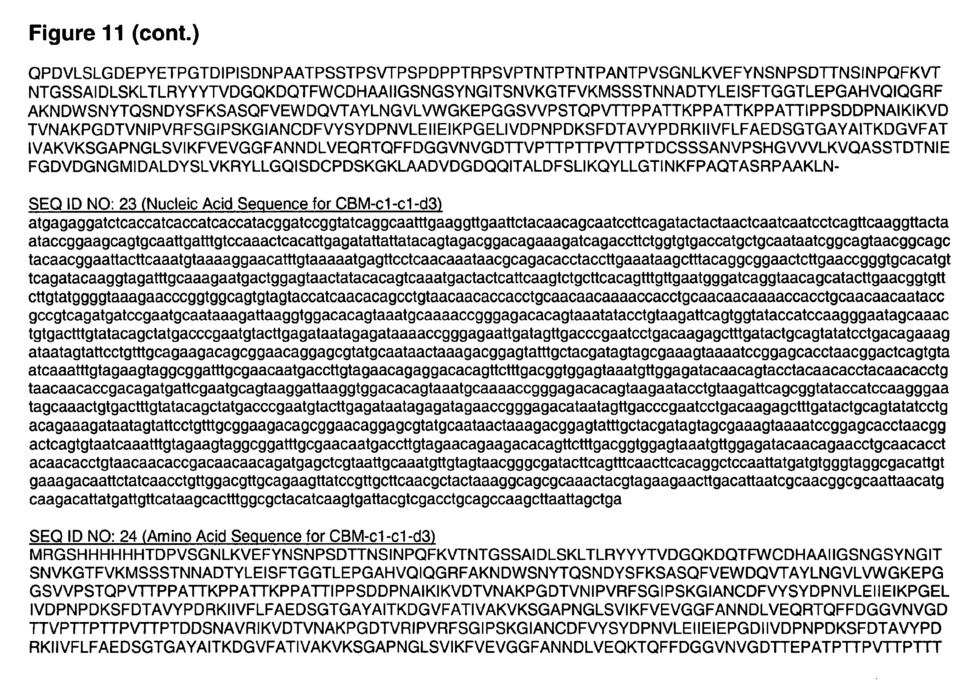

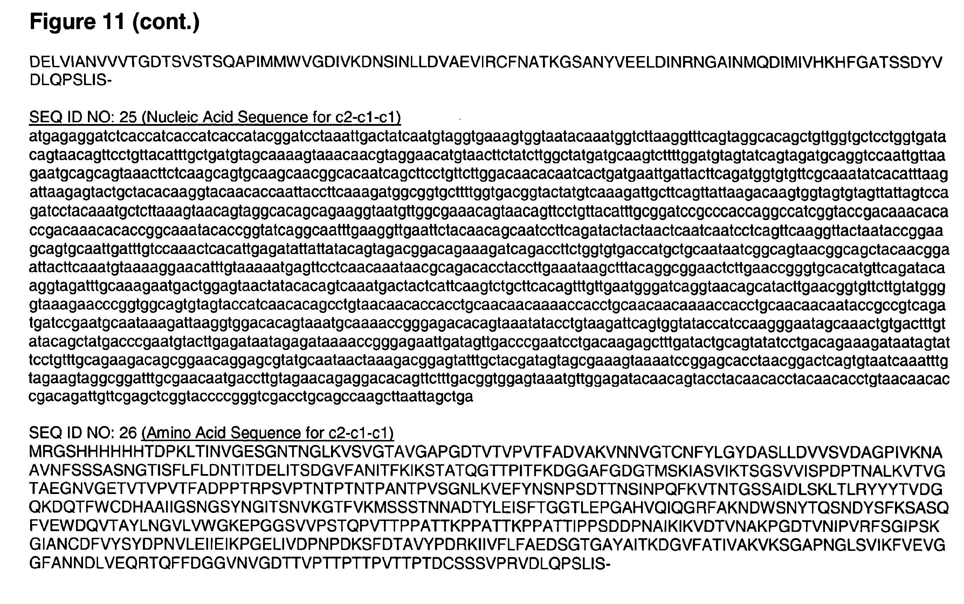

The pattern of products (glucose and cellodextrins) was identical from the free enzymes (SM901), the artificial complexes and the native cellulosomes for soluble as well as for insoluble cellulose (Fig. 11). In the case of insoluble cellulose as substrate, the main product is cellobiose with some glucose as a secondary product. The cellobiose has to be further degraded to glucose by addition of β-glucosidase to the complex. The β-glucosidase gene from *Thermotoga neapolitana,* genetically fused to a dockerin module, was used successfully.

Reconstitution of the cellulosome was thus possible. It could therefore be demonstrated for the first time that the order of components seems to be random along the scaffoldin and that the activity of the *in vitro* reconstituted cellulosome at least equalled that of the native cellulosome.

### 4. Results

The results showed clearly that the different cohesins bound the components equally and did not discriminate between cellulosomal components containing different dockerin modules. The binding to the cohesins was random. The assembly between cohesins and dockerins was fast and spontaneous. Once bound, the components were tightly fixed to the scaffoldin. An increase of the number of cohesins in the scaffoldin backbone by linking the individual scaffoldin backbones by cohesin-dockerin interaction did increase the activity on crystalline cellulose (Fig. 6). Further, Figure 6 shows that the addition of a certain type of CBM to the complex increased activity (Fig. 6). Furthermore, the "complete" scaffoldin forming a reconstituted cellulosome had the highest activity higher than that of the native cellulosome (even prior to systematic optimization).

### References:

**1**Schwarz, W. H., (2004). Cellulose "Struktur ohne Ende", Naturwiss. Rundschau, 8:443-445.
**2** Singhania, R. R., et al. (2010), "Advancement and comparative profiles in the production technologies using solid-state and submerged fermentation for microbial cellulases"; Enz. Microb. Technol. 46:541-549
**3**Lynd, L., Weimer, P. J., van Zyl, W. H., and Pretorius, I. S. (2002). Microbial cellulose utilization: fundamentals and biotechnology. Microbiol. Molec. Biol. Rev. 66, 506-577**.**
**4**Shoham, Y., R. Lamed, and E. A. Bayer. 1999. The cellulosome concept as an efficient microbial strategy for the degradation of insoluble polysaccharides. Trends in Microbiol. 7:275-281**.**
**5**Bayer, E. A., Y. Shoham, and R. Lamed. 2000. Cellulose-decomposing bacteria and their enzyme systems. In M. Dworkin, S. Falkow, E. Rosenberg, K.-H. Schleifer, and E. Stackebrandt (eds.), The Prokaryotes: An Evolving Electronic Resource for the Microbiological Community, 3rd edition. Springer-Verlag, New York.
**6**Schwarz, W. H. 2001. The cellulosome and cellulose degradation by anaerobic bacteria. Appl. Microbiol. Biotechnol. 56:634-649.
**7**Mechaly, A., S. Yaton, R. Lamed, H.-P. Fierobe, A. Belaich, J.-P. Belaich, Y. Shoham, and E.A. Bayer. 2000. Cohesin-dockerin recognition in cellulosome assembly: Experiment versus hypothesis. Proteins 39:170-177.
**8**Leibovitz, E., H. Ohayon, P. Gounon, and P. Béguin. 1997. Characterization and subcellular localization of the Clostridium thermocellum scaffoldin dockerin binding protein SdbA. J. Bacteriol. 179:2519-2523.
**9** Bayer, E. A., L. J. W. Shimon, Y. Shoham, and R. Lamed. 1998. Cellulosomes - structure and ultrastructure. J. Struct. Biol. 124:221-234.
**10**Zverlov, V. V., G. A. Velikodvorskaya, and W. H. Schwarz. 2003. Two new cellulosome components encoded downstream of cell in the genome of Clostridium thermocellum: the non-processive endoglucanase CeIN and the possibly structural protein CseP. Microbiol. 149:515-524.
**11** Zverlov, V. V., Kellermann, J., & Schwarz, W. H. (2005). Functional subgenomics of Clostridium thermocellum cellulosomal genes: Identification of the major catalytic components in the extracellular complex and detection of three new enzymes. Proteomics, 5. 3646-3653.
**12**DOE Joint Genome Institute: http://genome.jgi-psf.org/microbial/
**13** Gold N.D., & Martin V.J. (2007). Global view of the Clostridium thermocellum cellulosome revealed by quantitative proteomic analysis. J Bacteriol. 189:6787-95.
**14**Fierobe, H.-P. et al. (2002). Degradation of cellulose substrates by cellulosome chimeras. J. BioLChem. 277: 49621-49630.
**15**Bayer, E. A., R. Kenig, & R. Lamed (1983). Adherence of Clostridium thermocellum to cellulose. J. Bacteriol. 156: 818-827.
**16**Bayer, E.A., Y. Shoham, J. Tormo, & R. Lamed (1996). The cellulosome: a cell surface organelle for the adhesion to and degradation of cellulose. In: Bacterial adhesion: molecular and ecological diversity, pp. 155-182. Wiley-Liss, Inc..
**17**Zverlov, V.V., Klupp, M., Krauss, J., & Schwarz, W.H. (2008). Mutants in the scaffoldin gene cipA of Clostridium thermocellum with impaired cellulosome formation and cellulose hydrolysis: insertions of a new IS-element, IS1447, and implications for cellulase synergism on crystalline cellulose. J. Bacteriol. 190: 4321-4327.

## Claims

1. A complex comprising:
a) a backbone scaffold comprising at least four binding sites, wherein at least two of the binding sites have essentially the same binding specificity; and
b) an enzyme component bound to each of said four binding sites, wherein at least three of said enzyme components are different enzyme components.

2. The complex of claim 1, wherein the backbone scaffold is a linear, synthetic or biological backbone.

3. The complex of claim 1 or 2, wherein the backbone scaffold has at least four cohesin binding sites for dockerins.

4. The complex of any one of claim 1 to 3, wherein the backbone scaffold consists of one or more proteins, wherein the one ore more proteins are linked together by chemical interaction or by a cohesin-dockerin interaction, whereby the binding specificity of the linking interaction is different from the binding specificity of the enzymes.

5. The complex of any one of claims 1 to 4, wherein the backbone scaffold is derived from a non-catalytic scaffolding protein from cellulolytic, cellulosome forming microorganisms or genetically modified derivatives thereof.

6. The complex of any one of claims 1 to 5, wherein the backbone scaffold is derived from the non-catalytic scaffolding protein CipA from *Clostridium thermocellum* or genetically modified derivatives thereof.

7. The complex of claim 6, wherein the backbone scaffold comprises CBM-c1-c1-d3 as shown in SEQ ID NO: 24, c3-c1-c1-d2 as shown in SEQ ID NO: 22, c2-c1-c1 as shown in SEQ ID NO: 26 or derivatives thereof having more than 60 % amino acid sequence identity in their cohesin modules.

8. The complex of any one of claims 1 to 7, wherein the backbone scaffold comprises a carbohydrate binding module (CBM).

9. The complex of claim 8, wherein the carbohydrate binding module is a carbohydrate binding module (CBM3) from the *cipA* gene of *Clostridium thermocellum* that is integrated into or attached to the linear backbone scaffold.

10. The complex of any one of claims 1 to 9, wherein the enzyme component comprises a dockerin module and a catalytic module of an enzyme.

11. The complex of any one of claims 1 to 10, wherein the enzyme components are selected from the group consisting of: processive or non-processive endo-β-1,4-glucanases, processive exo-ß-1,4-glucanases and glycosidases from polysaccharolytic microorganisms or genetically modified derivatives thereof.

12. The complex of claim 11, wherein the enzyme components are derived from dockerin module containing components of the *Clostridium thermocellum* cellulosome or from non-cellulosomal components of *Clostridium thermocellum* having a dockerin module fused thereto.

13. The complex of any one of claims 1 to 12, wherein the enzyme components comprise CeIK-d1 as shown in SEQ ID NO: 8, CeIR-d1 as shown in SEQ ID NO: 10, CeIT-d1 as shown in SEQ ID NO: 14, CeIE-d1 as shown in SEQ ID NO: 16, CeIS-d1 as shown in SEQ ID NO: 6 as shown in SEQ ID NO: and BgIB-d1 as shown in SEQ ID NO: 4 or derivatives thereof having more than 50 % amino acid sequence identity in their dockerin modules.

14. A method for preparing the complex according to any one of claims 1 to 13
a) recombinantly producing the enzyme components of any one of claims 1 and 10 to 13,
b) recombinantly producing the backbone scaffold of any one of claims 1 to 8,
c) mixing the purified, partially purified or non-purified components of a) and b); and
d) randomly binding the enzyme components to the backbone scaffold.

15. The method of claim 14, wherein the total amount of backbone scaffolds in step c) and the total amount of enzyme components are mixed together in a molar ratio of 1 cohesin module to 1 enzyme component, and the at least three enzyme components are mixed together in a molar ratio of 1:1 to 1:15 to each other.

16. A method for enzymatic hydrolysis of polysaccharide substrates comprising the steps of:
a) mixing the complex of any one of claims 1 to 13 with insoluble cellulose; and optionally
b) isolating the degradation products.

17. Use of the complex of any one of claims 1 to 13 for enzymatic hydrolysis of polysaccharide substrates.

18. The method of claim 15 or 16, or the use of claim 17, wherein the polysaccharide substrate is crystalline cellulose or a crystalline cellulose containing substrate.
